(19)

**Europäisches Patentamt**

**European Patent Office**

**Office européen des brevets**

(11) **EP 2 837 934 A1**

(12) **EUROPEAN PATENT APPLICATION**
published in accordance with Art. 153(4) EPC

(43) Date of publication:
**18.02.2015 Bulletin 2015/08**

(21) Application number: **13775272.1**

(22) Date of filing: **10.04.2013**

(51) Int Cl.:
*G01N 27/62* [(2006.01)]   *C07K 5/00* [(2006.01)]
*C07K 7/00* [(2006.01)]   *G01N 33/483* [(2006.01)]

(86) International application number:
**PCT/JP2013/061345**

(87) International publication number:
**WO 2013/154208 (17.10.2013 Gazette 2013/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.04.2012  JP 2012089662**

(71) Applicants:
• **Gifu University**
  **Gifu-shi, Gifu 501-1193 (JP)**
• **Ke'ken Textile Testing&Certification Center**
  **Tokyo 113-0034 (JP)**

(72) Inventors:
• **NISHIKAWA, Kazuya**
  **Gifu-shi, Gifu 501-1193 (JP)**

• **YOKOGAWA, Takashi**
  **Gifu-shi, Gifu 501-1193 (JP)**
• **OHNO, Satoshi**
  **Gifu-shi, Gifu 501-1193 (JP)**
• **HAYASHI, Katsumasa**
  **Tokyo 113-0034 (JP)**
• **MATSUNAGA, Nobuyoshi**
  **Tokyo 113-0034 (JP)**
• **MOTOYAMA, Aya**
  **Tokyo 113-0034 (JP)**

(74) Representative: **Herzog, Fiesser & Partner**
**Patentanwälte PartG mbB**
**Patentanwälte**
**Dudenstrasse 46**
**68167 Mannheim (DE)**

(54) **METHOD FOR IDENTIFYING AND QUANTIFYING TYPES OF ANIMAL HAIR**

(57)   An object of the present invention is to provide a method, by which an animal species of an animal hair can be specified and quantified, through direct analysis of not DNA existing in a small amount in a sample containing animal hair, but proteins that are major components of animal hair. The present invention provides a method for specifying animal species of animal hair and quantifying an animal hair, comprising analyzing an animal hair sample by mass spectrometry to analyze a protein component such as keratin that is a major protein component of animal hair, specifying the masses, the mass-to-charge ratios (m/z), the LC elution times of the mass-to-charge ratios, and partial sequences useful as markers as analytical results, and thus specifying animal species of animal hair and quantifying the animal hair based thereon.

Fig. 1

EP 2 837 934 A1

SEQUENCE LISTING

```
<110>  Gifu University
       KEKEN Textile Testing & Certification Center

<120>  Method for ideintifying animal hair and method for determining
quatity
thereof

<130>  PH-5551-PCT

<150>  JP 2012-089662
<151>  2012-04-10

<160>  13

<170>  PatentIn version 3.5

<210>  1
<211>  12
<212>  PRT
<213>  Bos grunniens

<400>  1

Gly Leu Asn Asn Arg Phe Ala Ala Phe Ile Asp Lys
1               5                   10


<210>  2
<211>  42
<212>  DNA
<213>  Bos grunniens

<400>  2
ggtctcaaca acaggtttgc tgccttcatc gacaaggtgc gc                        42


<210>  3
<211>  19
<212>  PRT
<213>  Capra hircus laniger


<220>
<221>  misc_feature
<222>  (3)..(3)
<223>  Xaa is Leu or Ile

<220>
<221>  misc_feature
<222>  (6)..(6)
<223>  Xaa is Gln or Lys

<220>
<221>  misc_feature
<222>  (10)..(10)
<223>  Xaa is Leu or Ile

<220>
<221>  misc_feature
<222>  (11)..(11)
<223>  Xaa is Gln or Lys
```

```
<220>
<221>  misc_feature
<222>  (13)..(13)
<223>  Xaa is Gln or Lys

<400>  3

Ser Asp Xaa Glu Ala Xaa Val Glu Ser Xaa Xaa Met Xaa Phe Asp Val
1               5                   10                  15

Arg Pro Lys


<210>  4
<211>  3
<212>  PRT
<213>  Ovis aries


<220>
<221>  misc_feature
<222>  (3)..(3)
<223>  Xaa is Leu or Ile

<400>  4

Gln Phe Xaa
1


<210>  5
<211>  5
<212>  PRT
<213>  Ovis aries

<400>  5

Phe Glu Asn Glu Tyr
1               5


<210>  6
<211>  7
<212>  PRT
<213>  Ovis aries


<220>
<221>  misc_feature
<222>  (1)..(1)
<223>  Xaa is Gln or Lys

<220>
<221>  misc_feature
<222>  (6)..(6)
<223>  Xaa is Leu or Ile

<220>
<221>  misc_feature
<222>  (7)..(7)
```

<223>   Xaa is Gln or Lys

<400>   6

Xaa Asn Phe Val Ala Xaa Xaa
1               5


<210>   7
<211>   3
<212>   PRT
<213>   Ovis aries


<220>
<221>   misc_feature
<222>   (1)..(1)
<223>   Xaa is Leu or Ile

<400>   7

Xaa Phe Gln
1


<210>   8
<211>   5
<212>   PRT
<213>   Ovis aries

<400>   8

Tyr Glu Asn Glu Phe
1               5


<210>   9
<211>   7
<212>   PRT
<213>   Ovis aries


<220>
<221>   misc_feature
<222>   (1)..(1)
<223>   Xaa is Gln or Lys

<220>
<221>   misc_feature
<222>   (2)..(2)
<223>   Xaa is Leu or Ile

<220>
<221>   misc_feature
<222>   (7)..(7)
<223>   Xaa is Gln or Lys

<400>   9

Xaa Xaa Ala Val Phe Asn Xaa
1               5

```
<210>  10
<211>  4
<212>  PRT
<213>  Ovis aries


<220>
<221>  misc_feature
<222>  (3)..(3)
<223>  Xaa is Leu or Ile

<400>  10

Ser Asp Xaa Glu
1



<210>  11
<211>  8
<212>  PRT
<213>  Ovis aries


<220>
<221>  misc_feature
<222>  (1)..(1)
<223>  Xaa is Gln or Lys

<220>
<221>  misc_feature
<222>  (4)..(5)
<223>  Xaa is Leu or Ile

<220>
<221>  misc_feature
<222>  (7)..(7)
<223>  Xaa is Leu or Ile

<400>  11

Xaa Glu Glu Xaa Xaa Cys Xaa Lys
1                   5


<210>  12
<211>  4
<212>  PRT
<213>  Ovis aries


<220>
<221>  misc_feature
<222>  (2)..(2)
<223>  Xaa is Leu or Ile

<400>  12

Glu Xaa Asp Ser
1



<210>  13
<211>  8
```

```
<212>   PRT
<213>   Ovis aries


<220>
<221>   misc_feature
<222>   (2)..(2)
<223>   Xaa is Leu or Ile

<220>
<221>   misc_feature
<222>   (4)..(5)
<223>   Xaa is Leu or Ile

<220>
<221>   misc_feature
<222>   (8)..(8)
<223>   Xaa is Gln or Lys

<400>   13

Lys Xaa Cys Xaa Xaa Glu Glu Xaa
1                   5
```

**Description**

Technical Field

The present invention relates to a method for specifying animal species of animal hair. More specifically, it relates to a method for obtaining an extract from a sample containing animal hair, and specifying animal species of animal hair and/or quantifying the animal hair using mass spectrometry.

Background Art

Cashmere fibers are collected from cashmere goats that are raised in a harsh natural environment. Thus, they have unique excellent properties and are traded at high prices among animal hair products; however, concerns have been raised recently in regard to the disguising of wool blends as 100% cashmere fibers.
With respect to the labeling of such clothing, testing methods using microscopy are specified for fiber differentiation and the percentages of fibers mixed therein in the JIS (Japanese Industrial Standards) L 1030 (Testing methods for quantitative analysis of fiber mixtures of textiles). In the case of microscopy, differentiation is performed according to the appearance of hairs. Hence, the identification results of microscopy may be affected by changes in fiber surface due to processing or appearance very similar to that of cashmere. In particular, the fiber surface shape of yak is very similar to that of brown cashmere. Thus, it is very difficult to detect by microscopy when yak is mixed into a product labeled as cashmere 100%. A method of conveniently and reliably identifying the mixture of yak and cashmere has been required.
In addition, identification results of microscopy may differ depending on the skill of the differentiation expert. Identification is generally performed by several differentiation experts to ensure reliable differentiation. Thus, the development of supplemental techniques for microscopy and identification methods relating thereto has been an important issue.
As a supplement to microscopic differentiation techniques, differentiation methods based on DNA have been reported in recent years. With regard to the identification of fibers, particularly including cashmere products, Japan Spinners Inspecting Foundation (currently, Boken Quality Evaluation Institute) applied for a patent under JP Patent Application No. 2003-297332 (Patent Document 1: JP Patent Publication (Kokai) No. 2004-121229 A), and Japan Synthetic Textile Inspection Association (currently, KAKEN Test Center) applied for a patent under JP Patent Application No. H11-15616 (Patent Document 2: JP Patent Publication (Kokai) No. 2000-210084 A) and JP Patent Application No. 11-135993 (Patent Document 3: JP Patent Publication (Kokai) No. 2000-325098 A), for example. A patent was granted for JP Patent Application No. H11-15616 (Patent Document 4: JP Patent No. 3,566,570). These methods involve amplifying the mitochondrial DNA that exists in great number per cell. More specifically, it involves designing species-specific DNA primers based on the sequence of cytochrome b, performing PCR using DNA extracted from an animal hair product as a template, and analyzing the molecular weight or the like of the amplified DNA, so as to determine the species. The present inventors have also advanced the development of an identification method using DNA. A criterion for identification is the presence or absence of amplification by PCR; however, although amplification is confirmed for an animal species of animal hair, amplification is not always possible for wool products produced from the animal hair. DNA analysis is relatively easy for combed-out hair fibers containing hair roots; however, DNA analysis is difficult for root-free hair (e.g., fibers cut from animals). Industrially, cut hair fibers containing no hair roots are used in most cases. Another reason is that DNA cannot be efficiently extracted because of treatment with dyes, for example. There is also the risk of false positive results due to amplification of a trace amount of DNA. Actually, it is difficult to quantify such a trace amount of DNA.

Prior Art Documents

Patent Documents

Patent Document 1 Japanese Patent Publication (Kokai) No. 2004-121229A
Patent Document 2 Japanese Patent Publication (Kokai) No. 2000-210084 A
Patent Document 3 Japanese Patent Publication (Kokai) No. 2000-325098 A
Patent Document 4 JP Patent No. 3566570

Summary of the Invention

In order to avoid the above-mentioned problems, an object of the present invention is to provide a method by which the animal species of animal hair can be specified and quantified by directly analyzing not DNA existing in a trace amount of a sample containing animal hair but the proteins that are major components of animal hair.

An object of the present invention is to develop the supplemental testing methods for differentiation of animal hair products using microscopy as specified in JIS (Japanese Industrial Standards) L 1030 (Testing methods for quantitative analysis of fiber mixtures of textiles) or alternative techniques.

The present invention provides a method that comprises analyzing a major protein component (e.g., keratin) in an animal hair sample by mass spectroscopy, specifying the mass, the mass-to-charge ratio (m/z), the LC elution time for the mass-to-charge ratio, and a useful partial sequence as a marker, thus specifying animal species of animal hair and quantifying the animal hair, and calculating the percentage of animal hair mixed in a sample.

More specifically, the present inventors have provided the following invention, by which the above object is achieved.

A. The present invention

[1] A method for specifying animal species of animal hair and/or quantifying the animal hair contained in a sample, comprising the steps of:

(a) obtaining an analytical sample from the sample by pretreatment;
(b) analyzing the analytical sample obtained in step (a) above using mass spectrometry and then obtaining a result; and
(c) specifying animal species of the animal hair and/or quantifying the animal hair from the result obtain in step (b) above.

[2] The method according to [1], wherein, in addition to the above mass spectrometry, liquid chromatography is used in combination with mass spectroscopy (also referred to as "LC-MS" or "UPLC-MS").

[3] The method according to any one of [1] and [2], wherein the animal species is one or more species selected from the group consisting of a cashmere species of the genus *Capra* of the family Bovidae, an Angora species of the genus *Capra* of the family Bovidae, a yak species of the genus *Bos* of the family Bovidae, a sheep species of the genus *Ovis* of the family Bovidae, the genus *Camelus* of the family Camelidae, an alpaca species of the genus *Lama* of the family Camelidae (or an alpaca species of the genus *Vicugna),* and an Angora rabbit species of the genus *Oryctolagus* of the family Leporidae.

[4] The method according to any one of [1] to [3], wherein step (c) above comprises comparing the result obtained in step (b) above with at least one of reference data obtained by performing steps (a) and (b) above under the same conditions for a reference animal hair sample of which the animal species have been confirmed in advance, and then specifying animal species of animal hair and/or quantifying an animal hair based on the masses, and/or the mass-to-charge ratios (also referred to as "m/z"), and/or the elution times of the relevant mass-to-charge ratios, detected specifically to the animal hair.

[5] The method according to [4], wherein in the step of specifying animal species of animal hair and/or quantifying an animal hair, the reference data correspond to one or more types of mass and/or mass-to-charge ratio selected from the following masses and mass-to-charge ratios:

(I) yak species

(1) the mass detected specifically to the yak species:

5948.90 ± 0.02%, or 2137.92 ± 0.02%

(2) the mass-to-charge ratio (m/z) of molecular ions calculated from the mass of (1) above or another mass-to-charge ratio (m/z) detected specifically to the yak species: 9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, or 1069.98 ± 0.02%

(II) cashmere species

(1) the mass detected specifically to the cashmere species:

8976.45 ± 0.02%, 7102.18 ± 0.02%, or 7074.18 ± 0.02%,

(2) the mass-to-charge ratio (m/z) of molecular ions calculated from the mass of (1) above or another mass-to-charge ratio (m/z) detected specifically to the cashmere species: 10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, or 1415.83 ± 0.02%

(III) sheep species

(1) the mass detected specifically to the sheep species: 17538.88 ± 0.02% or 17584.26 ± 0.02%
(2) the mass-to-charge ratio (m/z) of molecular ions resulting from the above mass or another mass-to-charge ratio (m/z) detected specifically to the sheep species: 10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, or 1599.55 ± 0.02%

(IV) Angora species (also referred to as "mohair")

(1) the mass detected specifically to the Angora species: 6329.52 ± 0.02%
(2) the mass-to-charge ratio (m/z) of molecular ions calculated from the mass of (1) above

(V) alpaca species of the genus *Lama* of the family Camelidae (or alpaca species of the genus *Vicugna*)

(1) the mass detected specifically to the alpaca species:

6358.60 ± 0.02%, or 6342, 70 ± 0.02%

(2) the mass-to-charge ratio (m/z) of molecular ions calculated from the mass of (1) above

(VI) Angora rabbit species of the genus *Oryctolagus* of the family Leporidae (also referred to as "Angora")

(1) the mass detected specifically to the Angora rabbit species: 1042.43 ± 0.02%
(2) the mass-to-charge ratio (m/z) of molecular ions calculated from the mass of (1) above.

[6] The method according to any one of [1] to [3], wherein step (a) above of obtaining an analytical sample from the sample by pretreatment includes a step of using lysyl endopeptidase (also referred to as "Lys-C").
[7] The method according to [6], wherein step (c) above comprises comparing the result obtained in step (b) above with at least one of reference data obtained by performing steps (a) and (b) above under the same conditions for a reference animal hair sample of which animal species have been confirmed in advance, and then specifying animal species of an animal hair and/or quantifying the animal hair based on the masses, and/or the mass-to-charge ratios (also referred to as "m/z"), and/or the elution times of the relevant mass-to-charge ratios, detected specifically to an animal hair of the animal species.
[8] The method for specifying an animal species of animal hair according to [6], wherein step (c) above comprises displaying the result obtained in step (b) above by data processing on a map representing elution times, detected mass-to-charge ratios, and detected intensities, comparing the result with at least one of reference data obtained by performing steps (a) and (b) above under the same conditions for a reference animal hair sample of which animal species have been confirmed in advance, and then specifying the animal species of animal hair based on the degree of correlation.
[9] The method according to [7] or [8], wherein in the above step of specifying species of animal and/or quantifying an animal hair, the reference data correspond to the following one or more types of mass or mass-to-charge ratio,

(I) yak species of the genus *Bos* of the family Bovidae

(1) the mass detected specifically to the yak species:

1364.70 ± 0.02%, 1383.72 ± 0.02%, 1431.67 ± 0.02%, 2469.27 ± 0.02%, or 2495.30 ± 0.02%

(2) the mass-to-charge ratio (m/z) of molecular ions calculated from the mass of (1) above

(II) cashmere species of the genus *Capra* of the family Bovidae

(1) the mass detected specifically to the cashmere species:

2218.19 ± 0.02%, 3379.48 ± 0.02%, or 2391.99 ± 0.02%,

(2) the mass-to-charge ratio (m/z) of molecular ions calculated from the mass of (1) above

(III) sheep species of the genus *Ovis* of the family Bovidae

(1) the mass detected specifically to the sheep species,
2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, or 2174.00 ± 0.02%,
(2) the mass-to-charge ratio (m/z) of molecular ions calculated from the mass of (1) above

(IV) the genus *Camelus* of the family Camelidae

(1) the mass detected specifically to the genus *Camelus,*
2888.35 ± 0.02%,
(2) the mass-to-charge ratio (m/z) of molecular ions calculated from the mass of (1) above

(V) alpaca species of the genus *Lama* of the family Camelidae (or alpaca species of the genus *Vicugna*)

(1) the mass detected specifically to the alpaca species (or the alpaca species of the genus *Vicugna*),
4599.37 ± 0.02%, 901.47 ± 0.02%, or 532.32 ± 0.02%,
(2) the mass-to-charge ratio (m/z) of molecular ions calculated from the mass of (1) above

(VI) Angora rabbit species of the genus *Oryctolagus* of the family Leporidae

(1) the mass detected specifically to the Angora rabbit species,
2303.07 ± 0.02%, 2502.15 ± 0.02%, 2058.02 ± 0.02%, or 1554.81 ± 0.02%,
(2) the mass-to-charge ratio (m/z) of molecular ions calculated from the mass of (1) above.

[10] The method according to [7] or [9], wherein the above reference data are yak-species-specific mass-to-charge ratios and are the mass-to-charge ratios of molecular ions resulting from a peptide and/or a protein comprising the amino acid sequence represented by SEQ ID NO: 1: GLNNRFAAFIDK.
[11] The method according to [7] or [9], wherein the reference data are yak-species-specific mass-to-charge ratios and are the mass-to-charge ratios of molecular ions resulting from a protein that is expressed by a gene comprising the DNA sequence represented by SEQ ID NO: 2: GGT CTC AAC AAC AGG TTT GCT GCC TTC ATC GAC AAG GTG CGC.
[12] The method according to [7] or [9], wherein the reference data are cashmere-species-specific mass-to-charge ratios and are the mass-to-charge ratios of molecular ions resulting from a peptide and/or a protein comprising the amino acid sequence represented by SEQ ID NO: 3: SD (L or I) EA (Q or K) VES (L or I) (Q or K) M (Q or K) FDVRPK.
[13] The method according to [7] or [9], wherein the reference data are sheep-species-specific mass-to-charge ratios and are the mass-to-charge ratios of molecular ions resulting from a peptide and/or a protein comprising one or more amino acid sequences represented by SEQ ID NOS: 4 to 13,

SEQ ID NO: 4: QF (L or I),
SEQ ID NO: 5: FENEY,
SEQ ID NO: 6: (Q or K) NFVA (L or I) (Q or K),
SEQ ID NO: 7: (L or I) FQ,
SEQ ID NO: 8: YENEF,
SEQ ID NO: 9: (Q or K) (L or I) AVFN (Q or K),
SEQ ID NO: 10: SD (L or I) E,
SEQ ID NO: 11: (Q or K) EE (L or I) (L or I) C (L or I) K,
SEQ ID NO: 12: E (L or I) DS, and

SEQ ID NO: 13: K (L or I) C (L or I) (L or I) EE (Q or K).

[14] A peptide or a nucleic acid animal hair marker, which is any one of the following (1) to (5):

(1) a yak-specific peptide marker, which is shown in SEQ ID NO: 1;
(2) a cashmere-specific peptide marker, which is shown in SEQ ID NO: 3;
(3) a sheep-specific peptide marker, which contains a combination of 2 or more amino acid sequences shown in SEQ ID NOS: 4 to 9;
(4) a sheep-specific peptide marker, which contains a combination of 2 or more amino acid sequences shown in SEQ ID NOS: 10 to 13;
(5) a yak-specific nucleic acid marker, which is shown in SEQ ID NO: 2.

[15] A method for calculating the percentages of 2 animal species of animal hair mixed in a sample, comprising the steps of

(a) obtaining an analytical sample from the sample by pretreatment;
(b) analyzing the analytical sample obtained in step (a) above using mass spectrometry and then obtaining a result;
(c) after the steps (a) and (b) are performed under the same conditions,
drawing an ion chromatogram displaying only the mass-to-charge ratios specific to one of the two animal species of animal hair, which are extracted from the total mass ion chromatogram of a sample containing the relevant animal hair of the animal species, performing data processing for the chromatogram, and then calculating the peak area of the animal -species-pecific mass-to-charge ratios; and
(d) calculating the percentages of the animal species of animal hair mixed in the sample from the peak area.

[16] The method according to [15], wherein the following steps are added before step (d) above:

(i) drawing an ion chromatogram of mass-to-charge ratios (m/z) derived from common molecular species shared by 2 animal hair species, selecting the common mass-to-charge ratios of hair of each animal species, and then finding the peak area of the common mass-to-charge ratios;
(ii) calculating the peak area of animal-species-specific mass-to-charge ratios with respect to the peak area of the common mass-to-charge ratios.

[17] The method according to [15] or [16], comprising plotting the peak areas and the mixture ratio of animal species of animal hair mixed in a sample prepared by artificially mixing reference animal hairs in the sample, wherein the reference animal hairs of which animal species have been confirmed in advance, on a graph, preparing a calibration curve, and then calculating the percentage of an animal species of animal hair mixed in an unknown sample from the peak area using the calibration curve.

[18] A method for specifying 1 or more animal species of animal hair in a sample, and calculating the percentages of the animal species of the animal hair mixed in the sample when the sample contains animal hair of 2 or more animal species, comprising the steps of:

(a) obtaining an analytical sample from the sample by pretreatment;
(b) analyzing the analytical sample obtained in step (a) above using mass spectrometry and then obtaining a result;
(c) specifying the animal species of animal hair based on the result obtained in step (b) above;
(d) when 2 or more animal hair species are specified above, calculating the peak area of the specified-animal-specific mass-to-charge ratios for each of the thus identified animal varieties; and
(e) calculating the percentage of each animal species mixed in the sample through comparison with the peak area of a reference sample containing the 100% animal species.

[19] The method according to [18], wherein the following steps are added before step (e) above:

(i) drawing an ion chromatogram of mass-to-charge ratios (m/z) derived from the common molecular species shared by animal species of animal hair mixed in a sample, selecting the common mass-to-charge ratios of hair of each animal species, and then finding the peak area of the common mass-to-charge ratios;
(ii) calculating the peak area of animal-species-specific mass-to-charge ratios with respect to the peak area of the common mass-to-charge ratios.

[20] The method according to [15] to [19], comprising calculating the percentages of 2 or more animal species of animal hair (hereinafter, referred to as "N" species) mixed in a sample containing "N-1" species of animal hair, and then finding the percentages of N species of animal hair based on a difference from 100%.

[21] The method according to any one of [15] to [20] for calculating the percentage of one or more animal species of animal hair mixed in a sample containing animal hair of 1 or more animal species and non-animal-hair fibers, comprising a step of calculating the mixture ratio of non-animal-hair fibers to animal hair by the release method, the dissolution method, or the like specified in the JIS L1030, before step (a) above of obtaining an analytical sample from the sample by pretreatment, or, after a step of calculating the percentage of animal hair mixed in the sample.

[22] The method according to any one of [15] to [21], wherein the mass-to-charge ratio specific to an animal species is one or more mass-to-charge ratios selected from the following mass-to-charge ratios,

(I) yak species

(1) the mass detected specifically to the yak species:

5948.90 ± 0.02%, or 2137.92 ± 0.02%

(2) the mass-to-charge ratio (m/z) of molecular ions calculated from the mass of (1) above or another mass-to-charge ratio (m/z) detected specifically to the yak species: 9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, or 1069.98 ± 0.02%

(II) cashmere species

(1) the mass detected specifically to the cashmere species:

8976.45 ± 0.02%, 7102.18 ± 0.02%, or 7074.18 ± 0.02%,

(2) the mass-to-charge ratio (m/z) of molecular ions calculated from the mass of (1) above or another mass-to-charge ratio (m/z) detected specifically to the cashmere species:

10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, or 1415.83 ± 0.02%

(III) sheep species

(1) the mass detected specifically to the sheep species:

17538.88 ± 0.02% or 17585.26 ± 0.02%

the mass-to-charge ratio (m/z) of molecular ions calculated from the mass of (1) above or another mass-to-charge ratio (m/z) detected specifically to the sheep species:

10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17586.26 ± 0.02%, 1955.10 ± 0.02%, 1759.47 ± 0.02%, or 1599.55 ± 0.02%

(IV) Angora species (also referred to as "mohair")

(1) the mass detected specifically to the Angora species: 6329.52 ± 0.02%
(2) the mass-to-charge ratio (m/z) of molecular ions calculated from the mass of (1) above

(V) alpaca species of the genus *Lama* of the family Camelidae (or alpaca species of the genus *Vicugna)*

(1) the mass detected specifically to the alpaca species:

6358.60 ± 0.02% or 6342.70 ± 0.02%

(2) the mass-to-charge ratio (m/z) of molecular ions calculated from the mass of (1) above

(VI) Angora rabbit species of the genus *Oryctolagus* of the family Leporidae (also referred to as "Angora")

(1) the mass detected specifically to the Angora rabbit species:

1042.43 ± 0.02%

(2) the mass-to-charge ratio (m/z) of molecular ions calculated from the mass of (1) above.

[23] The method according to any one of [15] to [21], wherein the step (a) above of obtaining an analytical sample from the sample by pretreatment includes a step of using lysyl endopeptidase (also referred to as "Lys-C"), and the animal-species-specific mass-to-charge ratio is 1 or more mass-to-charge ratios selected from the following mass-to-charge ratios.

(I) yak species of the genus *Bos* of the family Bovidae

(1) the mass detected specifically to the yak species
1364.70 ± 0.02%, 1383.72 ± 0.02%, 1431.67 ± 0.02%, 2469.27 ± 0.02%, or 2495.30 ± 0.02%
(2) the mass-to-charge ratio (m/z) of molecular ions calculated from the mass of (1) above

(II) cashmere species of the genus *Capra* of the family Bovidae

(1) the mass detected specifically to the cashmere species,
2218.19 ± 0.02%, 3379.48 ± 0.02%, or 2391.99 ± 0.02%,
(2) the mass-to-charge ratio (m/z) of molecular ions calculated from the mass of (1) above

(III) sheep species of the genus *Ovis* of the family Bovidae

(1) the mass detected specifically to the sheep species,
2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, or 2174.00 ± 0.02%,
(2) the mass-to-charge ratio (m/z) of molecular ions calculated from the mass of (1) above

(IV) the genus *Camelus* of the family Camelidae

(1) the mass detected specifically to the genus *Camelus,* 2888.35 ± 0.02%,
(2) the mass-to-charge ratio (m/z) of molecular ions calculated from the mass of (1) above

(V) alpaca species of the genus *Lama* of the family Camelidae (or alpaca species of the genus *Vicugna*)

(1) the mass detected specifically to the alpaca species (or, the alpaca species of the genus *Vicugna),*
4599.37 ± 0.02%, 901.47 ± 0.02%, or 532.32 ± 0.02%,
(2) the mass-to-charge ratio (m/z) of molecular ions calculated from the mass of (1) above

(VI) Angora rabbit species of the genus *Oryctolagus* of the family Leporidae

(1) the mass detected specifically to the Angora rabbit species,
2303.07 ± 0.02%, 2502.15 ± 0.02%, 2058.02 ± 0.02%, or 1554.81 ± 0.02%,
(2) the mass-to-charge ratio (m/z) of molecular ions calculated from the mass of (1) above.

[24] The method according to [16] or [19], wherein the step (a) above of obtaining an analytical sample from the sample by pretreatment includes a step of using lysyl endopeptidase (also referred to as "Lys-C"), a common mass-to-charge ratio shared by the yak species, the cashmere species, and the sheep species is 1 or more mass-to-charge ratios selected from the following mass-to-charge ratios:

(1) the mass-to-charge ratio of molecular ions calculated from the mass of 1070.5 ± 0.02%;
(2) the mass-to-charge ratio of molecular ions calculated from the mass of 1555.76 ± 0.02%;

(3) the mass-to-charge ratio of molecular ions calculated from the mass of 1160.58 ± 0.02%; and

(4) the mass-to-charge ratio of molecular ions calculated from the mass of 602.30 ± 0.02%.

[25] A method for quantifying animal hair in a sample, comprising the steps of:

(a) obtaining an analytical sample from the sample by pretreatment;

(b) adding an animal hair marker containing a stable isotope to the analytical sample obtained in step (a) above, analyzing the analytical sample using mass spectrometry and then obtaining a result;

(c) calculating the peak area of animal-specific mass-to-charge ratios and the peak area of the mass-to-charge ratios of the animal hair marker containing the stable isotope, and then calculating the peak area of the mass-to-charge ratios of the animal hair marker in terms of concentration or amount based on the concentration of the animal hair marker added to the analytical sample; and

(d) quantifying animal hair contained in an extracted fluid sample through comparison with the peak area derived from the extracted fluid sample.

[26] The method according to [25], wherein step (a) above of obtaining an analytical sample from the sample by pretreatment includes a step of using lysyl endopeptidase (also referred to as "Lys-C") and the animal hair marker is any one of those described in [14].

B. The present invention further includes the following [1] to [23].

[1] A method for specifying animal species of animal hair and/or quantifying the animal hair contained in a sample,

(a) obtaining an analytical sample from the sample by pretreatment,

(b) analyzing the analytical sample obtained in step (a) above using mass spectrometry and then obtaining a result; and

(c) specifying animal species of animal hair and/or quantifying the animal hair based on the result obtained in step (b) above.

[2] The method according to [1], wherein, in addition to the mass spectrometry, liquid chromatography is used in combination with mass spectroscopy (also referred to as "LC-MS" or "UPLC-MS").

[3] The method according to [1] or [2], wherein the step of obtaining an analytical sample from the sample by pretreatment, includes a step of using lysyl endopeptidase (also referred to as "Lys-C").

[4] The method according to any one of [1] to [3], further comprising the step of specifying animal species of animal hair and/or quantifying animal hair by comparing the result obtained in step (b) above with at least one of reference data obtained by performing steps (a) and (b) above under the same conditions for a reference animal hair sample of which animal species have been confirmed in advance, and thus specifying animal species of animal hair and/or quantifying an animal hair based on the masses, and/or the mass-to-charge ratios (also referred to as "m/z"), and/or the elution times of the relevant mass-to-charge ratios, detected specifically to the animal species of animal hair.

[5] The method according to any one of [1] to [4], wherein the animal species is one or more species selected from the group consisting of a cashmere species of the genus *Capra* of the family Bovidae, an Angora species of the genus *Capra* of the family Bovidae, a yak species of the genus *Bos* of the family Bovidae, a sheep species of the genus *Ovis* of the family Bovidae, the genus *Camelus* of the family Camelidae, an alpaca species of the genus *Lama* of the family Camelidae (or an alpaca species of the genus *Vicugna),* and an Angora rabbit species of the genus *Oryctolagus* of the family Leporidae.

[6] The method according to [1] or [2], wherein in the step of specifying animal species of animal hair and/or quantifying animal hair, the animal species of animal hair is specified and/or animal hair is quantified based on

masses detected specifically to a yak species such as

5948.90 ± 0.02%, and 2137.92 ± 0.02%, and/or

Mass-to-charge ratios (also referred to as "m/z") detected specifically to the yak species such as

m/z=9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, and 1069.98 ± 0.02% including the mass-to-charge ratios of molecular ions resulting from the masses, and/or

the elution times of the relevant mass-to-charge ratios.

[7] The method according to [1] or [2], wherein in the step of specifying animal species of animal hair and/or quantifying an animal hair, the animal species of the animal hair is specified and/or the animal haire is quantified based on

masses detected specifically to a cashmere species such as

8976.45 ± 0.02%, 7102.18 ± 0.02%, and 7074.18 ± 0.02%, and/or

Mass-to-charge ratios (also referred to as "m/z") detected specifically to the cashmere species such as

m/z= 10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, and 1415.83 ± 0.02% including the mass-to-charge ratios of molecular ions resulting from the masses, and/or

the elution times of the relevant mass-to-charge ratios.

[8] The method according to [1] or [2], wherein in the step of specifying animal species of animal hair and/or quantifying an animal hair, the animal species of animal hair is specified and/or animal hair is quantified based on masses detected specifically to a sheep species such as

17538.88 ± 0.02%, and 17584.26 ± 0.02%, and/or

Mass-to-charge ratios (also referred to as "m/z") detected specifically to the cashmere species such as

m/z = 10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, and 1599.55 ± 0.02% including the mass-to-charge ratios of molecular ions resulting from the masses, and/or

the elution times of the relevant mass-to-charge ratios.

[9] The method according to [1] or [2], wherein in the step of specifying animal species of animal hair and/or quantifying an animal hair, the animal species of animal hair is specified and/or animal haire is quantified based on a mass-to-charge ratio (also referred to as "m/z") detected specifically to an Angora species (also referred to as "mohair") such as the mass-to-charge ratio of m/z = 1055.92 ± 0.02%, and/or the elution time of the relevant mass-to-charge ratio.

[10] The method according to [1] or [2], wherein in the step of specifying animal species of animal hair and/or quantifying an animal hair, the animal species of animal hair is specified and/or animal hair is quantified based on a mass-to-charge ratio (also referred to as "m/z") detected specifically to an alpaca species, such as the mass-to-charge ratio of m/z = 1272.72 ± 0.02% or 1269.54 ± 0.02%, and/or the elution time of the relevant mass-to-charge ratio.

[11] The method according to [1] or [2], wherein in the step of specifying animal species of animal hair and/or quantifying an animal hair, the animal species of animal hair is specified and/or animal hair is quantified based on a mass-to-charge ratio (also referred to as "m/z") detected specifically to an Angora rabbit species (also referred to as "Angora") such as the mass-to-charge ratio of m/z = 1043.43 ± 0.02%, and/or

the elution time of the relevant mass-to-charge ratio.

[12] The method according to [3], wherein in the step of specifying animal species of animal hair and/or quantifying an animal hair, the animal species of animal hair is specified and/or animal haire is quantified based on yak-species-specific masses (M) such as

1364.70 ± 0.02%, 1383.72 ± 0.02%, 1431.67 ± 0.02%, 2469.27 ± 0.02%, and 2495.30 ± 0.02%, or

the mass-to-charge ratios of molecular ions resulting from molecules having the yak-species-specific masses, and/or

the elution times of the relevant mass-to-charge ratios.

[13] The method according to [3], wherein in the step of specifying animal species of animal hair and/or quantifying an animal hair, the animal species of animal hair is specified and/or animal haire is quantified based on cashmere-species-specific masses (M) such as

2218.19 ± 0.02%, 3379.48 ± 0.02%, and 2391.99 ± 0.02%, or

the mass-to-charge ratios of molecular ions resulting from molecules having the cashmere-species-specific masses, and/or

the elution times of the relevant mass-to-charge ratios.

[14] The method according to [3], wherein in the step of specifying animal species of animal hair and/or quantifying an animal hair, the animal species of animal hair is specified and/or animal haire is quantified based on sheep-species-specific masses (M) such as

2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, and 2174.00 ± 0.02%, or

the mass-to-charge ratios of molecular ions resulting from molecules having the sheep-species-specific masses, and/or

the elution times of the relevant mass-to-charge ratios.

[15] The method according to [3], wherein in the step of specifying animal species of animal hair and/or quantifying an animal hair, the animal species of animal hair is specified and/or animal hair is quantified based on any one of or a combination of 2 or more yak-species-specific mass-to-charge ratios of m/z = 1365.7 ± 0.02%, 683.35 ± 0.02%, and 455.90 ± 0.02%, and/or

the elution times of the relevant mass-to-charge ratios.

[16] The method according to [15], wherein the yak-species-specific mass-to-charge ratio is a mass-to-charge ratio of molecular ions resulting from a peptide and/or a protein comprising the amino acid sequence represented by SEQ ID NO: 1: GLNNRFAAFID K.

[17] The method according to [15], wherein the yak-species-specific mass-to-charge ratios are mass-to-charge ratios of molecular ions resulting from a protein that is expressed by a gene comprising the DNA sequence represented by SEQ ID NO: 2: GGT CTC AAC AAC AGG TTT GCT GCC TTC ATC GAC AAG GTG CGC.

[18] The method according to [3], wherein in the step of specifying animal species of animal hair and/or quantifying an animal hair, the animal species of animal hair is specified and/or animal hair is quantified based on any one of or a combination of 2 or more cashmere-species-specific mass-to-charge ratios of m/z = 2219.19 ± 0.02%, 1110.10 ± 0.02%, 740.40 ± 0.02%, and 555.55 ± 0.02%, and/or

the elution times of the relevant mass-to-charge ratios.

[19] The method according to [18], wherein the cashmere-species-specific mass-to-charge ratios are mass-to-charge ratios of molecular ions resulting from a peptide and/or a protein comprising the amino acid sequence represented by SEQ ID NO: 3: SD (L or I) EA (Q or K) VES (L or I) (Q or K) M (Q or K) FDVRPK.

[20] The method according to [3], wherein in the step of specifying animal species of animal hair and/or quantifying an animal hair, the animal species of animal hair is specified and/or animal hair is quantified based on any one of or a combination of 2 or more sheep-species-specific mass-to-charge ratios of m/z = 2480.30 ± 0.02%, 1240.65 ± 0.02%, 827.43 ± 0.02%, 620.82 ± 0.02%, and 496.86 ± 0.02%, and/or the elution times of the relevant mass-to-charge ratios.

[21] The method according to [20], wherein the sheep-species-specific mass-to-charge ratios are mass-to-charge ratios of molecular ions resulting from a peptide and/or a protein containing 2 or more amino acid sequences represented by SEQ ID NOS: 4 to 9,

SEQ ID NO: 4: QF (L or I),
SEQ ID NO: 5: FENEY,
SEQ ID NO: 6: (Q or K) NFVA (L or I) (Q or K),
SEQ ID NO: 7: (L or I) FQ,
SEQ ID NO: 8: YENEF, and
SEQ ID NO: 9: (Q or K) (L or I) AVFN (Q or K).

[22] The method according to [3], wherein in the step of specifying animal species of animal hair and/or quantifying an animal hair, the animal species of animal hair is specified and/or anima hair is quantified based on

any one of or a combination of 2 or more sheep-species-specific mass-to-charge ratios of m/z = 2075.12 ± 0.02%, 1038.06 ± 0.02%, 692.37 ± 0.02%, 519.53 ± 0.02%, and 415.82 ± 0.02%, and/or

the elution times of the relevant mass-to-charge ratios.

[23] The method according to [22], wherein the sheep-species-specific mass-to-charge ratios are mass-to-charge ratios of molecular ions resulting from a peptide and/or a protein containing 1 or more amino acid sequences represented by SEQ ID NOS: 10 to 13,

SEQ ID NO: 10: SD (L or I) E,
SEQ ID NO: 11: (Q or K) EE (L or I) (L or I) C (L or I) K,
SEQ ID NO: 12: E (L or I) DS, and
SEQ ID NO: 13: K (L or I) C (L or I) (L or I) EE (Q or K).

This description includes all or part of the contents as disclosed in the description and/or drawings of Japanese Patent Application No. 2012-089662, from which the present application claims the priority.

Brief Description of the Drawings

Fig. 1 shows the results of analyzing animal hair extracts by mass spectroscopy (MALDI-TOF mass spectroscope). In addition, a cashmere species is denoted as "cashmere", a yak species is denoted as "yak" and a sheep species is denoted as "sheep".

Fig. 2-1 shows the ion chromatogram obtained by integration of the yak-species-specific mass-to-charge ratios of m/z = 1983.52 ± 0.02% and 1487.64 ± 0.02% for each animal hair extract.

Fig. 2-2 shows the ion chromatogram of the yak-species-specific mass-to-charge ratio of m/z = 1069.98 ± 0.02% for each animal hair extract.

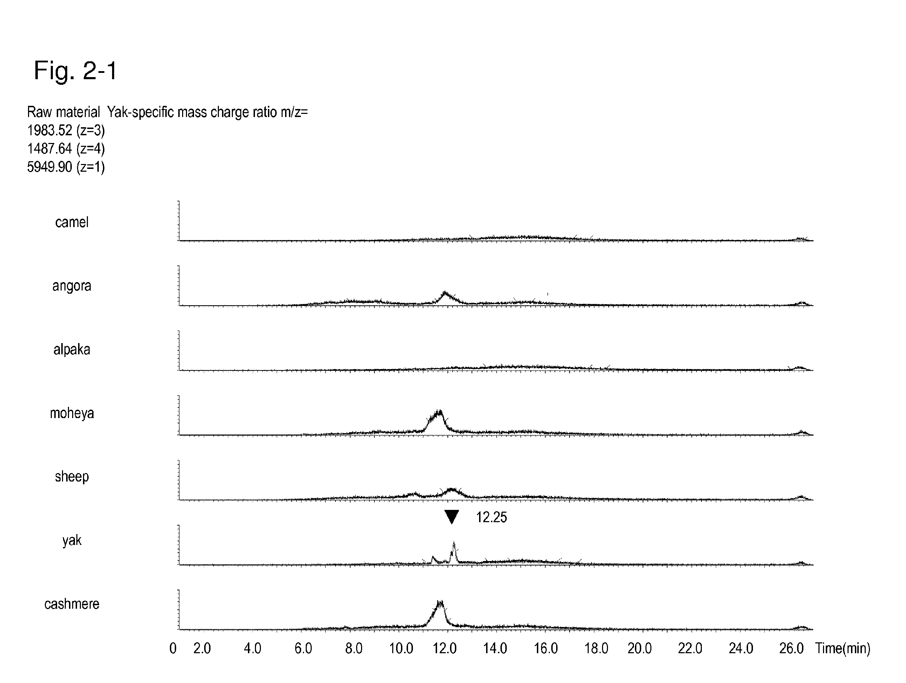

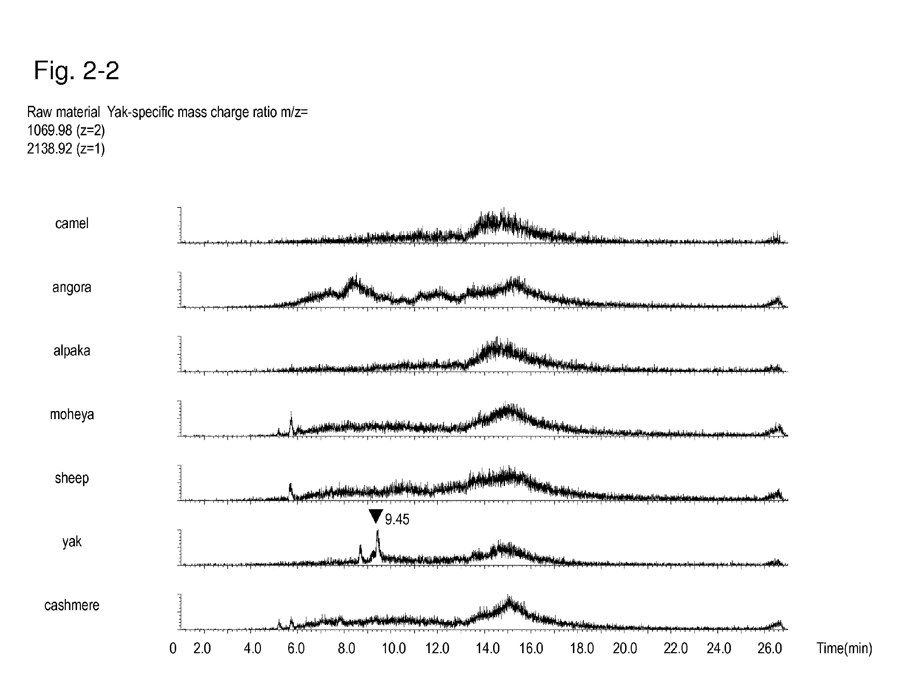

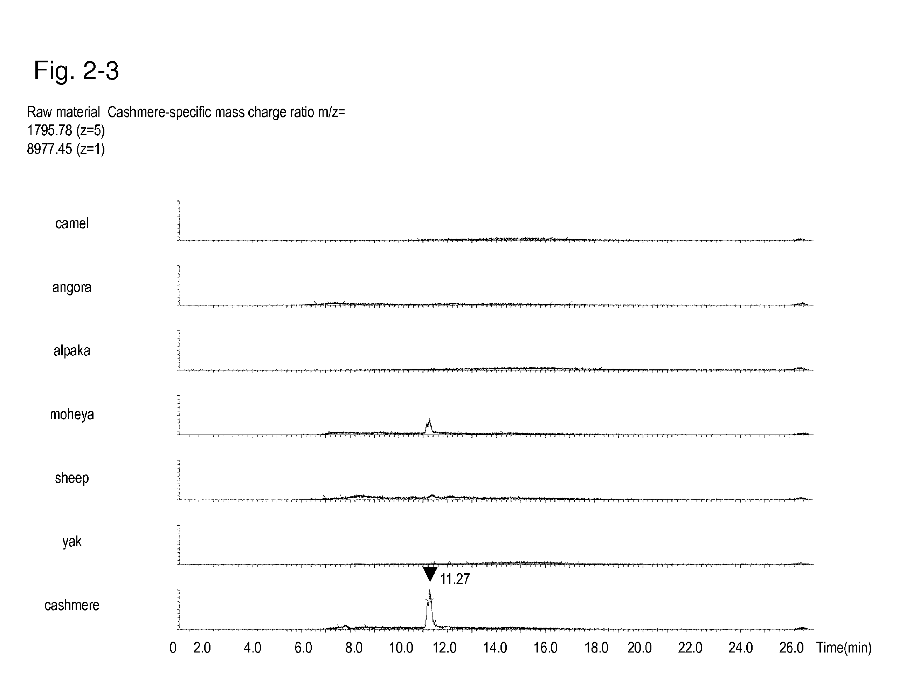

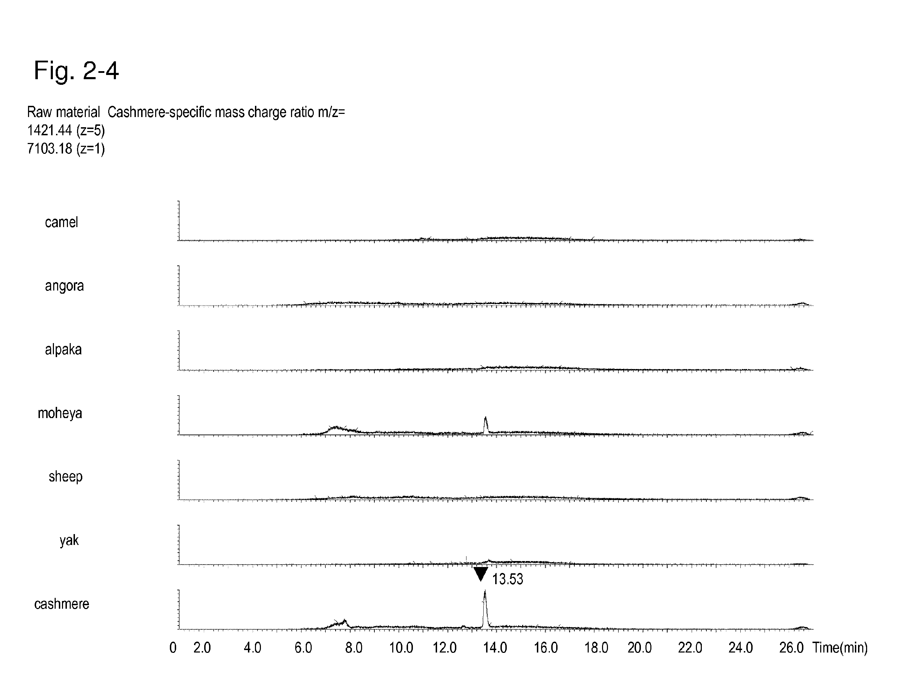

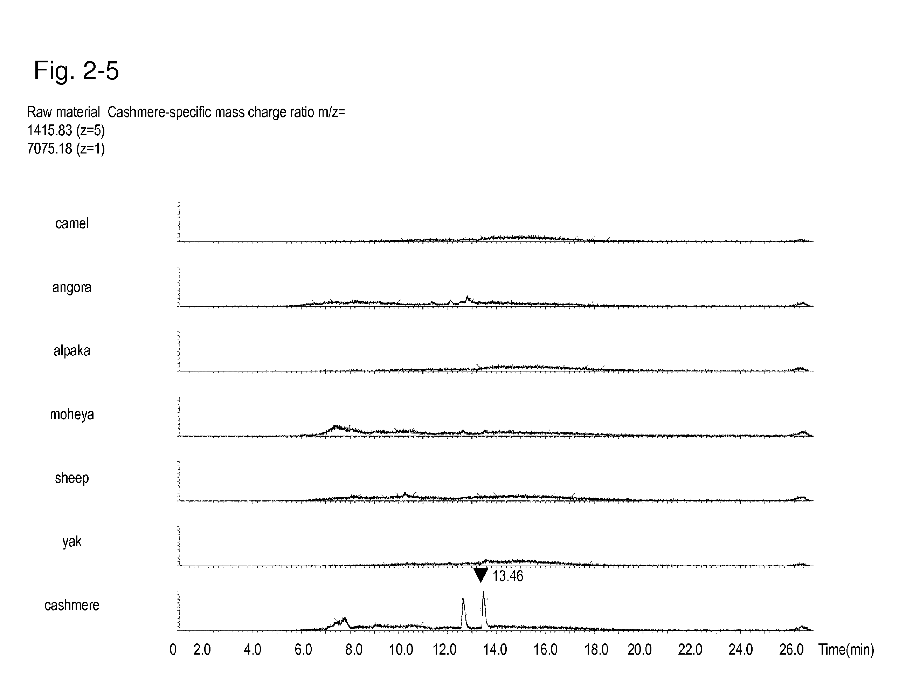

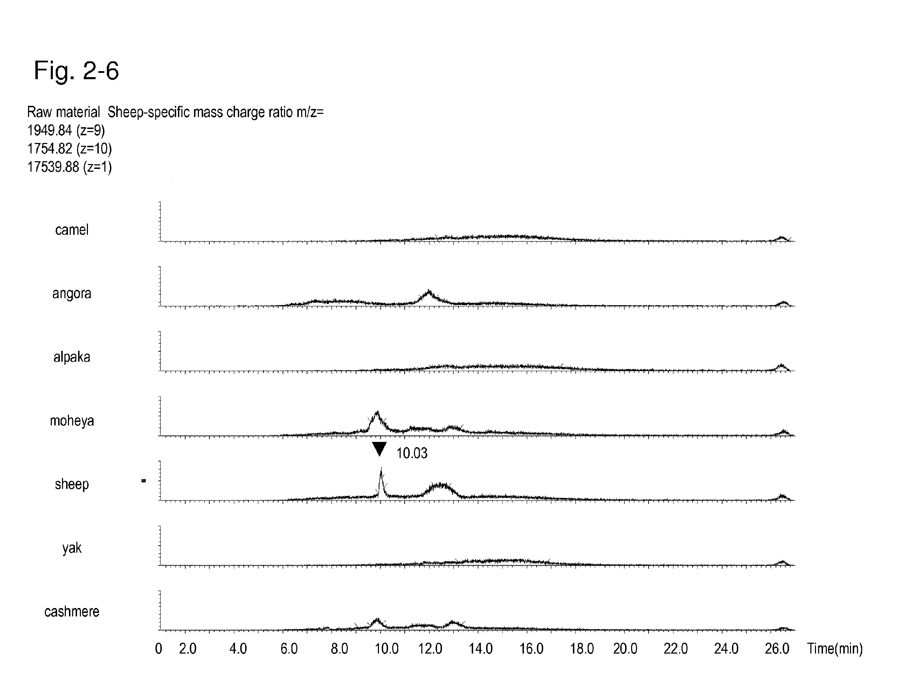

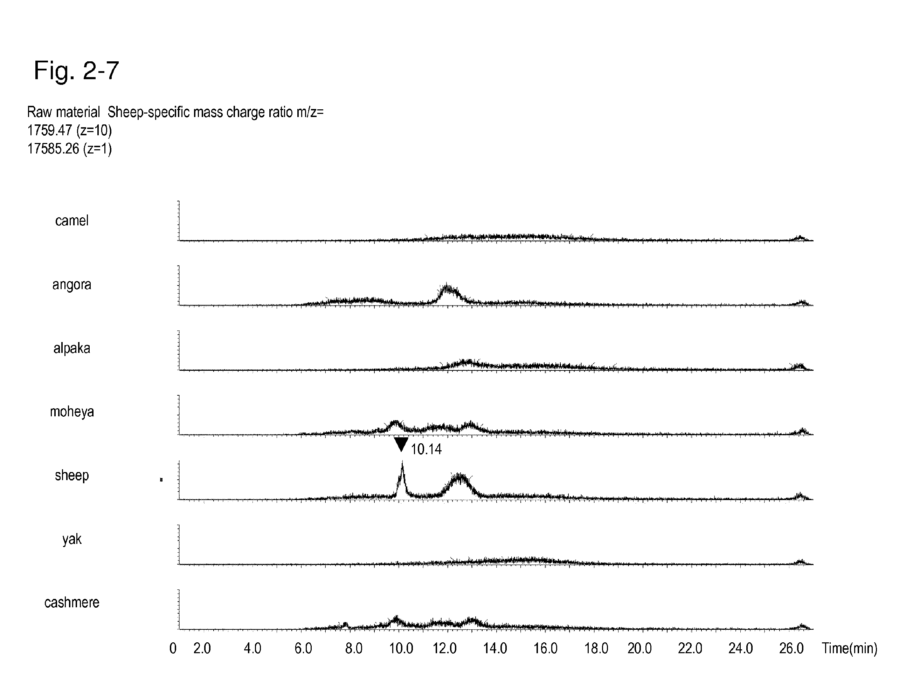

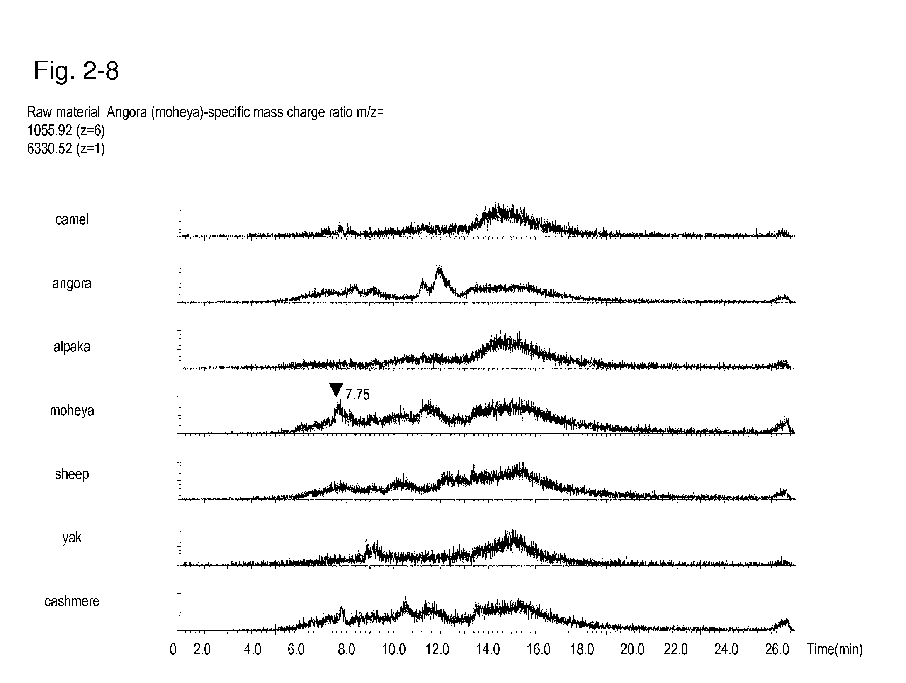

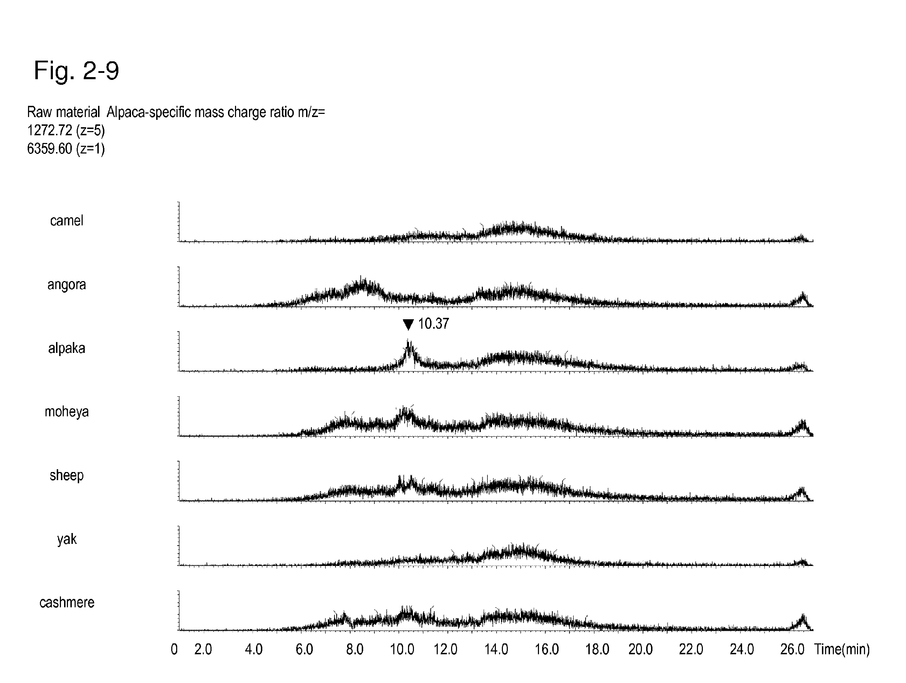

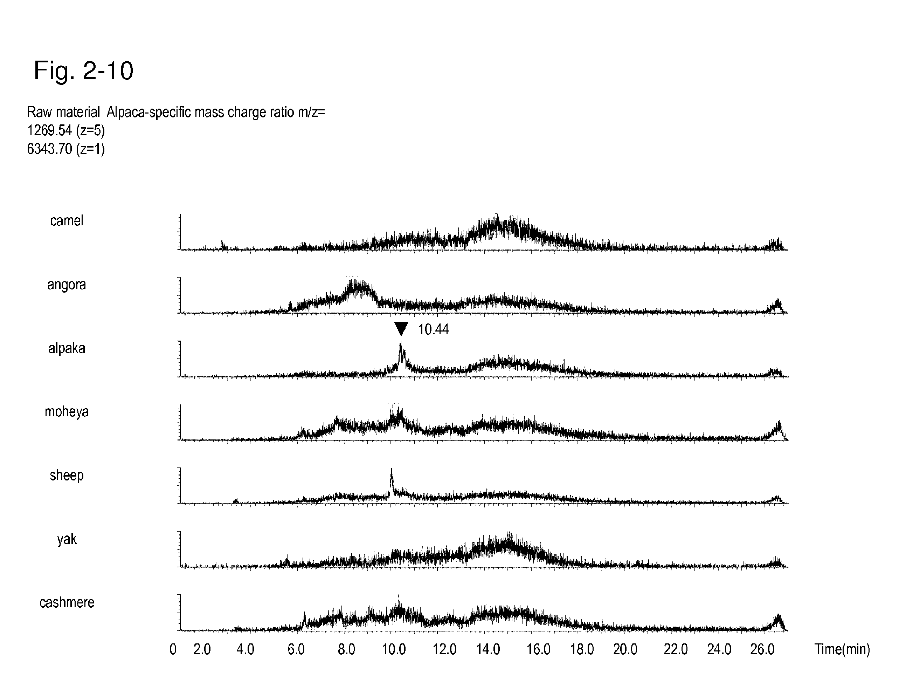

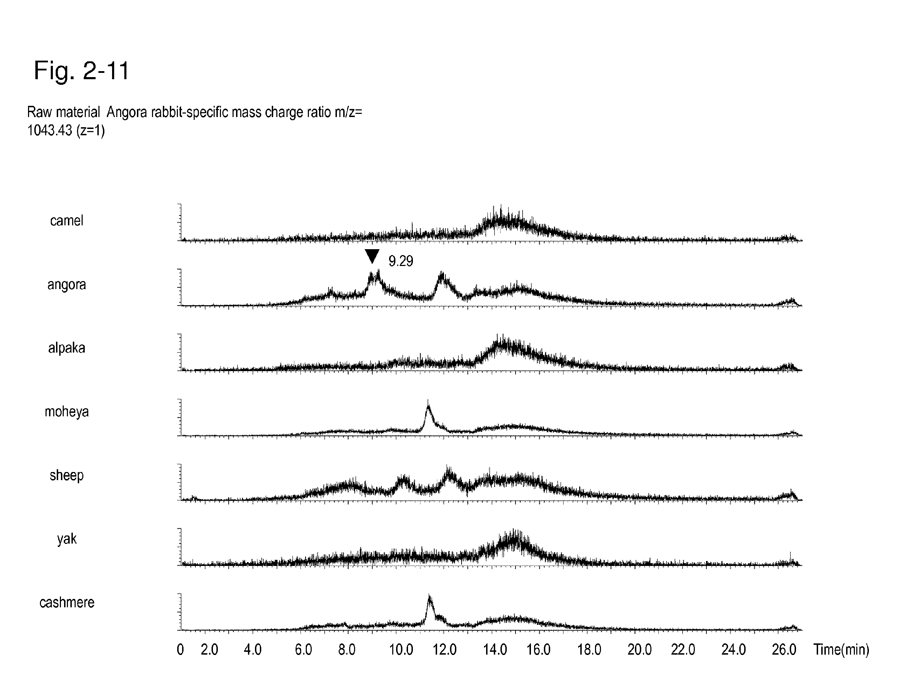

Fig. 2-3 shows the ion chromatogram of the cashmere-species-specific mass-to-charge ratio of m/z = 1795.78 ± 0.02% for each animal hair extract.

Fig. 2-4 shows the ion chromatogram of the cashmere-species-specific mass-to-charge ratio of m/z = 1421.44 ± 0.02% for each animal hair extract.

Fig. 2-5 shows the ion chromatogram of the cashmere-species-specific mass-to-charge ratio of m/z = 1415.83 ± 0.02% for each animal hair extract.

Fig. 2-6 shows the ion chromatogram obtained by integration of the sheep-species-specific mass-to-charge ratios of m/z = 1754.82 ± 0.02% and 1949.84 ± 0.02% for each animal hair extract.

Fig. 2-7 shows the ion chromatogram of the sheep-species-specific mass-to-charge ratio of m/z = 1759.47 ± 0.02% for each animal hair extract.

Fig. 2-8 shows the ion chromatogram of the Angora-species-(mohair)-specific mass-to-charge ratio of m/z = 1055.92 ± 0.02% for each animal hair extract.

Fig. 2-9 shows the ion chromatogram of the alpaca-species-specific mass-to-charge ratio of m/z = 1272.72 ± 0.02% for each animal hair extract.

Fig. 2-10 shows the ion chromatogram of the alpaca-species-specific mass-to-charge ratio of m/z = 1269.54 ± 0.02% for each animal hair extract.

Fig. 2-11 shows the ion chromatogram of the Angora rabbit species-specific mass-to-charge ratio of m/z = 1043.43 ± 0.02% for each animal hair extract.

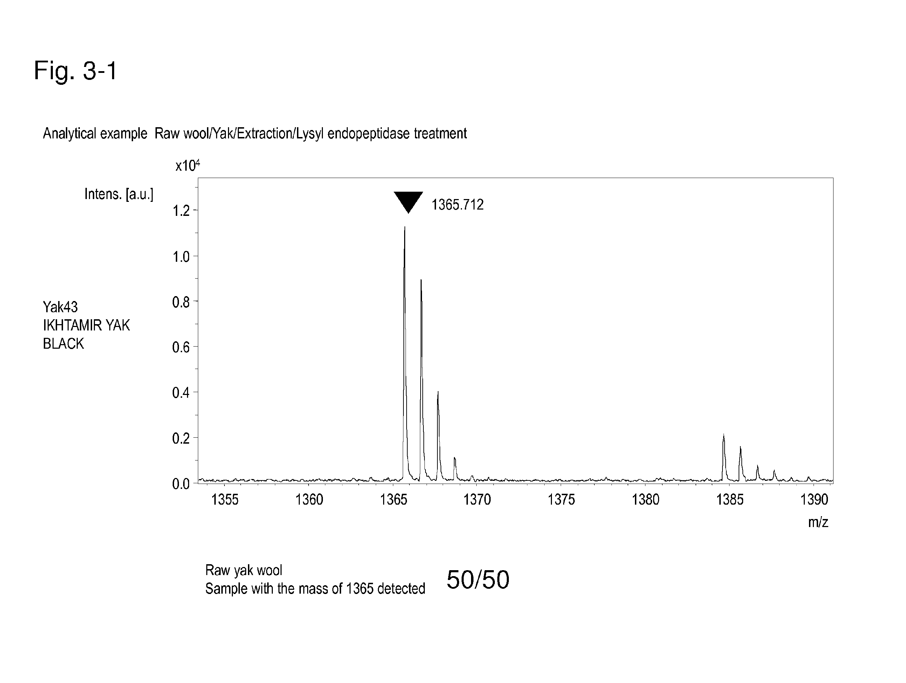

Fig. 3-1 is an enlarged view around the mass-to-charge ratio of m/z = 1365.7.

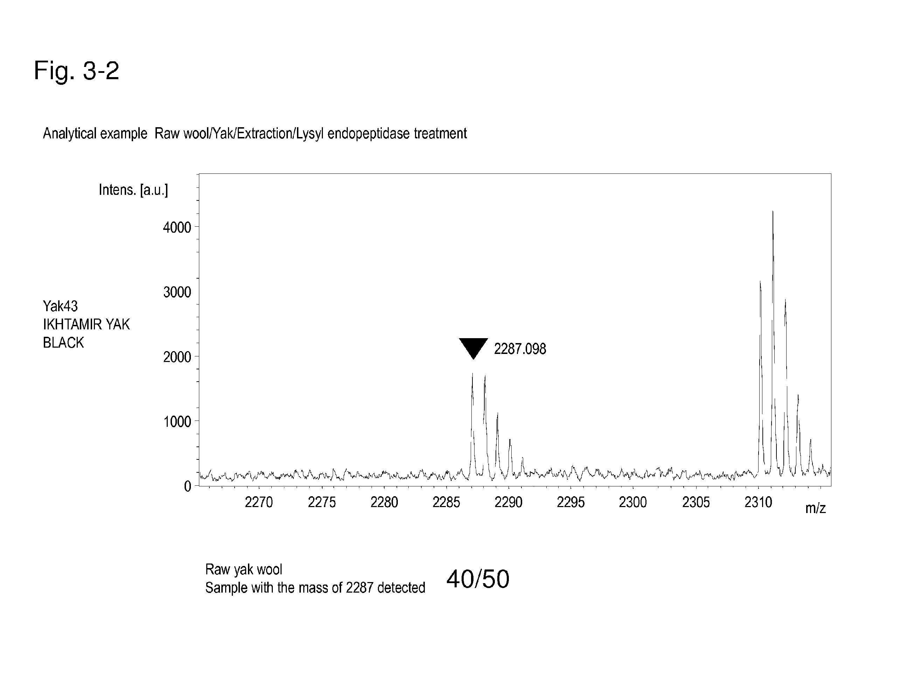

Fig. 3-2 shows an enlarged view around the mass-to-charge ratio of m/z = 2287.

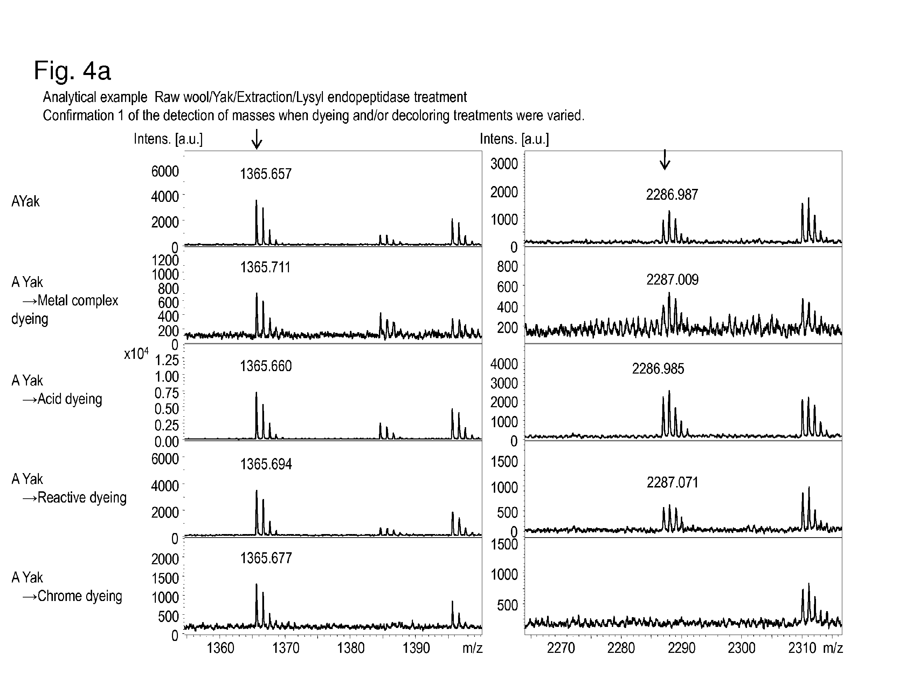

Fig. 4a shows the results of performing extraction from animal hair subjected to dyeing treatment or the like, treating extracts with an enzyme, and then performing mass spectroscopy 1.

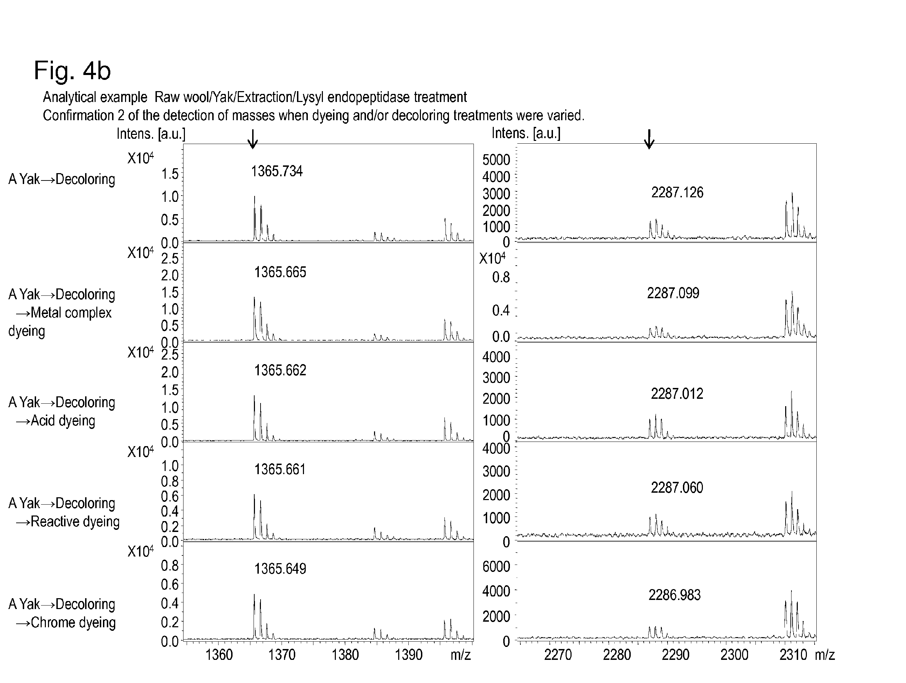

Fig. 4b shows the results of performing extraction from animal hair subjected to dyeing treatment or the like, treating extracts with an enzyme, and then performing mass spectroscopy 2.

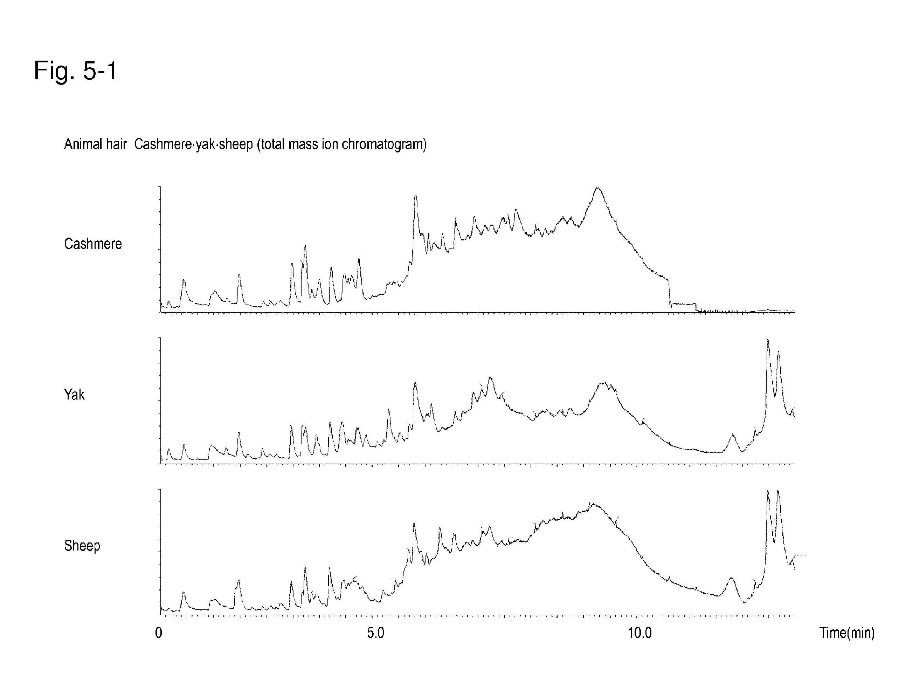

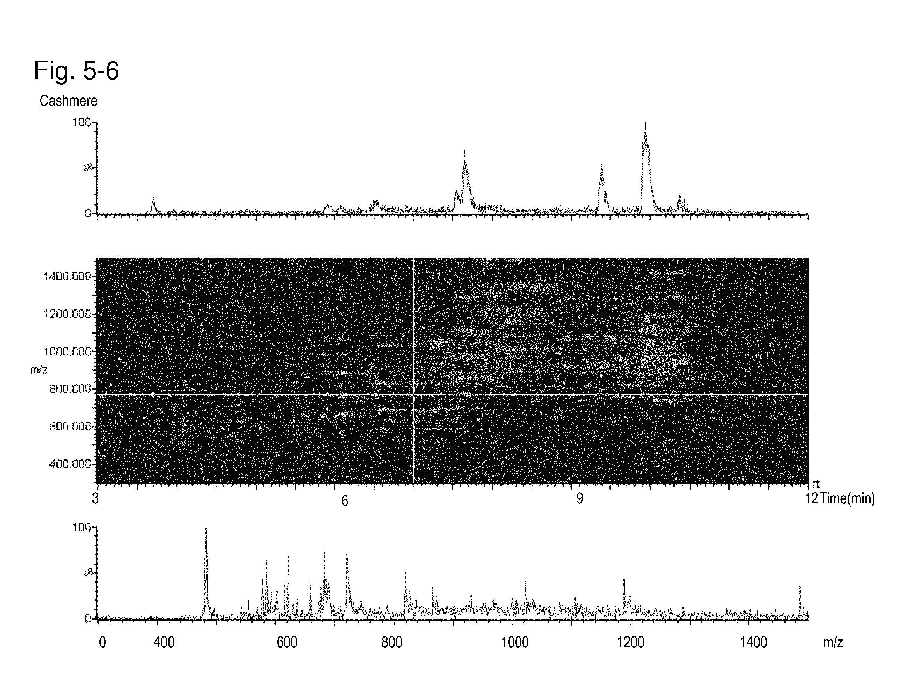

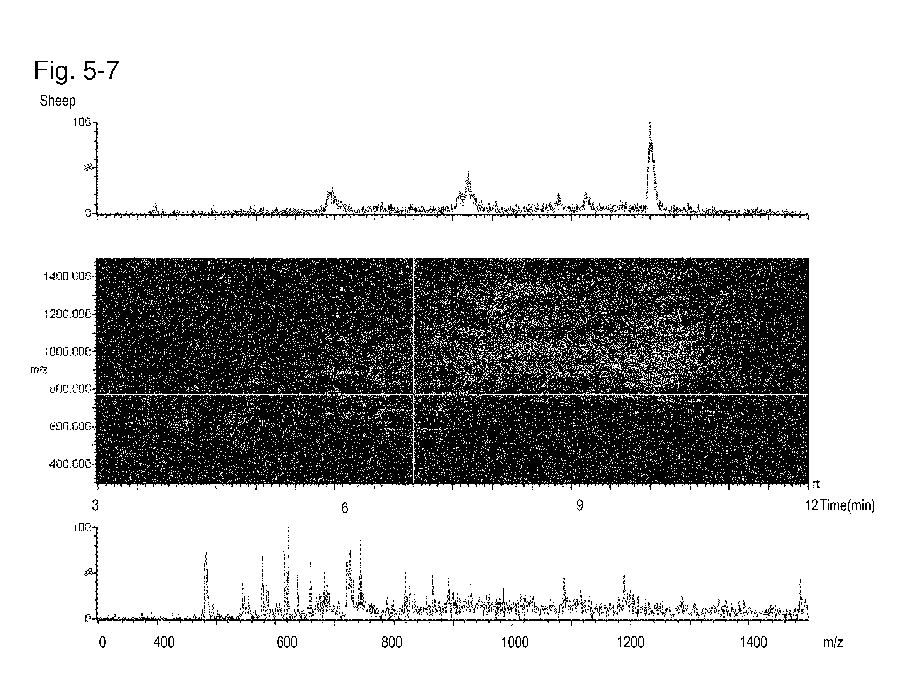

Fig. 5-1 shows the results of treating each animal hair extract with an enzyme and then performing mass spectroscopy (total mass chromatogram).

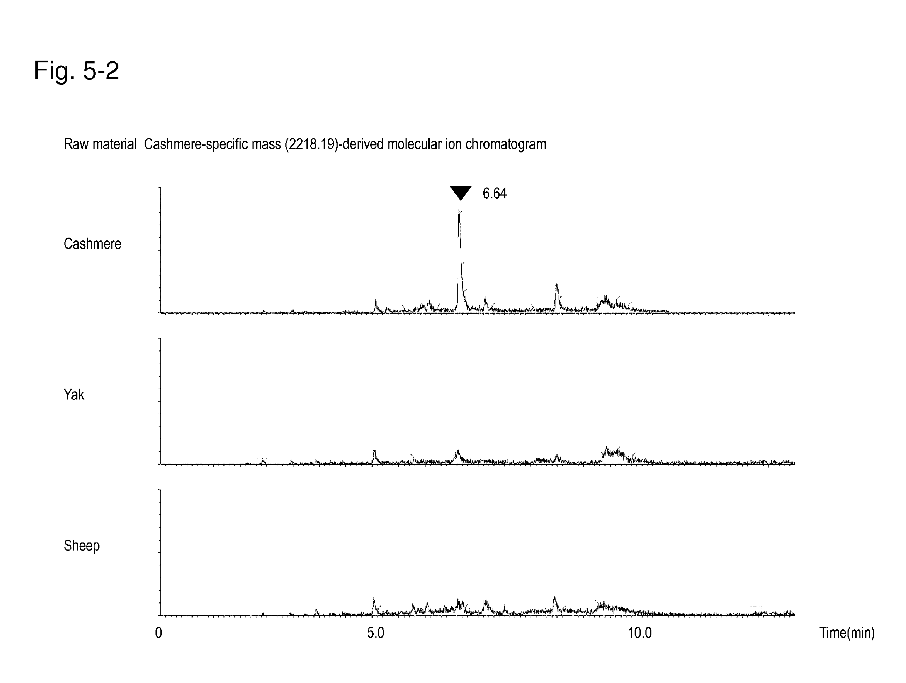

Fig. 5-2 shows the chromatogram of cashmere-specific masses in Fig. 5-1 above.

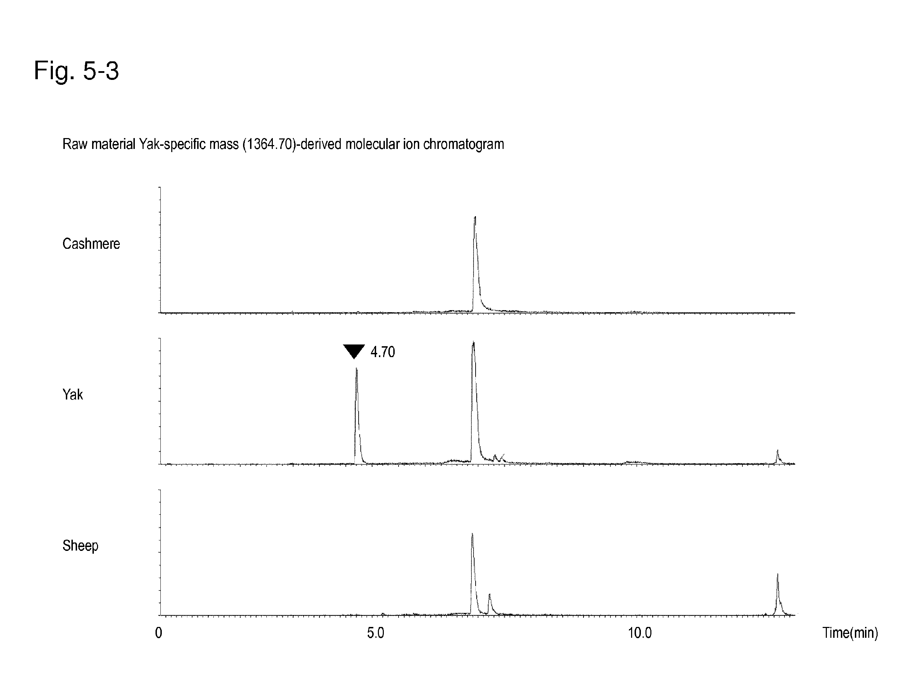

Fig. 5-3 shows the chromatogram derived from yak-specific masses in Fig. 5-1 above.

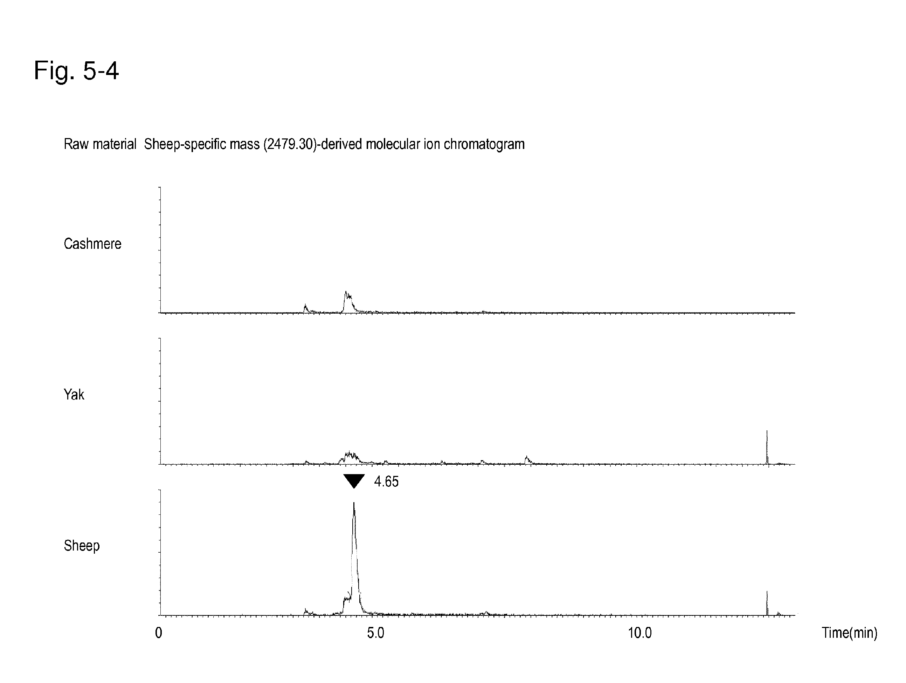

Fig. 5-4 shows the chromatogram derived from sheep-specific masses in Fig. 5-1.

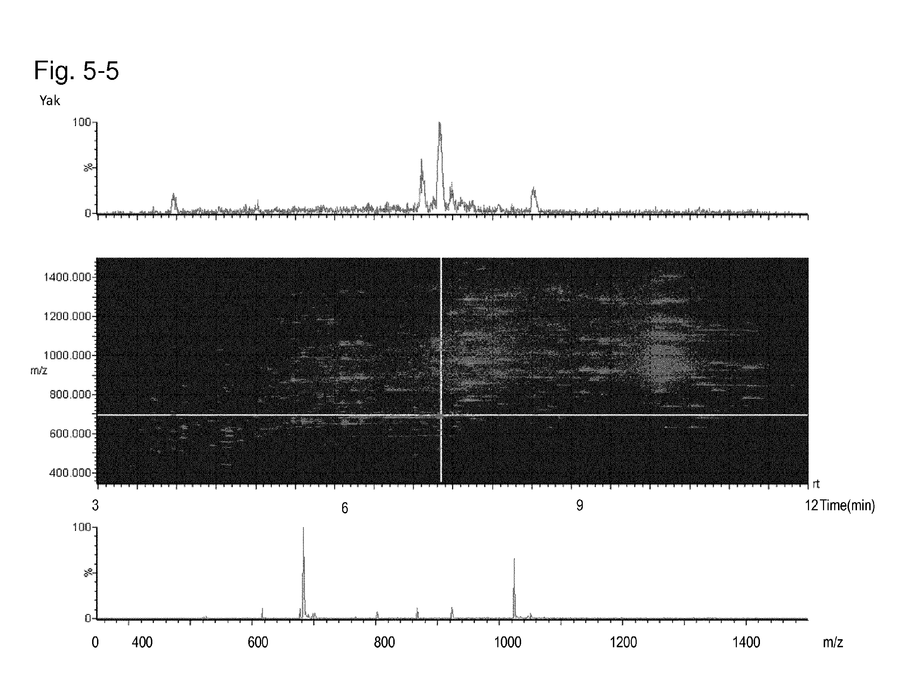

Fig. 5-5 is a map showing the results of treating an animal hair (yak) extract with an enzyme and then performing mass spectroscopy. The horizontal axis indicates the elution time, and the vertical axis indicates the mass distribution. Ion intensity becomes stronger as the color becomes red.

Fig. 5-6 is a map showing the results of treating an animal hair (cashmere) extract with an enzyme and then performing mass spectroscopy. The horizontal axis indicates the elution time, and the vertical axis indicates the mass distribution. Ion intensity becomes stronger as the color becomes red.

Fig. 5-7 is a map showing the results of treating an animal hair (sheep) extract with an enzyme and then performing mass spectroscopy. The horizontal axis indicates the elution time, and the vertical axis indicates the mass distribution. Ion intensity becomes stronger as the color becomes red.

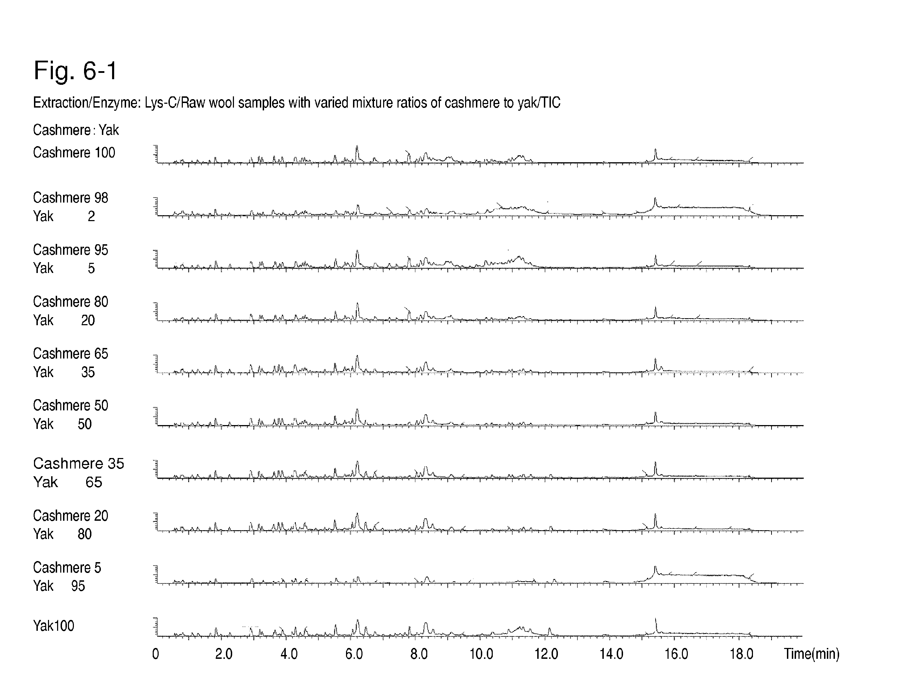

Fig. 6-1 shows the total mass ion chromatogram of each of samples with different mixture ratios of cashmere raw material to yak raw material.

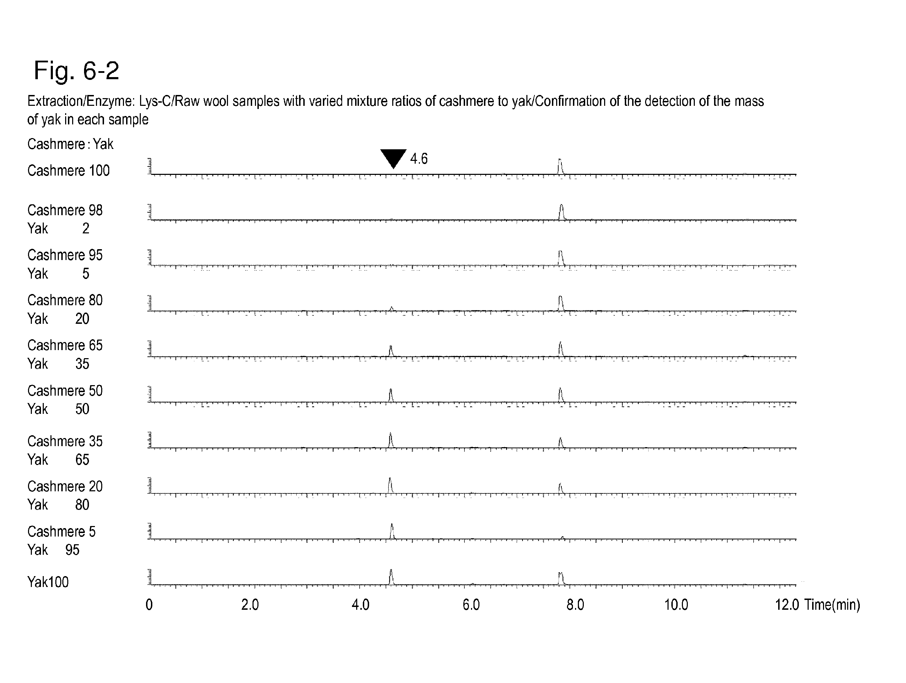

Fig. 6-2 shows the ion chromatogram of the yak-species-specific mass-to-charge ratio of m/z = 683.35 in Fig. 6-1 above. It is shown that detection is possible when the percentage of yak mixed therein is at least 2%.

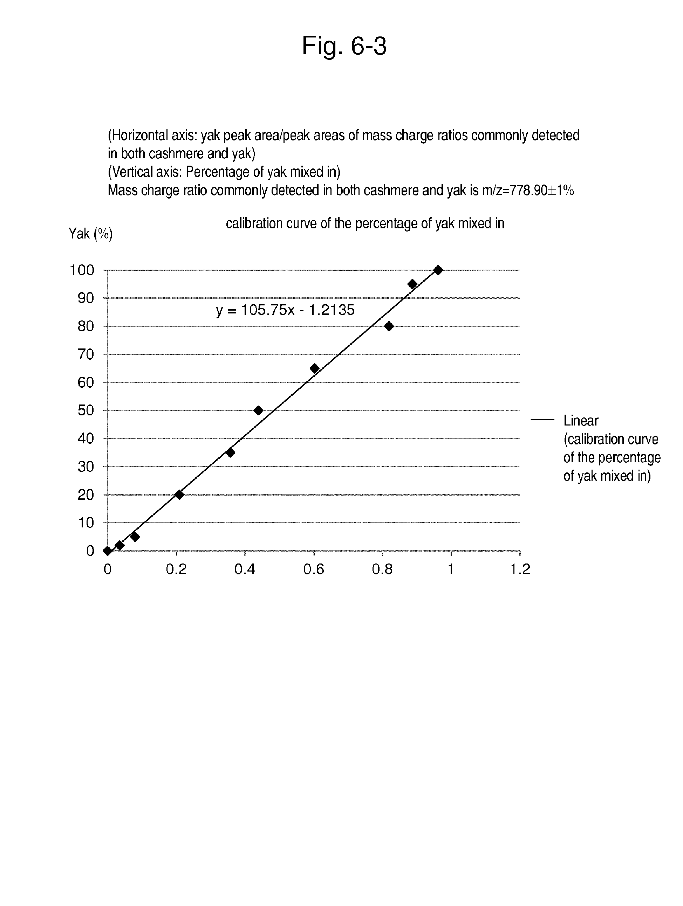

Fig. 6-3 shows the relationship between the peak area of yak-specific masses in Fig. 6-2 above and the mixture ratio.

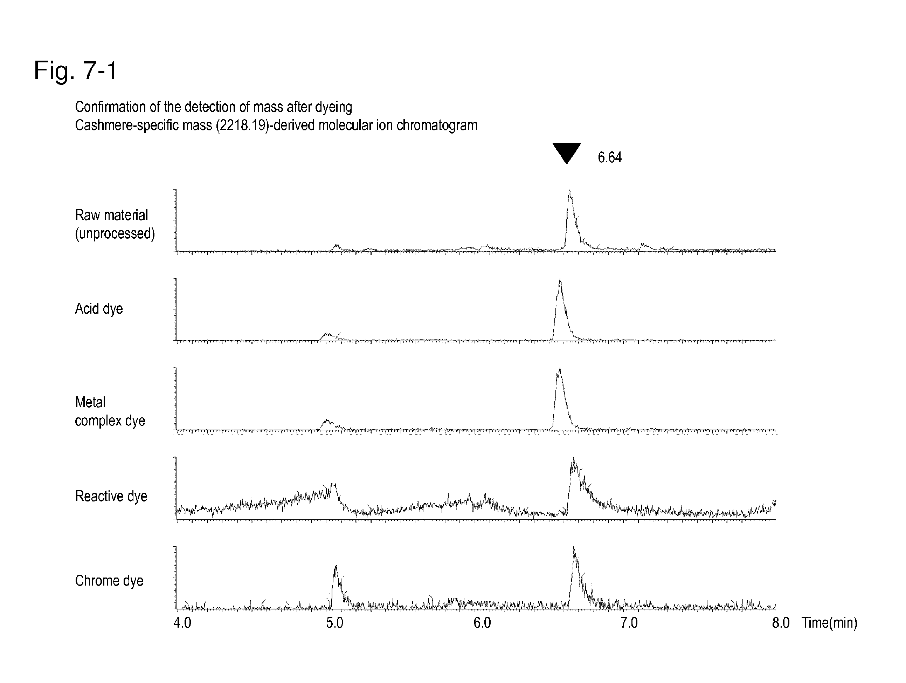

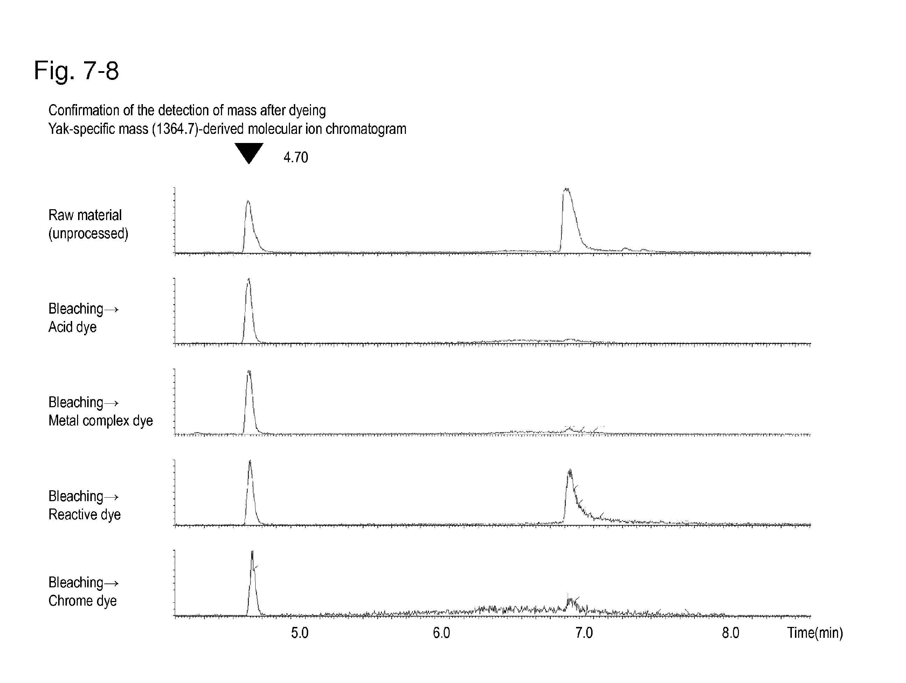

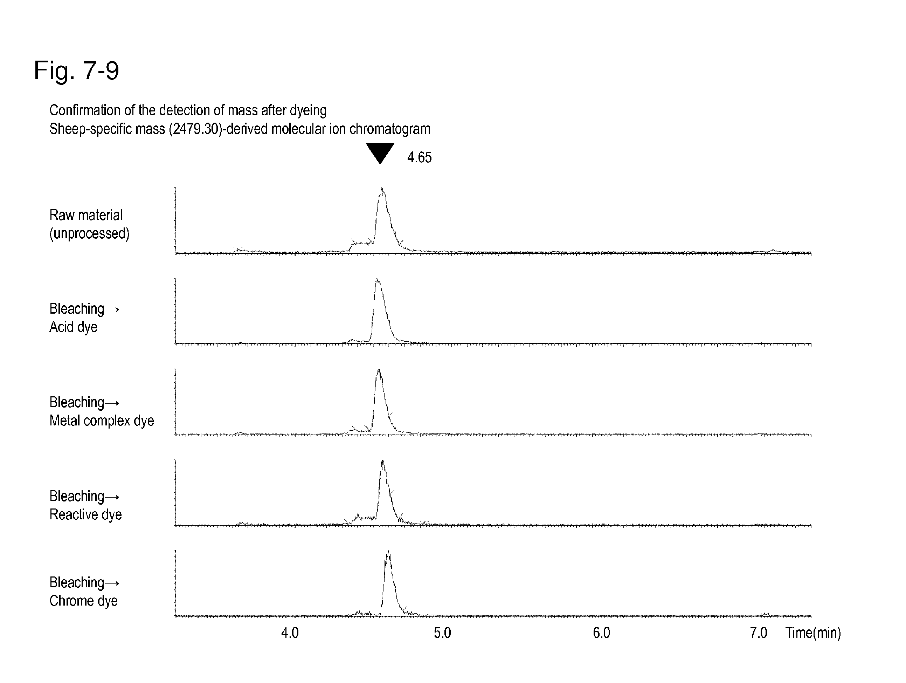

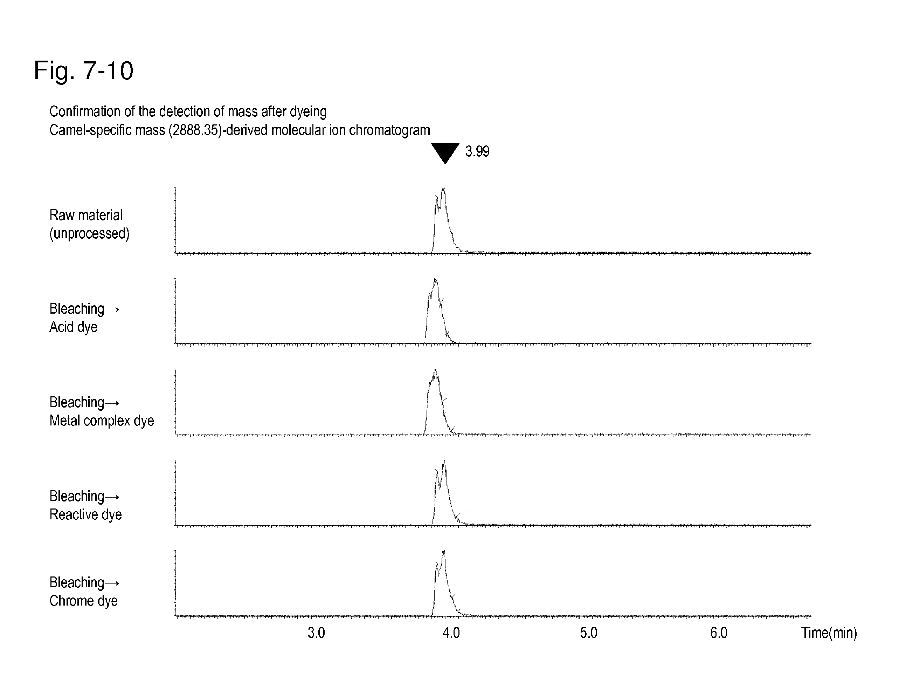

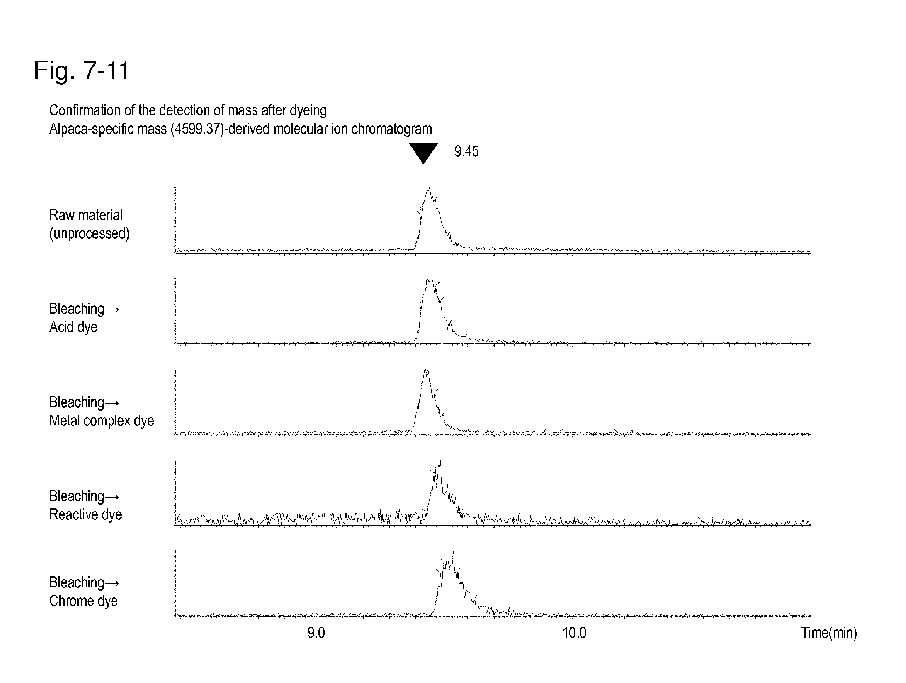

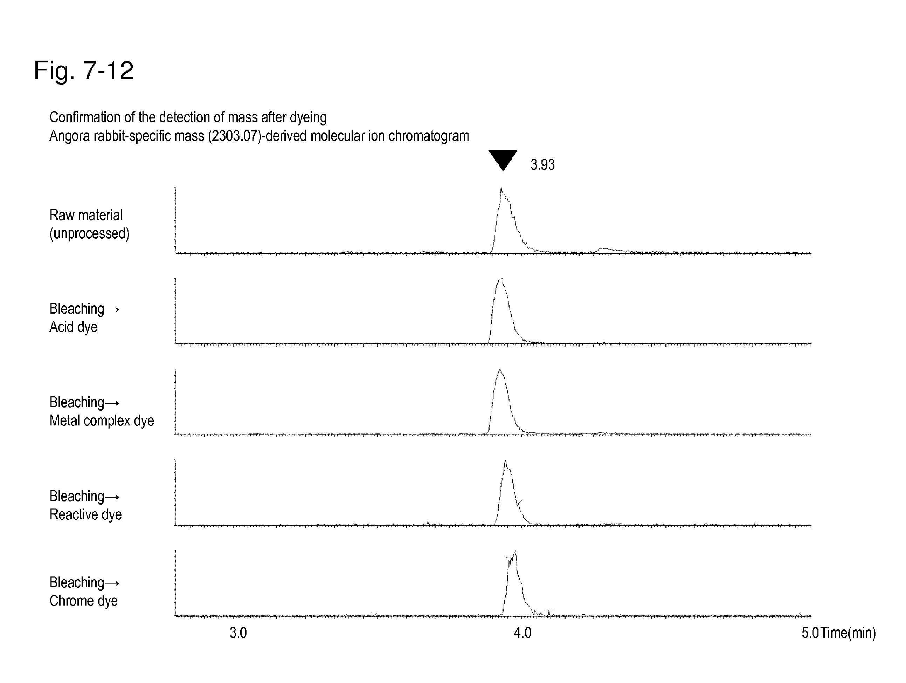

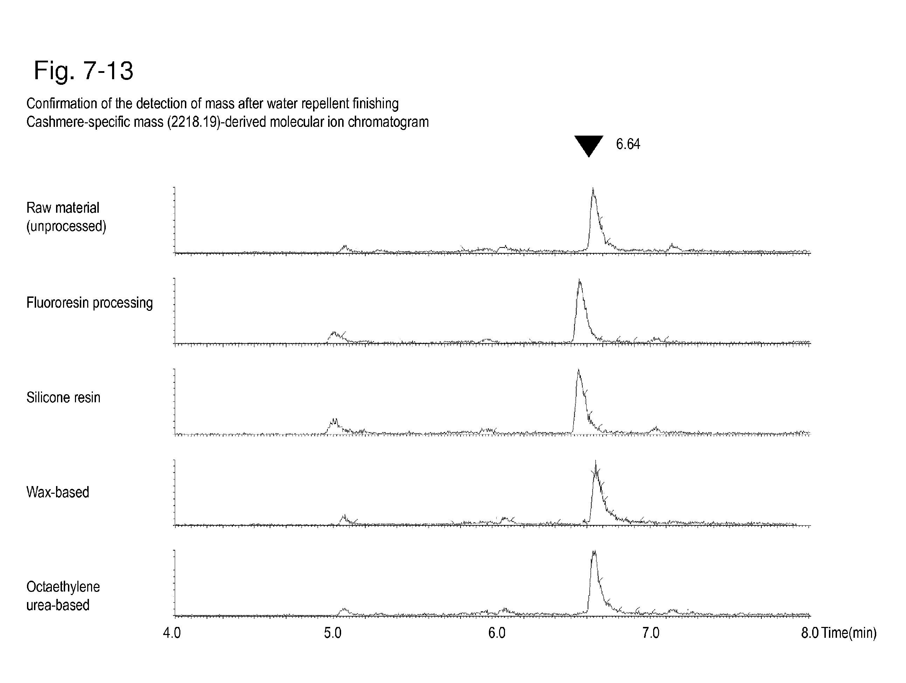

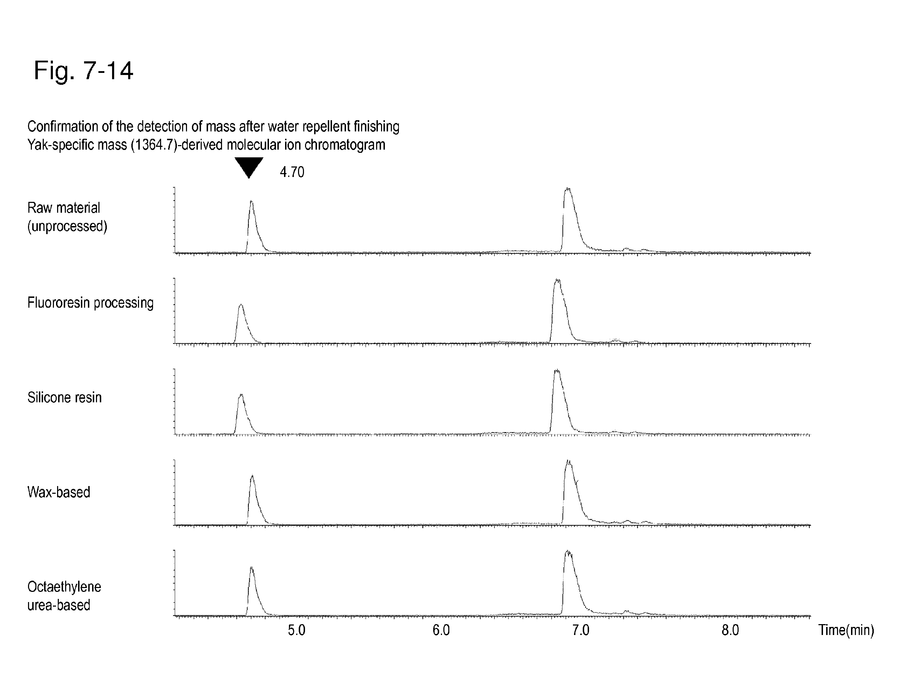

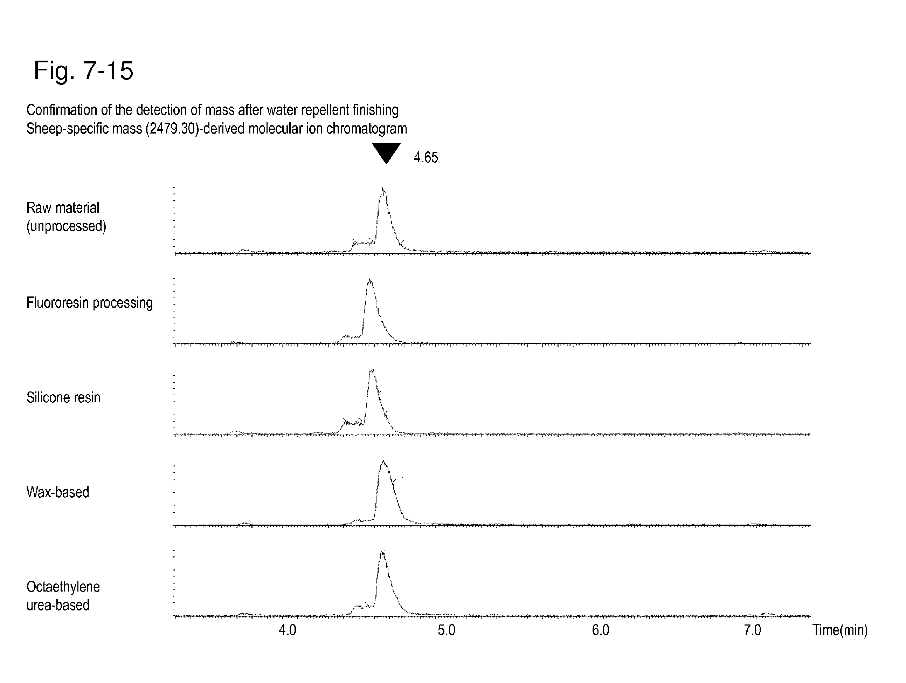

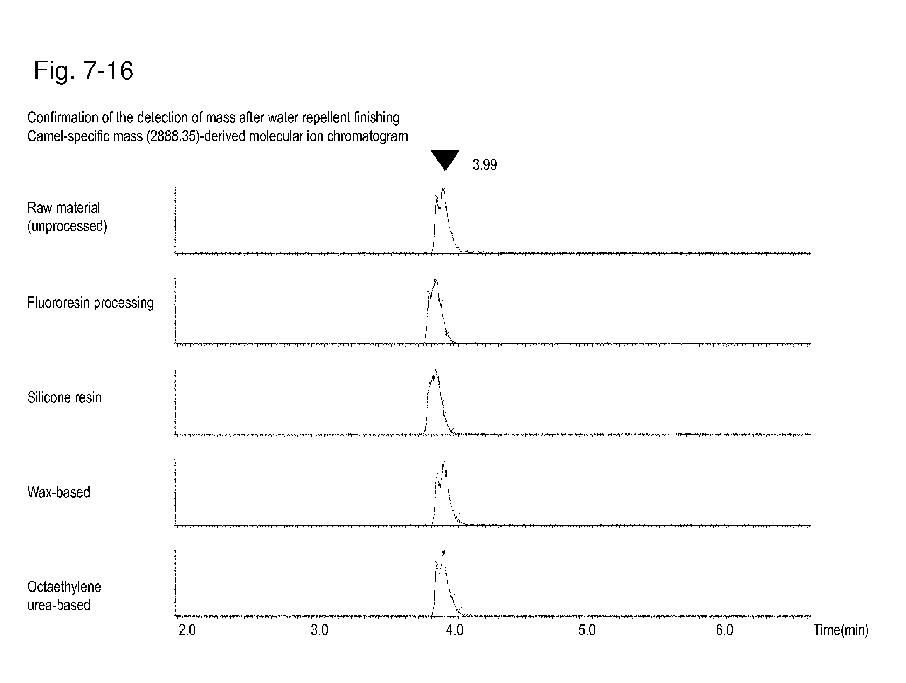

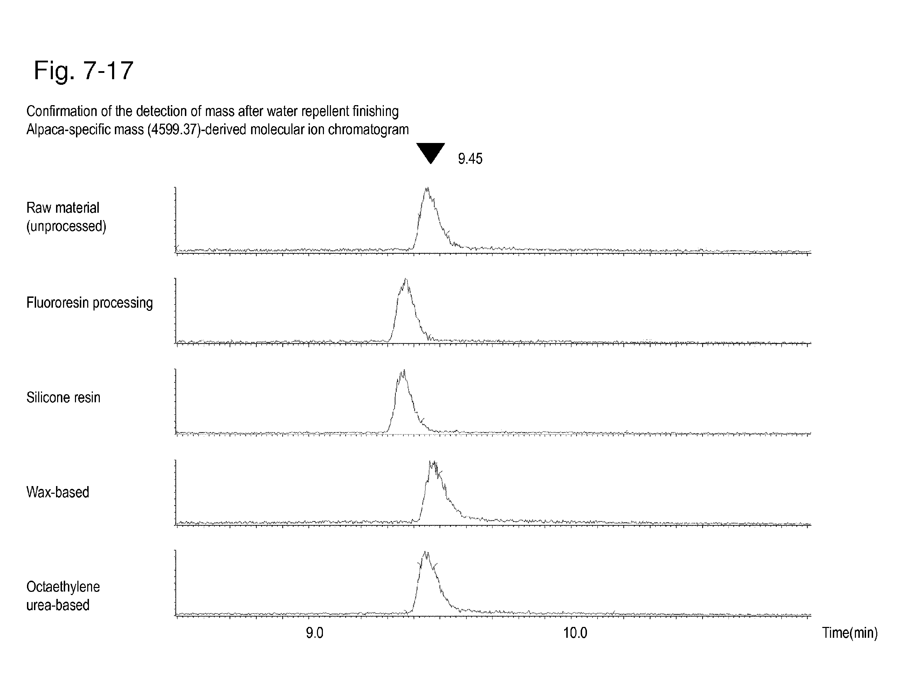

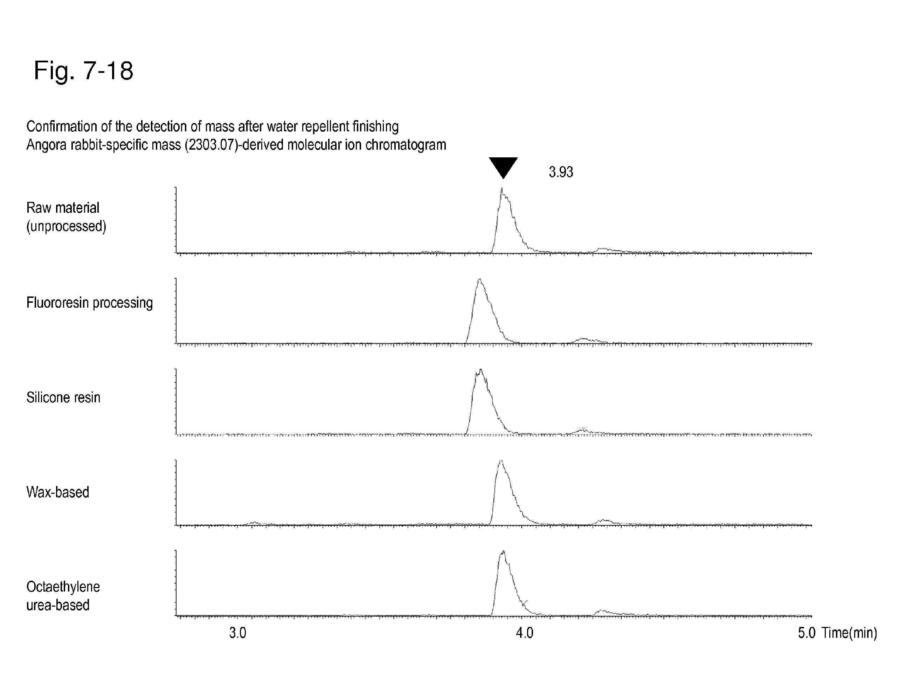

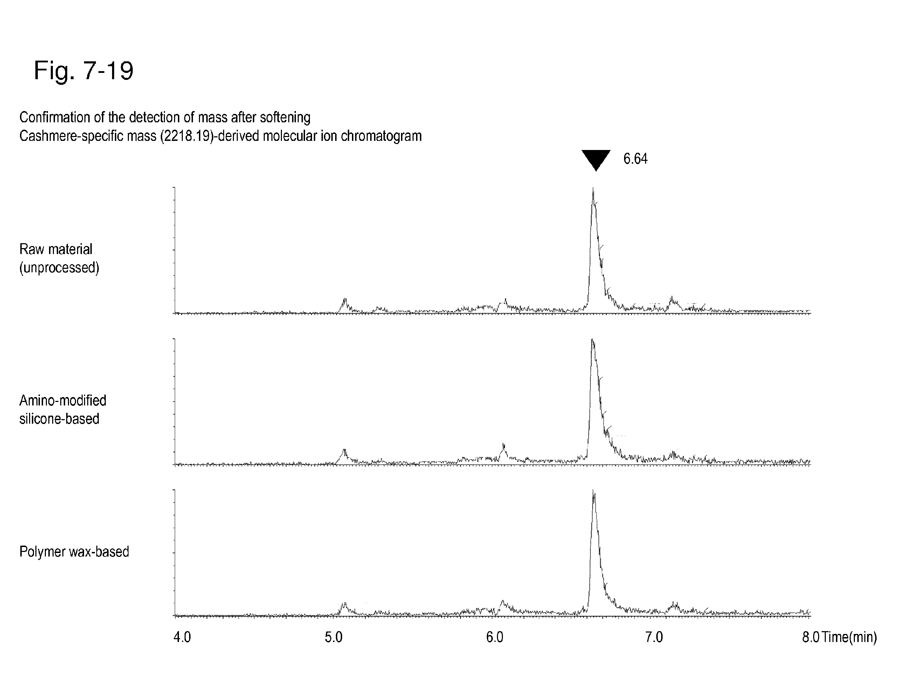

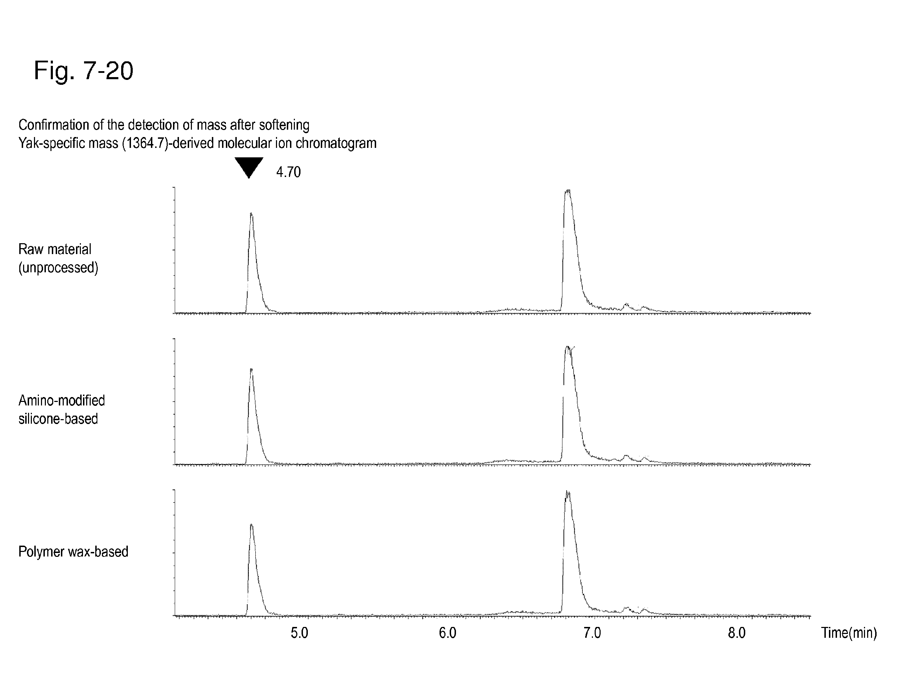

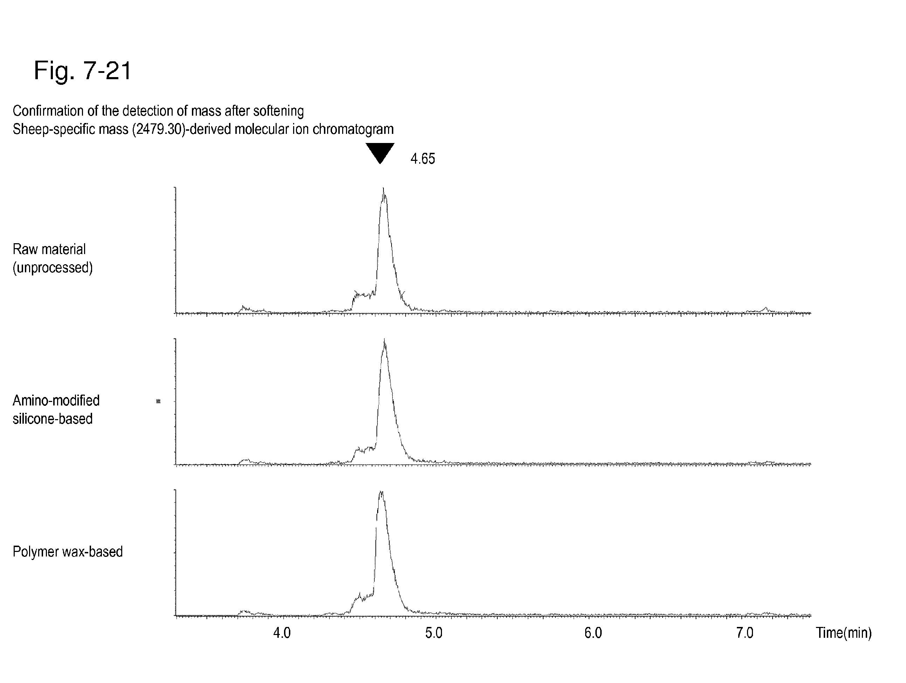

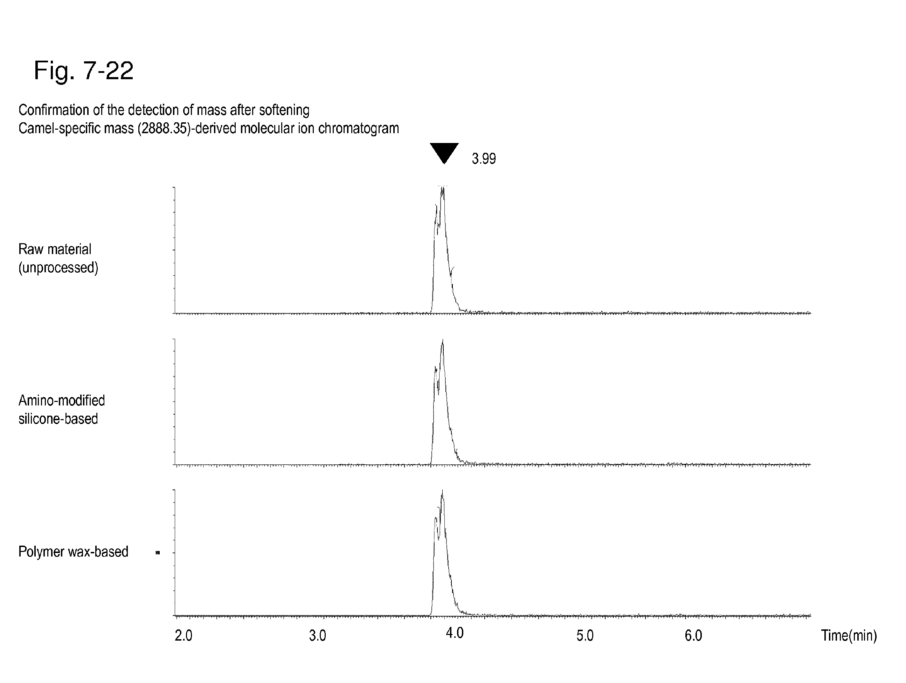

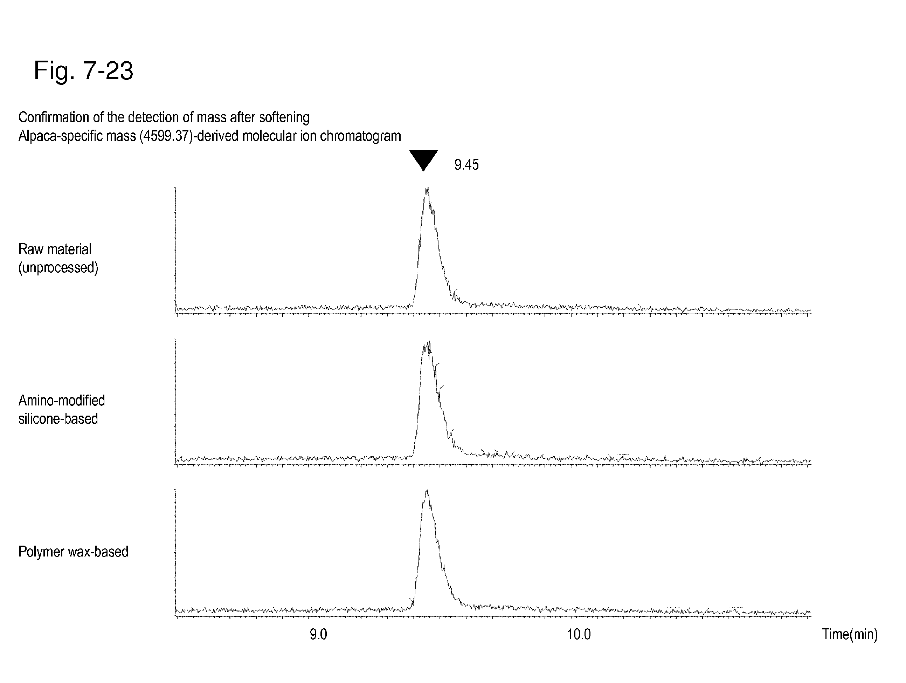

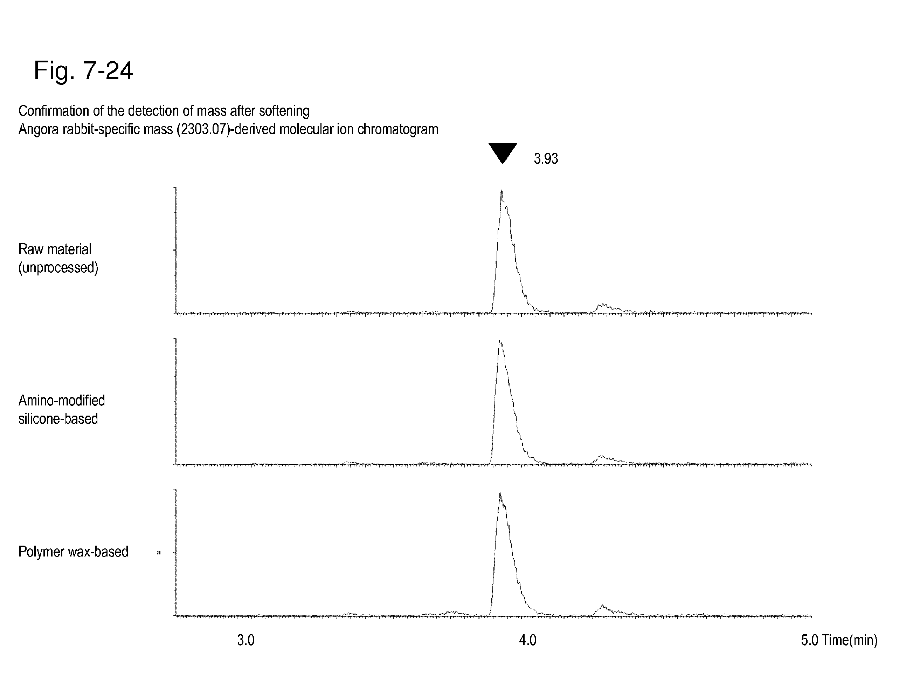

Fig. 7-1 shows the results of performing extraction from animal hair (also denoted as "raw wool" or "raw material") subjected to dyeing treatment or the like, treating with an enzyme, and then performing mass spectroscopy.

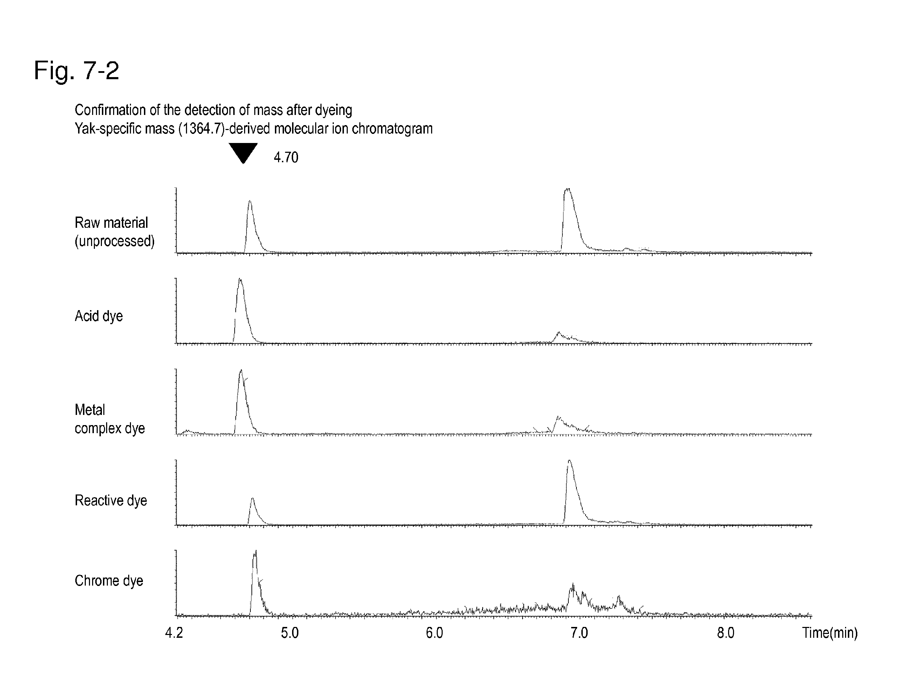

Fig. 7-2 shows the results of performing extraction from animal hair (also denoted as "raw wool" or "raw material") subjected to dyeing treatment or the like, treating with an enzyme, and then performing mass spectroscopy.

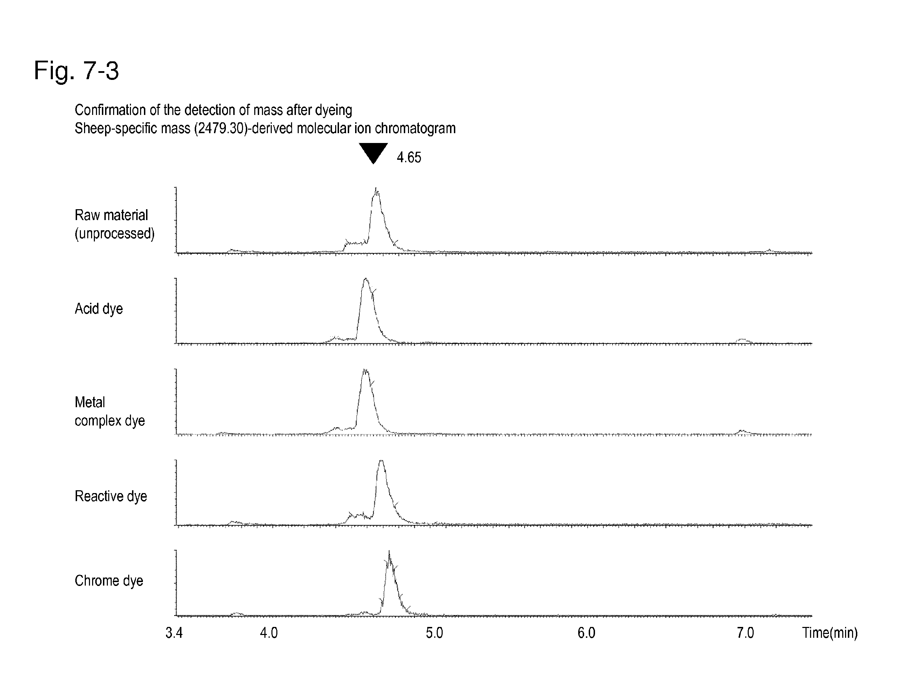

Fig. 7-3 shows the results of performing extraction from animal hair (also denoted as "raw wool" or "raw material") subjected to dyeing treatment or the like, treating with an enzyme, and then performing mass spectroscopy.

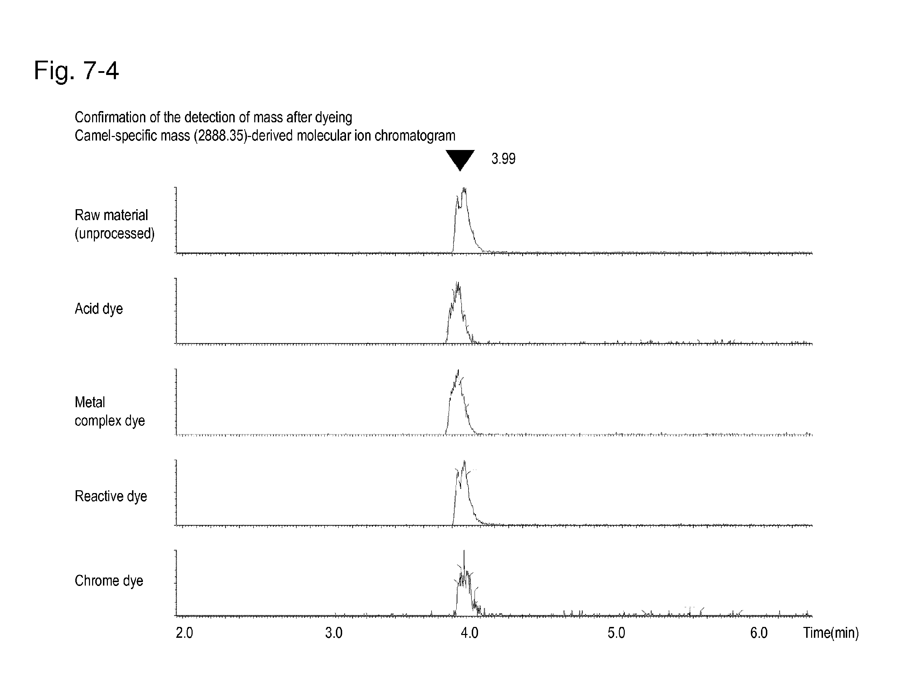

Fig. 7-4 shows the results of performing extraction from animal hair (also denoted as "raw wool" or "raw material") subjected to dyeing treatment or the like, treating with an enzyme, and then performing mass spectroscopy.

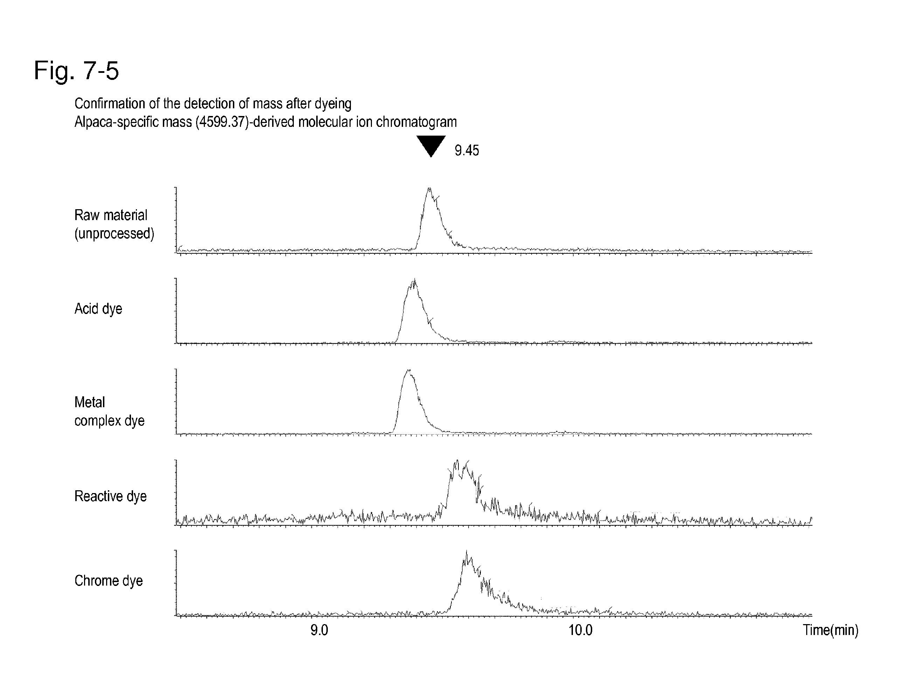

Fig. 7-5 shows the results of performing extraction from animal hair (also denoted as "raw wool" or "raw material") subjected to dyeing treatment or the like, treating with an enzyme, and then performing mass spectroscopy.

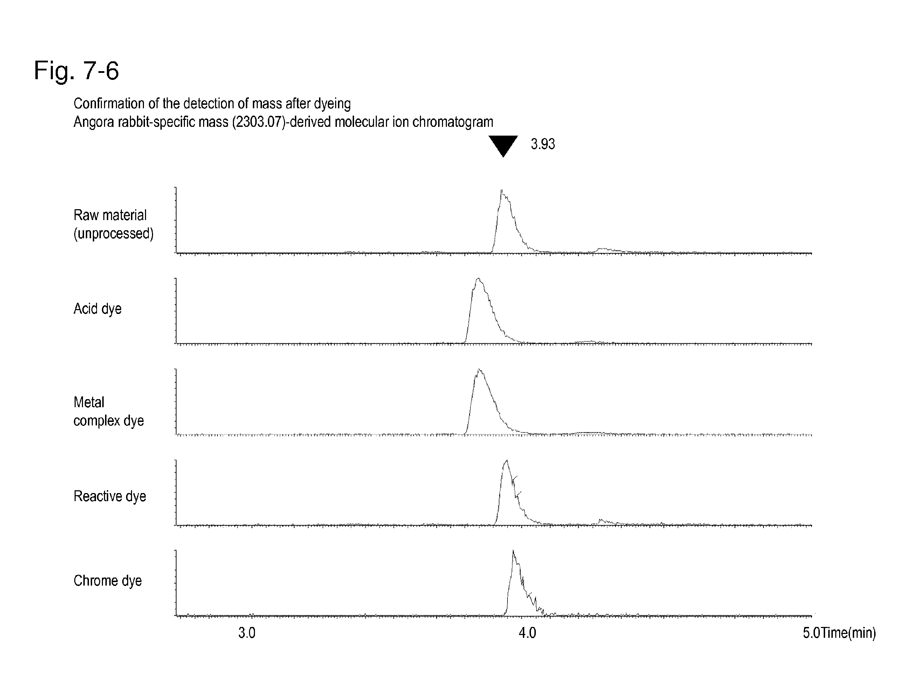

Fig. 7-6 shows the results of performing extraction from animal hair (also denoted as "raw wool" or "raw material") subjected to dyeing treatment or the like, treating with an enzyme, and then performing mass spectroscopy.

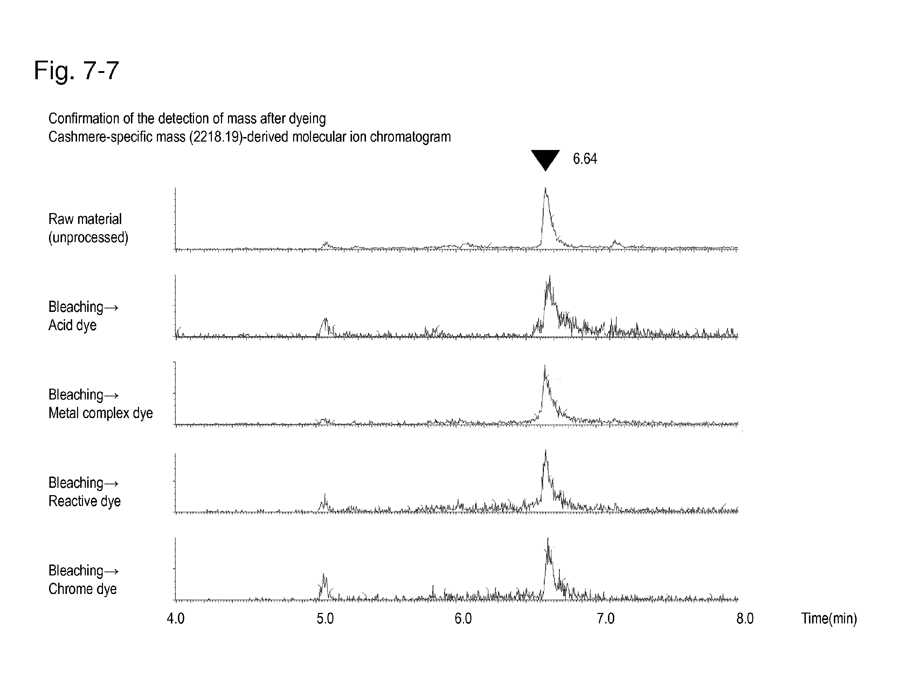

Fig. 7-7 shows the results of performing extraction from animal hair (also denoted as "raw wool" or "raw material") subjected to dyeing treatment or the like, treating with an enzyme, and then performing mass spectroscopy.

Fig. 7-8 shows the results of performing extraction from animal hair (also denoted as "raw wool" or "raw material") subjected to dyeing treatment or the like, treating with an enzyme, and then performing mass spectroscopy.

Fig. 7-9 shows the results of performing extraction from animal hair (also denoted as "raw wool" or "raw material") subjected to dyeing treatment or the like, treating with an enzyme, and then performing mass spectroscopy.

Fig. 7-10 shows the results of performing extraction from animal hair (also denoted as "raw wool" or "raw material") subjected to dyeing treatment or the like, treating with an enzyme, and then performing mass spectroscopy.

Fig. 7-11 shows the results of performing extraction from animal hair (also denoted as "raw wool" or "raw material") subjected to dyeing treatment or the like, treating with an enzyme, and then performing mass spectroscopy.

Fig. 7-12 shows the results of performing extraction from animal hair (also denoted as "raw wool" or "raw material") subjected to dyeing treatment or the like, treating with an enzyme, and then performing mass spectroscopy.

Fig. 7-13 shows the results of performing extraction from animal hair (also denoted as "raw wool" or "raw material") subjected to water repellent finishing or the like, treating with an enzyme, and then performing mass spectroscopy.

Fig. 7-14 shows the results of performing extraction from animal hair (also denoted as "raw wool" or "raw material") subjected to water repellent finishing or the like, treating with an enzyme, and then performing mass spectroscopy.

Fig. 7-15 shows the results of performing extraction from animal hair (also denoted as "raw wool" or "raw material") subjected to water repellent finishing or the like, treating with an enzyme, and then performing mass spectroscopy.

Fig. 7-16 shows the results of performing extraction from animal hair (also denoted as "raw wool" or "raw material") subjected to water repellent finishing or the like, treating with an enzyme, and then performing mass spectroscopy.

Fig. 7-17 shows the results of performing extraction from animal hair (also denoted as "raw wool" or "raw material") subjected to water repellent finishing or the like, treating with an enzyme, and then performing mass spectroscopy.

Fig. 7-18 shows the results of performing extraction from animal hair (also denoted as "raw wool" or "raw material") subjected to water repellent finishing or the like, treating with an enzyme, and then performing mass spectroscopy.

Fig. 7-19 shows the results of performing extraction from animal hair (also denoted as "raw wool" or "raw material") subjected to softening or the like, treating with an enzyme, and then performing mass spectroscopy.

Fig. 7-20 shows the results of performing extraction from animal hair (also denoted as "raw wool" or "raw material") subjected to softening or the like, treating with an enzyme, and then performing mass spectroscopy.

Fig. 7-21 shows the results of performing extraction from animal hair (also denoted as "raw wool" or "raw material") subjected to softening or the like, treating with an enzyme, and then performing mass spectroscopy.

Fig. 7-22 shows the results of performing extraction from animal hair (also denoted as "raw wool" or "raw material") subjected to softening or the like, treating with an enzyme, and then performing mass spectroscopy.

Fig. 7-23 shows the results of performing extraction from animal hair (also denoted as "raw wool" or "raw material") subjected to softening or the like, treating with an enzyme, and then performing mass spectroscopy.

Fig. 7-24 shows the results of performing extraction from animal hair (also denoted as "raw wool" or "raw material") subjected to softening or the like, treating with an enzyme, and then performing mass spectroscopy.

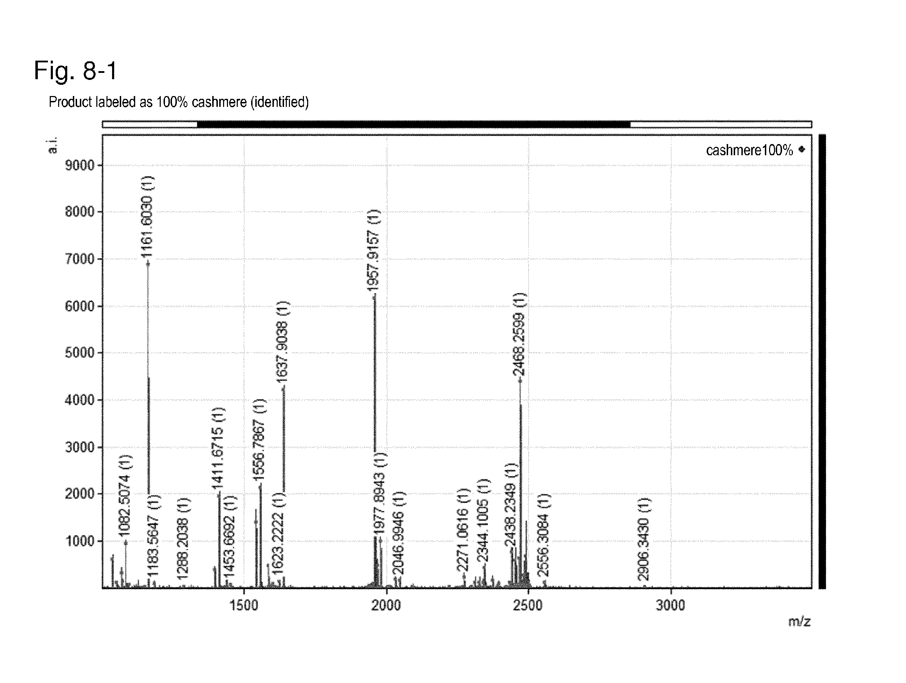

Fig. 8-1 shows the results of treating extracts from products with an enzyme and the performing mass spectroscopy (MALDI-TOF mass spectroscope).

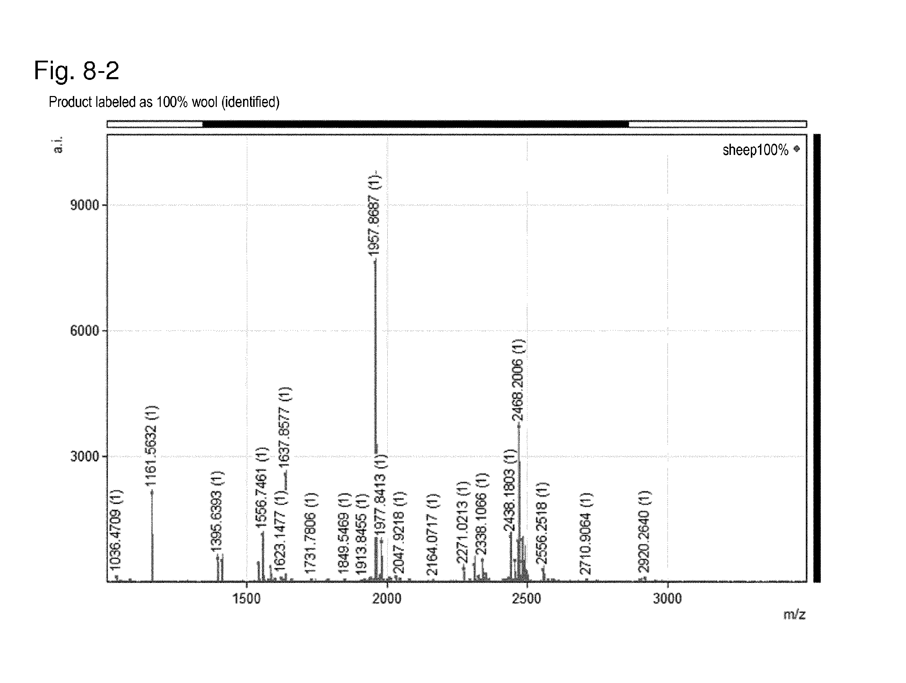

Fig. 8-2 shows the results of treating extracts from products with an enzyme and the performing mass spectroscopy (MALDI-TOF mass spectroscope).

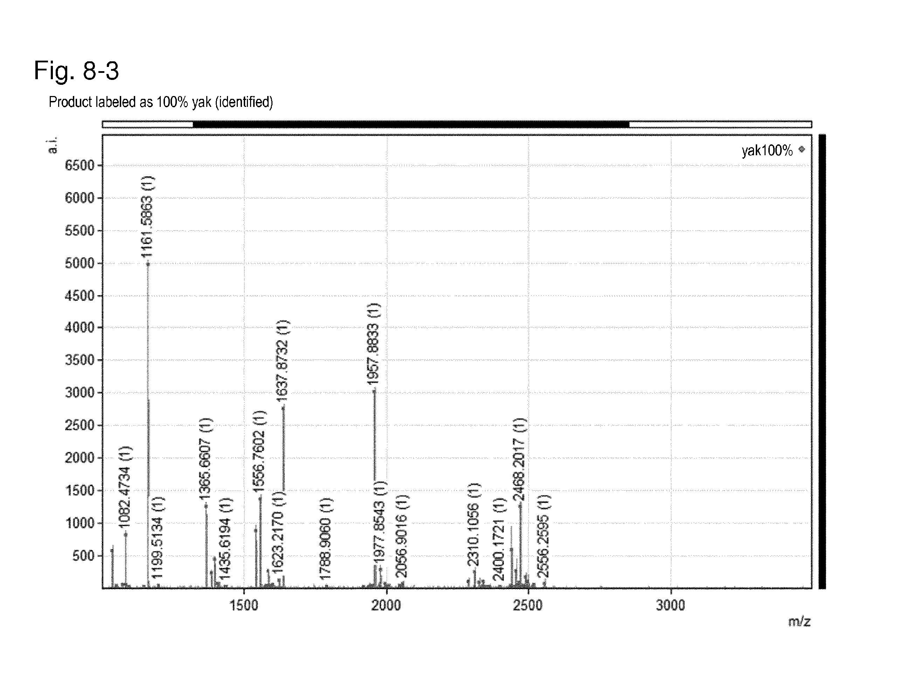

Fig. 8-3 shows the results of treating extracts from products with an enzyme and the performing mass spectroscopy (MALDI-TOF mass spectroscope).

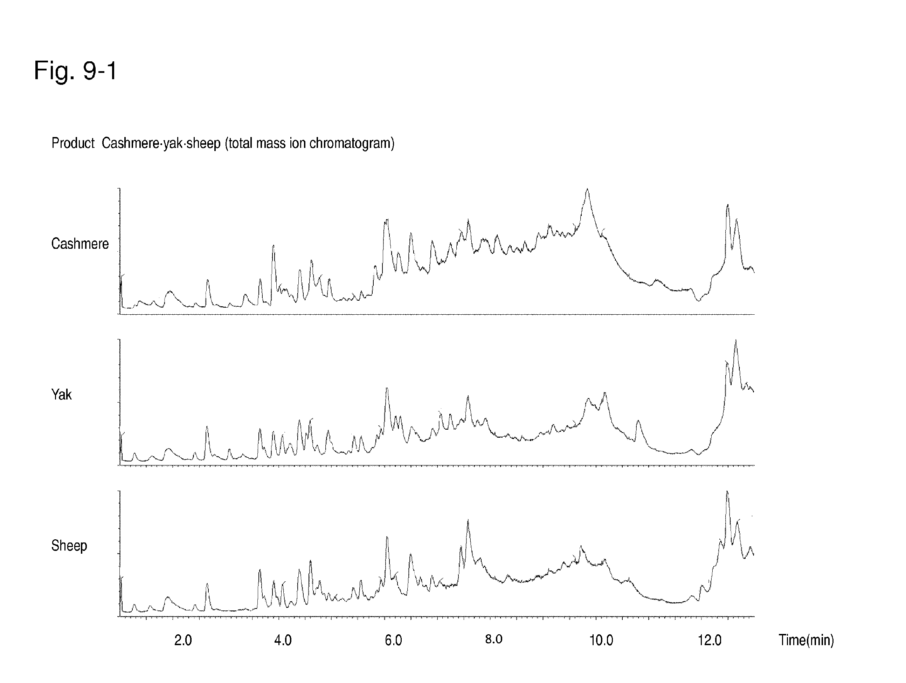

Fig. 9-1 shows the results of treating extracts from products with an enzyme and then performing mass spectroscopy.

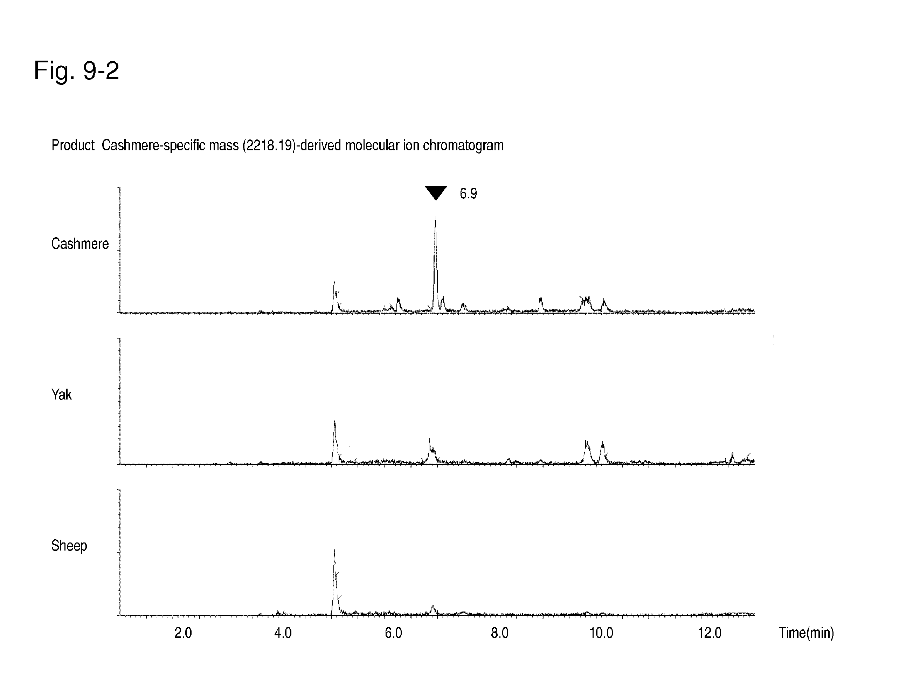

Fig. 9-2 shows the chromatogram of cashmere-specific masses in Fig. 9-1 above.

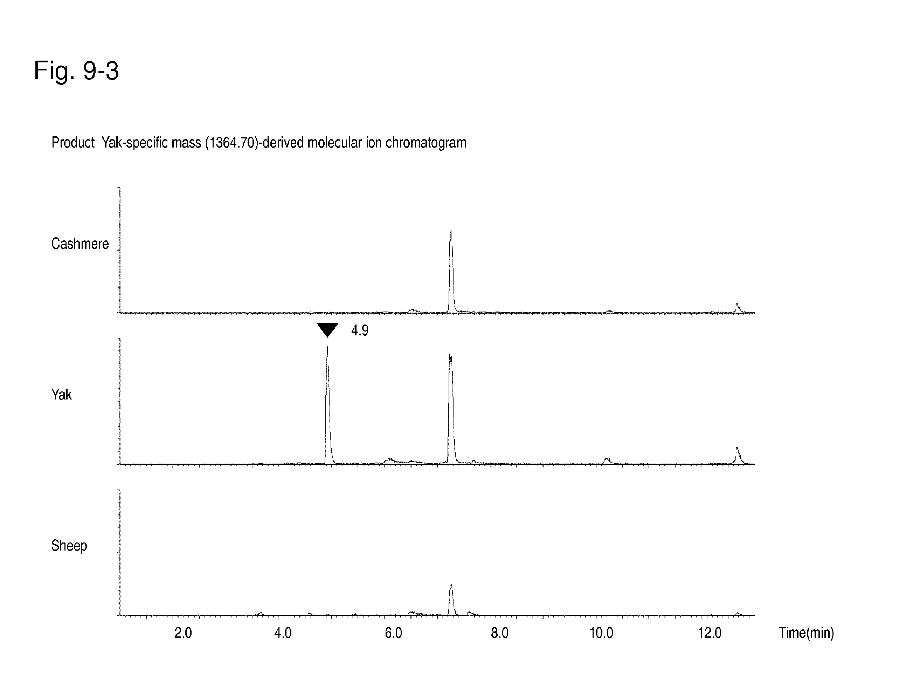

Fig. 9-3 shows the chromatogram of yak-specific masses in Fig. 9-1 above.

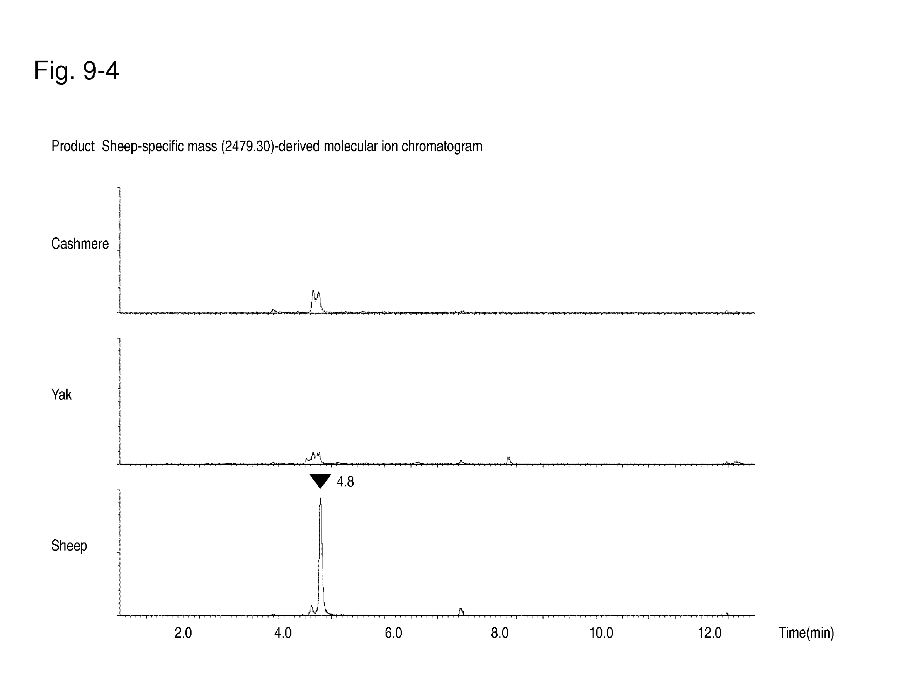

Fig. 9-4 shows the chromatogram of sheep-specific masses in Fig. 9-1 above.

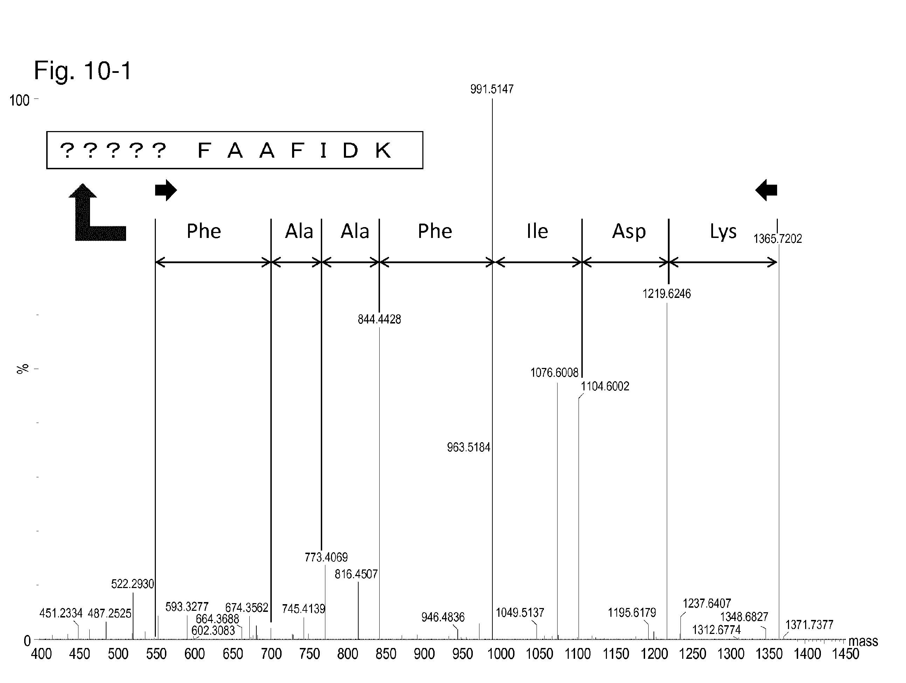

Fig. 10-1 shows the results of analyzing the yak-species-specific mass-to-charge ratio of m/z = 683.35.

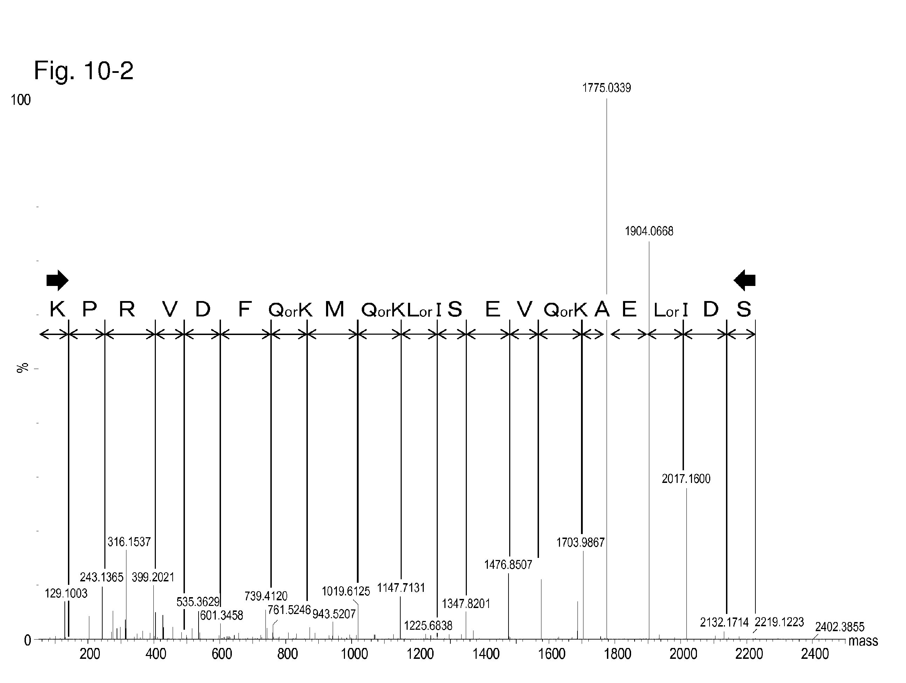

Fig. 10-2 shows the results of analyzing the cashmere-species-specific mass-to-charge ratio of m/z = 740.4.

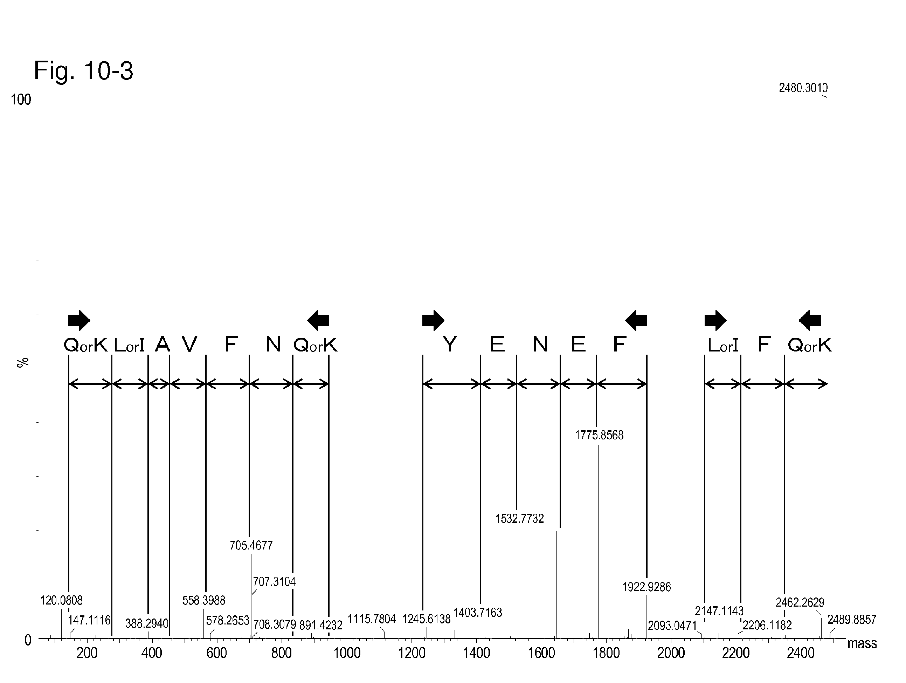

Fig. 10-3 shows the results of analyzing the sheep-species-specific mass-to-charge ratio of m/z = 620.8.

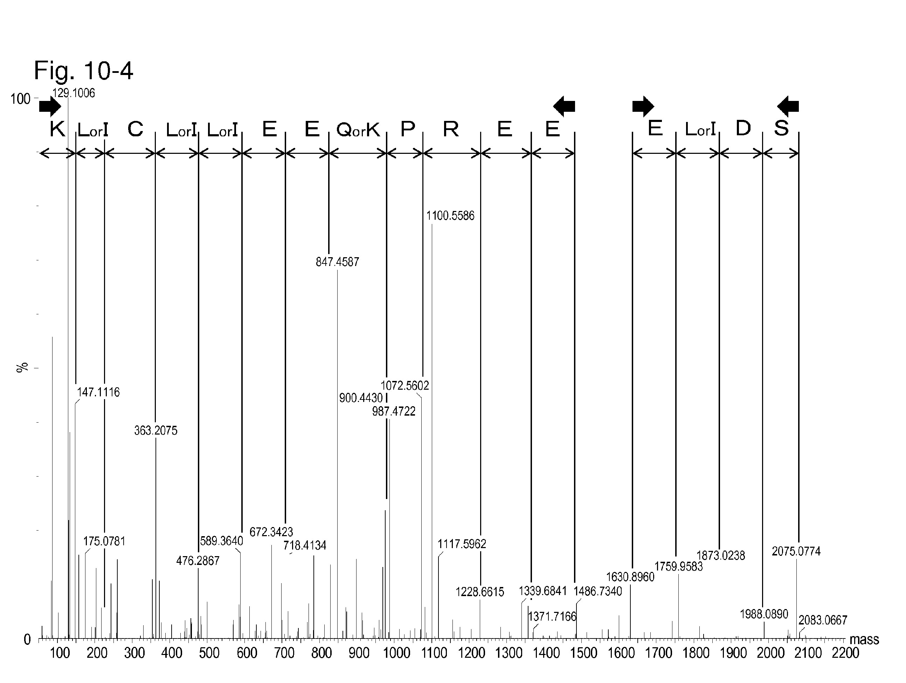

Fig. 10-4 shows the results of analyzing the sheep-species-specific mass-to-charge ratio of m/z = 692.4.

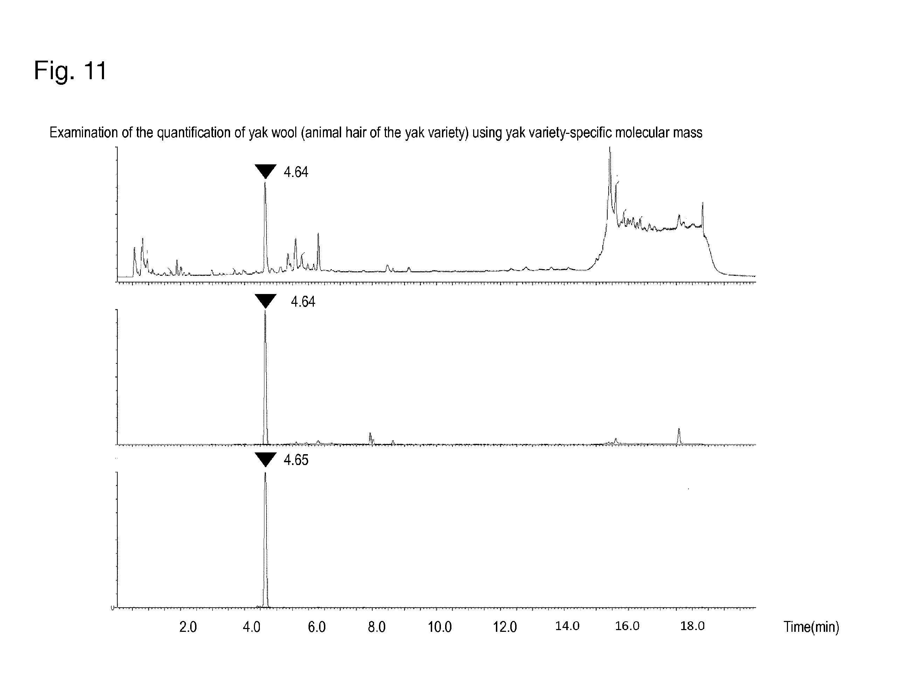

Fig. 11 shows the results of examining the quantification of yak using yak-species-specific molecular masses.

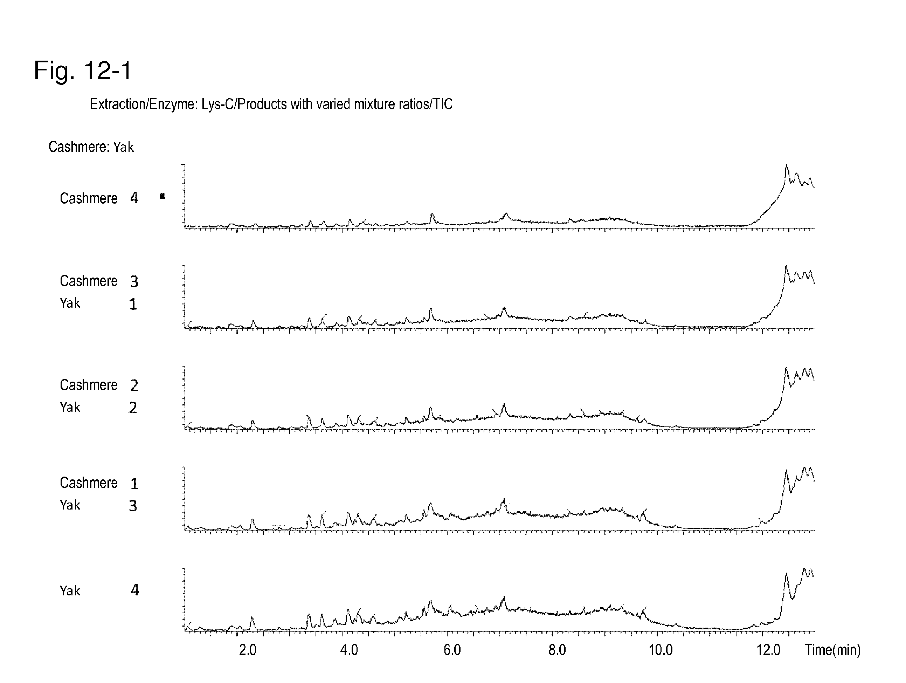

Fig. 12-1 shows the total mass ion chromatograms of samples with varied mixture ratios of cashmere products to yak products.

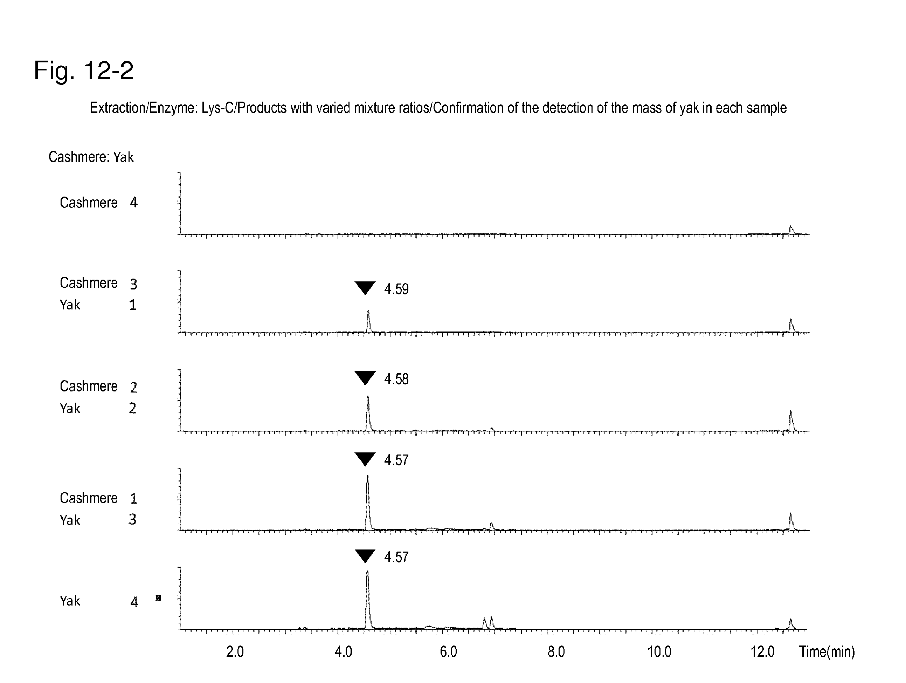

Fig. 12-2 shows the ion chromatogram of the yak-species-specific mass-to-charge ratio of m/z = 683.35 in Fig. 12-1.

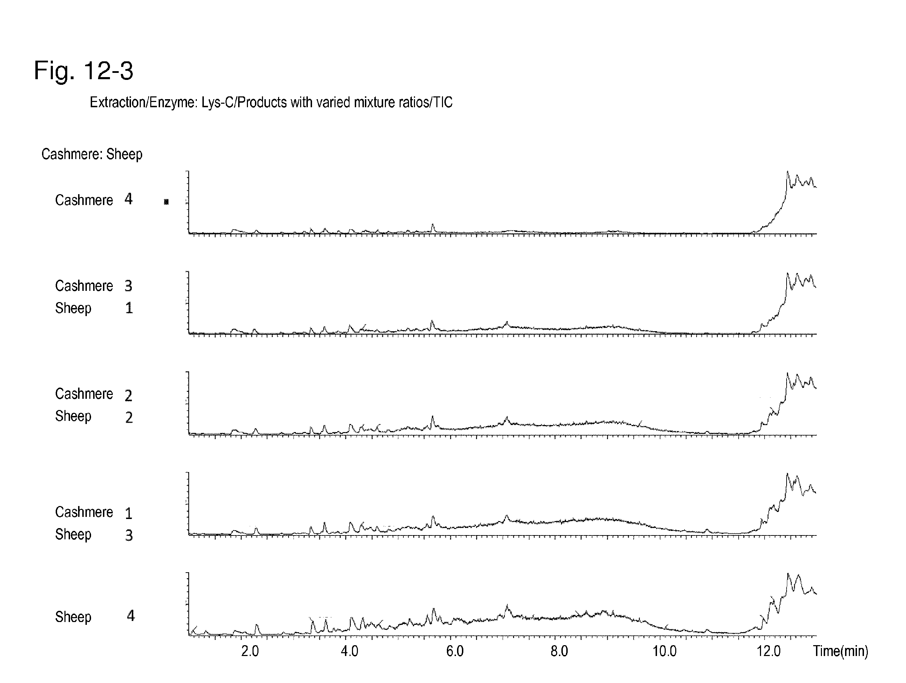

Fig. 12-3 shows the total mass ion chromatograms of samples with varied mixture ratios of cashmere products to sheep products (wool).

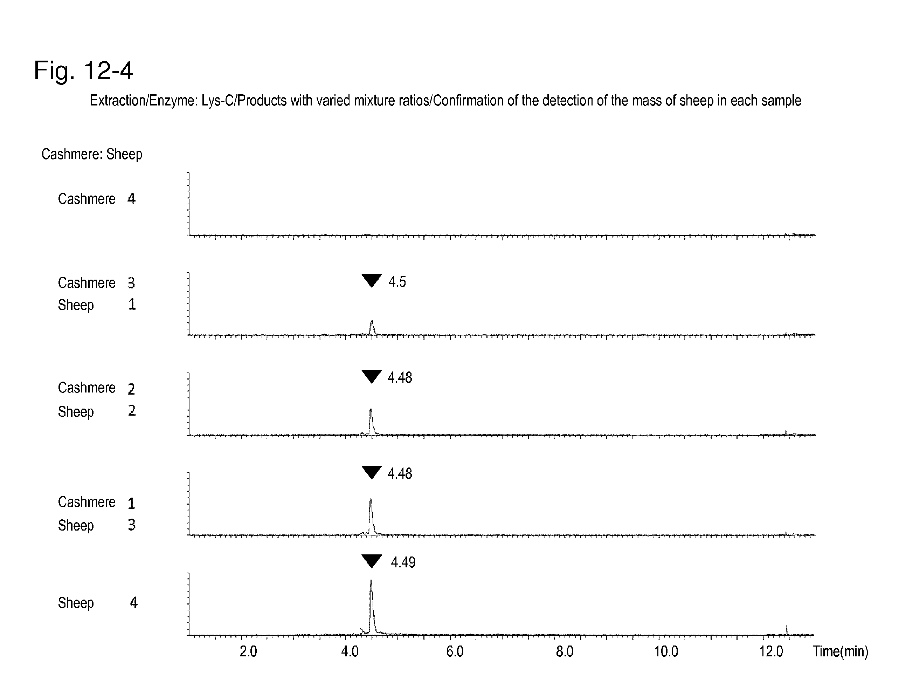

Fig. 12-4 shows the ion chromatogram of the sheep-species-specific mass-to-charge ratio of m/z = 620.82 in Fig. 12-3.

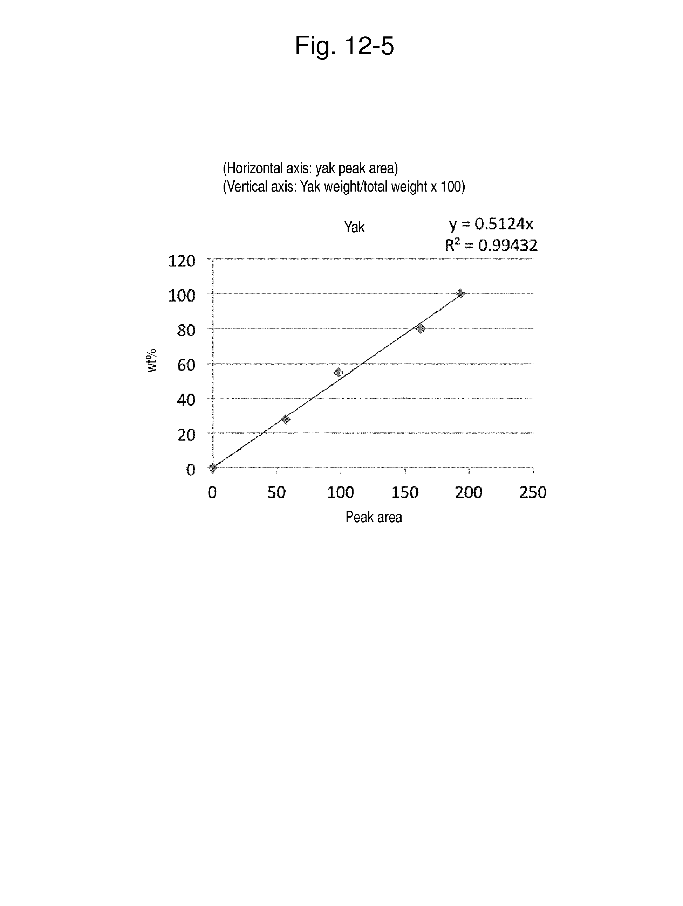

Fig. 12-5 shows the relationship between the peak area of the yak-species-specific mass-to-charge ratio of m/z = 683.35 obtained from Fig. 12-2 and wt% of a yak product in each analysis.

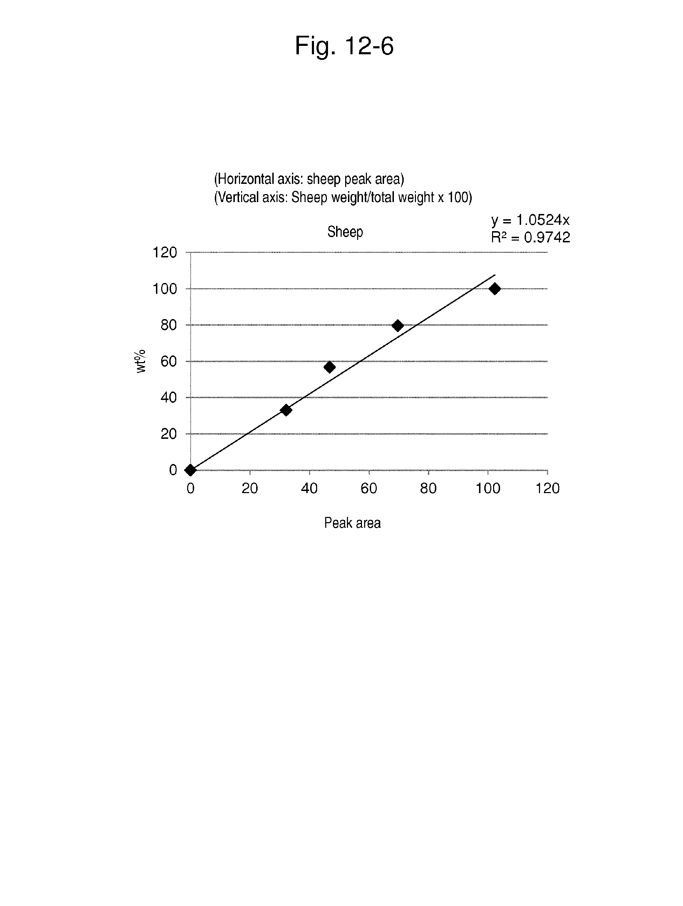

Fig. 12-6 shows the relationship between the peak area of the sheep-species-specific mass-to-charge ratio of m/z = 620.82 obtained from Fig. 12-4 and wt% of a sheep product (wool) in each analysis.

Modes for Carrying Out the Invention

1. Introduction

Not all wool products can be analyzed and/or differentiated by conventional analytical methods on the basis of DNA, such as a method for determining a animal species of animal hair, which involves amplifying DNA by PCR using DNA extracted from an animal hair product as a template, and then analyzing the molecular weight or the like of the amplified DNA, for example. Hence, the present inventors have studied to provide a more reliable determination method for animal species of animal hair.

The present inventors have examined various analytical methods to develop an analytical and differentiation method that comprises not by amplifying and detecting DNA contained in a trace amount in a wool product, but by directly analyzing proteins and the like that are constitutional molecules of animal hair.

Various methods are known as methods for analyzing proteins. The present inventors have attempted to analyze proteins by mass spectrometry as a method capable of conveniently analyzing a protein with any sequence with high reproducibility. When proteins in animal hair are analyzed by mass spectroscopy, mass spectroscopic results corresponding to the sequence of an animal-species-specific protein are obtained. Accordingly, the present inventors have examined the possibility of identifying animal species of animal hair by analyzing such mass spectroscopic results. As a result of trial and error, the present inventors have discovered that a specific mass peak is observed depending on the animal species of animal hair, in other words, the species of an animal having the animal hair, by pretreating, as necessary, a sample prepared from a product or the like containing animal hair, and then performing mass spectroscopy.

2. Method for differentiating animal hair and a method for quantifying an animal hair using mass spectroscopy

The present invention encompasses the methods described in the above "Means for Solving the Problem" and further encompasses "a method that comprises pretreating, as necessary, an animal-hair-containing sample collected from a product containing animal hair, and the like, performing mass spectroscopy, or, liquid chromatography and mass spectroscopy, detecting a mass peak corresponding to a species-specific sequence for a partial sequence of a protein such as keratin contained in animal hair, thereby specifying the animal species of animal hair and quantifying the animal hair as necessary".

2-1. Animal species of animal hair that can be specified by the present invention

According to the present invention, the animal species of animal hair of all mammals can be determined and quantified. The term "animal hair" as used herein refers to hair collected from an animal; that is, animal fiber. In addition, the present inventors have already obtained the mass data of the cashmere species of the genus *Capra* of the family Bovidae, the Angora species of the genus *Capra* of the family Bovidae, the yak species of the genus *Bos* of the family Bovidae, the sheep species of the genus *Ovis* of the family Bovidae, the genus *Camelus* of the family Camelidae, the alpaca species of the genus *Lama* of the family Camelidae (or the alpaca species of the genus *Vicugna),* and the Angora rabbit species of the genus *Oryctolagus* of the family Leporidae, which can be used as reference data, based on which these animal species of animal hair can be identified. In particular, these animal species of animal hair can be reliably identified using the following reference data.

2-1-1. Samples containing animal hair

A sample containing animal hair may be any sample that contains animal hair. Examples thereof include fur and animal hair itself, of course, that are obtained from the nature, and products produced using animal hair. Examples of a product produced using animal hair include, yarn, thread, fabric, knitted fabric, and felt. These products produced using these animal hair may be produced via various chemical treatments and heat treatments during the production, such as bleaching, dyeing, finishing, waterproof treatment, water repellent finishing, and softening. Examples of water repellent finishing include processing with a fluorocarbon polymer, processing with a silicon resin, wax-based processing and octaethylene urea-based processing. Examples of an agent for softening include amino-modified silicone-based agents and polymer wax-based agents.

2-2. Pretreatment

Examples of pretreatment include washing and the extraction of an analytical sample. A buffer containing a denaturing agent and a reducing agent can be used as a solution for extracting an analytical sample. As such a denaturing agent, urea or guanidinium hydrochloride can be used. Moreover, thiourea may also be added. An example of a reducing agent is DTT or β mercaptoethanol. Such a buffer may be any buffer as long as it is a general buffer. For example, a tris buffer can be used herein.

Pretreatment can include treatment with protease (proteolytic enzyme). Various proteases can be used herein and a preferable example thereof is endopeptidase (e.g., lysyl endopeptidase). Protein components in a sample can be fragmented by treatment with protease. For example, lysyl endopeptidase cuts the site immediately following lysine, but trypsin cuts two sites (the site immediately following lysine and the other site immediately following arginine), thereby fragmenting the protein.

2-3. Mass spectroscopy

The measurement principle of the present invention is based on the fact that animal species of animal hair can be differentiated from each other by partially cleaving proteins as necessary into fragments, or without performing fragmentation, ionizing the proteins or the fragments, separating the proteins or the fragments based on the masses, and detecting them with respect to each mass, so as to detect animal- species-specific signals, and thus differentiating animal species of animal hair from each other using the signals as indicators.

Examples of a mass spectrometer to be used in the methods of the present invention include MALDI-TOF MS and ESI-Q MS, and examples of a tandem mass spectrometer with an additional means for separation and detection include MALDI-QIT-TOF MS (addition of quadrupole ion trap), MALDI-TOF-TOF MS (addition of time-of-flight), ESI-QqQ (addition of quadrupole), and ESI-Q-TOF MS (addition of time-of-flight).

2-3-1. Ionization

As ionization methods for mass spectroscopy, an electron ionization (EI) method, a chemical ionization (CI) method, a fast atom bombardment (FAB) method, an electrospray ionization (ESI) method, an atmospheric pressure chemical ionization (APCI) method, a matrix-assisted laser desorption/ionization (MALDI) method, and the like are already known. An ionization means desired for the method of the present invention is MALDI or ESI.

2-3-2. Means for separation and detection

As mass detection devices for mass spectroscopy, for example, a magnetic sector mass spectrometer (Sector MS), a quadrupole mass spectrometer (QMS), an ion trap mass spectrometer (IT MS), a Time-of-Flight mass spectrometer (TOF MS), and a Fourier transform ion cyclotron resonance mass spectrometer (FT-ICR MS) are known. In the method of the present application, in accordance with an ionization method, a combination with a Time-of-Flight mass spectrometer (TOF MS) is desirably employed in the case of MALDI, and a combination with a quadrupole mass spectrometer (Q MS) is desirably employed in the case of ESI.

2-3-2-1. Tandem mass spectrometer involving the use of two separation and detection methods in combination

A tandem mass spectrometer involves the use of a combination of two separation and detection methods can also be used. A tandem mass spectrometer sends a specific mass alone to a collision chamber upon first separation, decomposes ions in the collision chamber, sends the decomposed products to a $2^{nd}$ separator to measure the mass distribution of decomposed products having specific masses. Such a tandem mass spectrometer enables to obtain the information concerning the structures of molecules with specific masses. For example, in the case of peptides, information such as amino acid sequences thereof can be obtained. In the method of the present application, a combined use of a Time-of-Flight mass spectrometer (TOF MS), a quadrupole mass spectrometer (Q MS), and an ion trap mass spectrometer (IT MS), and the like is desired. In addition, a tandem mass spectrometer enables to obtain the same information as that obtained by a single separation and detection method using a mass spectrometer depending on the setting. Hence, the use of a tandem mass spectrometer is desired.

2-3-3. Combination of mass spectroscopy and liquid chromatography

A higher degree of analysis can be performed by combining mass spectroscopy with liquid chromatography. Examples of liquid chromatography to be combined include high-precision liquid chromatography (HPLC) and ultrahigh-speed liquid chromatography (ULPC).

The MALDI-TOF MS method using MALDI as a means of ionization generally involves directly mixing a mixture with a matrix for crystallization, and then performing measurement; however, sufficient analytical information cannot be obtained with such a mixture. Hence, such a sample is recently analyzed in combination with liquid chromatography. On the other hand, the MALDI-TOF MS method involves mixing an analytical sample with a matrix for crystallization, so that the flow path of liquid chromatography and the flow path of a mass spectrometer cannot be used on-line (i.e., with the two flow paths connected) but rather off-line.

Meanwhile, the ESI ionization method involves spraying liquid for desolvation and ionization, and thus the flow path of liquid chromatography can be connected. For example, this can be used as LC-MS ("liquid chromatography-mass spectrometer) or UPLC MS. Hence, whereas the MALDI-TOF type involves treating an extract, measuring a mixture of various molecules, and performing determination with the mass information alone, UPLC MS is able to perform the determination based on two types of information - through the combined use of the mass information and the elution time of the masses.

2-3-4. Results of mass spectroscopy

(1) Monovalent protonated molecules are often detected by MALDI-TOF.

For example, when a molecule represented by $C_{89}H_{136}N_24O_{34}$ is subjected to mass spectroscopy, even if monoisotopic mass is 2084.9651, an object of mass spectroscopy is the ionized molecule. Thus, the mass that is measured herein is the protonated mass of 2085.9723 (to which the mass of the proton has been added). In addition, in the case of a mass spectrometer, a value obtained by dividing the mass of ions by the charge is detected as follows: (2084.96 + one proton (about 1 Da)) ÷ charge (1). Due to the presence of isotopes such as carbon 12 ($C^{12}$) and carbon 13 ($C^{13}$), the results are distributed with a difference of 1 Da. Even in the case of a single molecule such as $C_{89}H_{136}N_{24}O_{34}$, several masses are observed.

In the case of divalent ions for such distribution, 1043.49 is detected from 2084.96 + two protons (about $1 \times 2$ Da) ÷ charge (2). Because of division by 2, a difference of 1 Da due to the presence of isotopes is detected as 0.5 Da in this case. Conversely, ions can be determined to be divalent because of the difference of 0.5 Da. In the case of trivalent ions, a difference of about 0.3 Da is detected because of division by 3; and in the case of tetravalent ions, a difference of 0.25 Da is detected because of division by 4.

(2) Monovalent ions are often detected by MALDI; however, in the case of ESI, multivalent ions such as divalent and trivalent ions are often detected depending on molecules.

In addition, once a molecular mass is known, the mass-to-charge ratio of the molecular ions resulting from the mass can be found by calculation.

For example, when the mass of H is 1 and the molecule is a protonated molecule (positive ions) with a molecular mass of 1000,

the mass-to-charge ratio of a monovalent protonated molecule can be calculated as follows:

$$1001 \ (1000{+}1)/1 = 1001.$$

the mass-to-charge ratio of a divalent protonated molecule can be calculated as follows:

$$501 \ (1000{+}2)/2 = 501.$$

the mass-to-charge ratio of a trivalent protonated molecule can be calculated as follows:

$$334 \ (1000{+}3)/3 = 334$$

the mass-to-charge ratio of a tetravalent protonated molecule can be calculated as follows:

$$251 \ (1000{+}4)/4 = 251.$$

the mass-to-charge ratio of a pentavalent protonated molecule can be calculated as follows:

$$201 \ (1000{+}5)/5 = 201.$$

the mass-to-charge ratio of a 10-valent protonated molecule can be calculated as follows: 101 (1000+10)/10 = 101, for example.

(3) LC-MS involves detecting elution time and mass, and detecting with the use of a photodiode array detector, which is added before mass detection, thus enabling us to obtain the absorbance data detected.

Elution time differs depending on the type of column and the program of elution conditions. Absorbance detection may be performed at specific wavelengths alone, or spectra may be integrated. Examples of mass detection generally include integration of masses within the set range, and extraction of specific masses. Furthermore, when a composition contains various components, those components that are likely ionized become easily detected; however, in the case of LC-MS, such a composition is fractionated to some extent, so that those components which cannot be easily detected by MALDI can be detected.

(4) A tandem mass spectrometer is capable of converting the data into an amino acid sequence based on difference in mass; thus, a sequence composing a molecule with a specific mass can be analyzed. Specifically, with the use of the following Table 1 that lists the masses of amino acids, an amino acid can be assigned based on a difference in mass; however, different amino acids may be considered to be the same because of mass. As denoted as I (isoleucine) or L (leucine), further identification is not always possible. In consideration of a peptide bond, the masses obtained by subtracting the water are listed in Table 1

| Amino acid | 3-letter code | 1-letter code | Molecular weight | Molecular weight-$H_2O$ | |
|---|---|---|---|---|---|
| Alanine | Ala | A | 89.1 | 71.1 | |
| Arginine | Arg | R | 174.2 | 156.2 | |
| Asparagine | Asn | N | 132.1 | 114.1 | |
| Aspartic acid | Asp | D | 133.1 | 115.1 | |
| Cysteine | Cys | C | 121.2 | 103.2 | |
| Glutamine | Gln | Q | 146.2 | 128.2 | $C_5H_{10}N_2O_3$ |
| Glutamic acid | Glu | E | 147.1 | 129.1 | |
| Glycine | Gly | G | 75.1 | 57.1 | |
| Histidine | His | H | 155.2 | 137.2 | |
| Isoleucine | Ile | I | 131.2 | 113.2 | $C_6H_{13}NO_2$ |
| Leucine | Leu | L | 131.2 | 113.2 | $C_6H_{13}NO_2$ |
| Lysine | Lys | K | 146.2 | 128.2 | $C_6H_{14}N_2O_2$ |
| Methionine | Met | M | 149.2 | 131.2 | |
| Phenylalanine | Phe | F | 165.2 | 147.2 | |
| Proline | Pro | P | 115.1 | 97.1 | |
| Serine | Ser | S | 105.1 | 87.1 | |
| Threonine | Thr | T | 119.1 | 101.1 | |
| Tryptophan | Trp | W | 204.2 | 186.2 | |
| Tyrosine | Tyr | Y | 181.2 | 163.2 | |
| Valine | Val | V | 117.15 | 99.2 | |

2-4. Method for specifying animal species of animal hair based on mass spectroscopic results

2-4-1. Obtainment of reference data

Animal hair reference samples of a plurality of animal varieties for which the species names have been confirmed in advance are pretreated as described in 2-2 above. Mass spectroscopy is performed using the same mass spectroscope and the same measurement conditions (with setting values for a device to be generally used herein) as described in 2-3 Mass spectroscopy above. The thus obtained detection data are collected every animal species. Next, collected detection data are compared between varieties, and then detection data that are observed for only specific varieties can be considered as species-specific data.

Examples of detection data include detected masses, mass-to-charge ratios (also referred to as "m/z"), and the elution times of mass-to-charge ratios. As described above, as species-specific values, species-specific masses, species-specific mass-to-charge ratios (also referred to as "m/z"), and the elution times of species-specific mass-to-charge ratios are obtained.

In addition, for example, MALDI and ESI differ in terms of ionization method, and can also differ in terms of mass-to-charge ratio detected. Also, elution times obtained under the same conditions are compared.

2-4-1-1. In addition, the present inventors have obtained mass spectroscopic results of animal hair of the following varieties not treated with protease, as reference data as follows.

(1) The mass detected specifically to the yak species: 5948.90 ± 0.02%, or 2137.92 ± 0.02%.
(2) The mass-to-charge ratio (m/z) of molecular ions resulting from the mass above or another mass-to-charge ratio (m/z) detected specifically to the yak species: 9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, or 1069.98 ± 0.02%.

(II) Cashmere species

(1) The mass detected specifically to the cashmere species:

8976.45 ± 0.02%, 7102.18 ± 0.02%, or 7074.18 ± 0.02%.

(2) The mass-to-charge ratio (m/z) of molecular ions resulting from the mass above or another mass-to-charge ratio (m/z) detected specifically to the cashmere species:

10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, or 1415.83 ± 0.02%.

(III) Sheep species

(1) The mass detected specifically to the sheep species: 17538.88 ± 0.02% or 17584.26 ± 0.02%.
(2) The mass-to-charge ratio (m/z) of molecular ions resulting from the above mass or another mass-to-charge ratio (m/z) detected specifically to the sheep species:

10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, or 1599.55 ± 0.02%.

(IV) Angora species (also referred to as "mohair")

(1) The mass detected specifically to the Angora species: 6329.52 ± 0.02%
(2) The mass-to-charge ratio (m/z) of molecular ions calculated from the mass of (1) above.

(V) Alpaca species of the genus *Lama* of the family Camelidae (or alpaca species of the genus *Vicugna)*

(1) the mass detected specifically to the alpaca species:

6358.60 ± 0.02% or 6342.70 ± 0.02%.

(2) the mass-to-charge ratio (m/z) of molecular ions calculated from the mass of (1) above

(VI) Angora rabbit species of the genus *Oryctolagus* of the family Leporidae (also referred to as "Angora")

(1) The mass detected specifically to the Angora rabbit species: 1042.43 ± 0.02%.

(2) The mass-to-charge ratio (m/z) of molecular ions calculated from the mass of (1) above.

2-4-1-2. In the above pretreatment, when treatment with lysyl endopeptidase was performed as protease treatment, the following reference data were obtained.

(I) Yak species of the genus *Bos* of the family Bovidae

(1) The mass detected specifically to the yak species:

1364.70 ± 0.02%, 1383.72 ± 0.02%, 1431.67 ± 0.02%, 2469.27 ± 0.02%, or 2495.30 ± 0.02%.

(2) The mass-to-charge ratio (m/z) of molecular ions calculated from the mass of (1) above

(II) Cashmere species of the genus *Capra* of the family Bovidae

(1) The mass detected specifically to the cashmere species:
2218.19 ± 0.02%, 3379.48 ± 0.02%, or 2391.99 ± 0.02%.
(2) The mass-to-charge ratio (m/z) of molecular ions calculated from the mass of (1) above

(III) Sheep species of the genus *Ovis* of the family Bovidae

(1) The mass detected specifically to the sheep species:

2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, or 2174.00 ± 0.02%.

(2) The mass-to-charge ratio (m/z) of molecular ions calculated from the mass of (1) above

(IV) The genus *Camelus* of the family Camelidae

(1) The mass detected specifically to the genus *Camelus,* 2888.35 ± 0.02%.
(2) The mass-to-charge ratio (m/z) of molecular ions calculated from the mass of (1) above

(V) Alpaca species of the genus *Lama* of the family Camelidae (or alpaca species of the genus *Vicugna*)

(1) The mass detected specifically to the alpaca species (or the alpaca species of the genus *Vicugna),*
4599.37 ± 0.02%, 901.47 ± 0.02%, or 532.32 ± 0.02%.
(2) The mass-to-charge ratio (m/z) of molecular ions calculated from the mass of (1) above

(VI) Angora rabbit species of the genus *Oryctolagus* of the family Leporidae

(1) The mass detected specifically to the Angora rabbit species:
2303.07 ± 0.02%, 2502.15 ± 0.02%, 2058.02 ± 0.02%, or 1554.81 ± 0.02%.
(2) The mass-to-charge ratio (m/z) of molecular ions calculated from the mass of (1) above 2-4-2. Identification of animal species of animal hair in samples

Pretreatment described in 2-2 above and mass spectroscopy described in 2-3 above are performed for animal-hair-containing samples to be subjected to the identification of animal species of animal hair, under the same conditions as those for the above reference samples, and then data detected for the samples are compared with the above species-specific reference data. When the species-specific values are contained, such a sample is found to contain the animal hair of the relevant species. More specifically, examples of the methods are as follows.

(1) Masses or mass-to-charge ratios (m/z) obtained as a result of performing pretreatment of 2-2 above and mass spectroscopy of 2-3 above for an animal-hair-containing test sample without performing protease treatment are compared with the reference data of masses or mass-to-charge ratios (m/z) specific to the cashmere species of the genus *Capra* of the family Bovidae, the Angora species of the genus *Capra* of the family Bovidae, the yak species of the genus *Bos* of the family Bovidae, the sheep species of the genus *Ovis* of the family Bovidae, the alpaca species of the genus *Lama* of the family Camelidae (or the alpaca species of the genus *Vicugna),* and the Angora rabbit species of the genus *Oryctolagus* of the family Leporidae, respectively, listed in 2-4-1-1 above. If masses or mass-to-charge ratios (m/z) specific to any one of these varieties are included in the mass spectroscopic results, it can be determined that the sample contains the animal hair of the relevant species.

(2) When an animal-hair-containing test sample is treated with lysyl endopeptidase that is protease, masses or mass-to-charge ratios (m/z) obtained as a result of performing pretreatment of 2-2 above and mass spectroscopy of 2-3 above are compared with the reference data of masses or mass-to-charge ratios (m/z) specific to the cashmere species of the genus *Capra* of the family Bovidae, the yak species of the genus *Bos* of the family Bovidae and the sheep species of the genus *Ovis* of the family Bovidae, respectively, listed in 2-4-1-2 above. If masses or mass-to-charge ratios (m/z) specific to any one of these varieties are included in the mass spectroscopic results, it can be determined that the sample contains the animal hair of the relevant animal species.

(3) Furthermore, an animal-hair-containing test sample is treated or not treated with protease. Regarding the m/z elution time derived from animal species-specific molecular species obtained as a result of performing pretreatment of 2-2 above and mass spectroscopy of 2-3 above, the same elution time is always detected if the program elution conditions are the same for liquid chromatography (LC or UPLC). Therefore, when the results of 2 samples are the same in terms of the m/z elution time derived from animal species-specific molecular species under the same program conditions, animal hair in one sample and that in the other sample can be considered to be derived from the same animal species.

2-5. Animal-species-specific polypeptide sequence

The present inventors have found by tandem mass spectrometry (MSMS), peptide sequences and nucleic acid sequences encoding the sequences unique to several animal species of animal hair.

(1) Specifically, regarding the yak species, as specific peptides, molecular ions resulting from a peptide and/or a protein comprising the amino acid sequence represented by SEQ ID NO: 1: GLNNRFAAFIDK, and SEQ ID NO: 2: GGT CTC AAC AAC AGG TTT GCT GCC TTC ATC GAC AAG GTG CGC encoding the above SEQ ID NO: 1 were identified.

(2) Regarding the cashmere species, as specific polypeptides, molecular ions resulting from a peptide and/or a protein comprising the amino acid sequence represented by SEQ ID NO: 3: SD (L or I) EA (Q or K) VES (L or I) (Q or K) M (Q or K) FDVRPK were identified.

(3) Regarding the sheep species, the following (a) and (b) were found as specific polypeptides.

    (a) Molecular ions resulting from a peptide and/or a protein comprising 2 or more of the amino acid sequences represented by SEQ ID NOS: 4 to 9 in combination.
    SEQ ID NO: 4: QF (L or I)
    SEQ ID NO: 5: FENEY
    SEQ ID NO: 6: (Q or K) NFVA (L or I) (Q or K)
    SEQ ID NO: 7: (L or I) FQ
    SEQ ID NO: 8: YENEF
    SEQ ID NO: 9: (Q or K) (L or I) AVFN (Q or K)
    (b) Molecular ions resulting from a peptide and/or a protein comprising 2 or more of the amino acid sequences represented by SEQ ID NOS: 10 to 13 in combination.
    SEQ ID NO: 10: SD (L or I) E
    SEQ ID NO: 11: (Q or K) EE (L or I) (L or I) C (L or I) K
    SEQ ID NO: 12: E (L or I) DS
    SEQ ID NO: 13: K (L or I) C (L or I) (L or I) EE (Q or K)

Peptides listed above can be separately used as markers for yak (animal hair of the yak species), cashmere (animal hair of the cashmere species), and sheep (animal hair of the sheep species). Animal species of animal hair (or animal species from which animal hair is derived) can also be identified by mass spectrometry, or a conventionally known amino acid sequence analysis method or a method for analyzing nucleic acid sequences encoding the above amino acid sequences, or the like based on the presence or the absence of these marker molecules that are animal-species-specific sequences.

Moreover, these markers can be used as standard substances in the following quantification methods.

2-6. Method for quantifying animal hair

Animal hair can be quantified by various known methods. An example of which is described below.

(1) Method 1 for calculating the percentage of animal hair mixed in

(1-1) Identification of animal species of animal hair:

Regarding an animal hair sample, an animal species of animal hair is first identified according to 2-5 above. When one or more animal species are identified therein, the percentages thereof mixed in the sample can be calculated as follows. In this case, reference data can be used similarly to 2-5 above. If no enzyme treatment is performed as pretreatment, the reference data of species-specific masses or mass-to-charge ratios (m/z) listed in 2-4-1-1 can be used. If enzyme treatment (treatment with lysyl endopeptidase) is performed as pretreatment, the reference data of species-specific masses or mass-to-charge ratios (m/z) listed in 2-4-1-2 can be used.

(1-2) Selection of mass-to-charge ratio specific to hair of each animal species and calculation of peak area:

An ion chromatogram is prepared for m/z derived from molecular species specific to one of every two varieties identified and then the peak area is found. For example, an ion chromatogram is prepared to display only the animal-species-specific mass-to-charge ratios that are extracted from the total mass ion chromatogram of a sample containing one of the two identified varieties. This chromatogram is subjected to data processing, then the peak area of the relevant animal species -specific mass-to-charge ratios can be calculated.

In addition, as such animal-species -specific mass-to-charge ratios, when no enzyme treatment is performed as pretreatment, the reference data of animal species-specific mass-to-charge ratios (m/z) listed in 2-4-1-1 can be used. When enzyme treatment (treatment with lysyl endopeptidase) is performed as pretreatment, the reference data of animal species-specific mass-to-charge ratios (m/z) listed in 2-4-1-2 can be used.

(1-3) Selection of common mass(es) for hair of each animal species and calculation of the peak area(s) thereof:

Similarly, an ion chromatogram of molecular-species-derived specific molecular mass-to-charge ratios (m/z) shared by two varieties is prepared and the peak area thereof is found.

The following mass-to-charge ratios can be used as common mass-to-charge ratios shared by cashmere, yak, and sheep varieties:

(i) a mass-to-charge ratio of molecular ions, which is calculated from the mass of $1070.5 \pm 0.02\%$;
(ii) a mass-to-charge ratio of molecular ions, which is calculated from the mass of $1555.76 \pm 0.02\%$;
(iii) a mass-to-charge ratio of molecular ions, which is calculated from the mass of $1160.58 \pm 0.02\%$; and
(iv) a mass-to-charge ratio of molecular ions, which is calculated from the mass of $602.30 \pm 0.02\%$.

(1-4) Preparation of calibration curve: The ratio of the peak area of the common masses to the peak area of masses specific to a target animal species, the percentage of which mixed in is to be found, is found, a graph is prepared therefrom, and a calibration curve is prepared. A calibration curve can also be prepared using the peak area of masses specific to a target animal species, the percentage of which mixed in is to be found. At this time, data correction with common masses enables us to obtain a calibration curve with higher accuracy.

(1-5) Calculation of the percentage of animal hair mixed in

The percentage of animal hair mixed in a sample is calculated using a calibration curve.

(2) Method 2 for calculating the percentage of animal hair mixed in

Firstly, for an unknown sample, the animal species of animal hair that is used therein is identified using the prepared reference data according to 2-5 above.

For example, when two animal species are included, animal species 1 and animal species 2 are each subjected to measurement of animal species-specific mass-to-charge ratios. Then, the peak areas of animal species-specific mass-to-charge ratios are calculated. Next, using a plurality of reference samples containing animal species 1 and 2, for which the percentage of species 1 mixed in and the percentage of species 2 mixed in are already known and varied, the peak area is measured and a calibration curve is prepared.

In addition, as reference data of animal species-specific mass-to-charge ratios, the reference data of animal species-specific mass-to-charge ratios (m/z) listed in 2-4-1-1 above and the reference data of animal species-specific mass-to-charge ratios (m/z) listed in 2-4-1-2 can be used, similarly to method 1 mentioned above for calculating the percentage of animal hair mixed in.

The percentage of animal species 1 or 2 mixed in the sample can be calculated using the calibration curve.

(3) Method 3 for calculating the percentage of animal species of animal hair mixed in

The percentages of animal species of animal hair mixed in a sample containing 2 or more animal species can also be similarly calculated. Specifically, the percentages of animal species of animal hair mixed in a sample containing 2 or more animal species (hereinafter, described as "N species") of animal hair can be found by calculating the percentages of (N-1) species of animal hair mixed in the sample, and then finding the percentages of N species mixed in the sample based on a difference from 100%. For example, the percentages of 3 species (cashmere, yak, and sheep) mixed in a sample can be calculated by the use of masses detected commonly in cashmere, yak, sheep, and the like. The percentage of animal species of an animal hair mixed in such a sample can be calculated by, with respect to the peak area of mass-to-charge ratios of the common masses:

> (i) calculating the percentage of cashmere mixed in the sample through comparison with a cashmere 100% sample, using the peak area of cashmere-specific mass-to-charge ratios;
> (ii) calculating the percentage of yak mixed in the sample through comparison with a yak 100% sample using the peak area of yak-specific masses;
> (iii) calculating the percentage of sheep mixed in the sample through comparison with a sheep 100% sample using the peak area of sheep-specific masses, and thus
> (iv) calculating the percentages of cashmere, yak, and sheep mixed in the sample based on the above results.

In addition, as animal species-specific mass-to-charge ratios, the reference data of animal species-specific mass-to-charge ratios (m/z) listed in 2-4-1-1 above and the reference data of animal species-specific mass-to-charge ratios (m/z) listed in 2-4-1-2 can be used, similarly to method 1 mentioned above for calculating the percentage of animal hair mixed in.

Moreover, as the mass-to-charge ratios of common masses, mass-to-charge ratios detected commonly in cashmere, yak, and sheep, which are described in the above-mentioned method 1 (1-3) for calculating the percentage thereof mixed in, can be used.

(4) Analysis of samples in which animal hair is mixed with non-animal-hair fibers

The mixture ratio of animal hair to non-animal-hair fibers can be calculated using the release method, the dissolution method, and microscopy as specified in the JIS L1030-2. The animal species of animal hair that is used in such a sample can be specified, and the percentage of hair of each animal species that is mixed in such a sample can be calculated by the method of the present invention.

(4-1) The release method as specified in the JIS L1030-2 involves disentangling a product into yarns, classifying by fiber types, and then calculating the percentages of fiber types mixed therein.

For example, in the case of a product composed of a warp made of sheep and a weft made of polyester, the product is disentangled into warp and weft. The mass of each is found, and then represented as a percentage.

(4-2) The dissolution method specified in the JIS L1030-2 involves dissolving specific components in a selected solution. The amount of a component(s) that is (are) not dissolved is then measured, and the percentage of the specific component mixed therein is calculated. Depending on the components to be dissolved, concentrated sulfuric acid, sodium hypochlorite, sodium hydroxide solution, acetone, dimethylformamide, and the like are used at various concentrations. For example, animal hair such as sheep, mohair, or cashmere can be dissolved in a test method using sodium hypochlorite.

(5) Quantification method

A standard peptide, the sequence of which has been revealed above, is used as follows. The peak area of the standard peptide molecule is calculated by mass spectroscopy. The amount of the standard peptide is then calculated from the mass-peak area of the relevant molecule derived from a test sample; thus, the amount of animal hair contained in the sample (extract) can be determined. In addition, the same sequence of such a standard peptide is always used for determination such that the degrees of ionization efficiency can be leveled and absolute quantification becomes possible.

Specifically, calculation for yak can be performed as follows.

(i) An ion chromatogram of yak-species-specific mass-to-charge ratios is displayed, and the presence or the absence of ions is confirmed. When ions are detected, the peak area is calculated via analysis. Next, molecular ions derived from a synthetic peptide (molecular mass=1371.66 Da) is also analyzed to calculate the peak area.

(ii) The synthetic peptide-derived peak area is converted into a concentration or an amount from the concentration of the synthetic peptide, and the resultant is compared with the peak area derived from an extracted fluid sample. Thus, the peptide determined to be of the yak species, which is contained in the extracted fluid sample, can be quantified.

[Example 1] Mass spectroscopy (MALDI-TOF mass spectrometer) of animal hair (also referred to as "raw wool" or "raw material") extracts

(1) Preparation of animal hair extracts

500 $\mu$l of a protein extraction buffer (aqueous solution (pH 9.5) containing 7 M urea, 0.05 M DTT (dithiothreitol) and 0.05 M Tris-HCl) was added to 8 mg of an analytical sample (animal hair (raw wool) and then left to stand at 37°C overnight. After reaction, centrifugation was performed at 4°C (5 min, 15000 rpm), and then the supernatant was collected as an extract.

In addition, animal hair of the cashmere species of the genus *Capra* of the family Bovidae, that of the yak species of the genus *Bos* of the family Bovidae, and that of the sheep species of the genus *Ovis* of the family Bovidae were used.

(2) Mass spectroscopy

4 $\mu$l of a matrix solution (7.5 mg of CHCA ($\alpha$-cyano-4-hydroxycinnamic acid) was dissolved in 500 $\mu$l of 0.1% trifluoroacetic acid/acetonitrile (2:1)) was added to and mixed with 1 $\mu$l of the extract. Drops (1 $\mu$l) of the solution were placed on a target plate for mass spectroscopy. After drying at room temperature, analysis was conducted using a MALDI-TOF (matrix-assisted laser desorption ionization-Time-of-Flight) mass spectrometer (Ultraflex-TOF/TOF (trademark), Bruker Daltonics) in reflector mode : positive ion mode and linear mode : positive ion mode.

(3) Analytical results

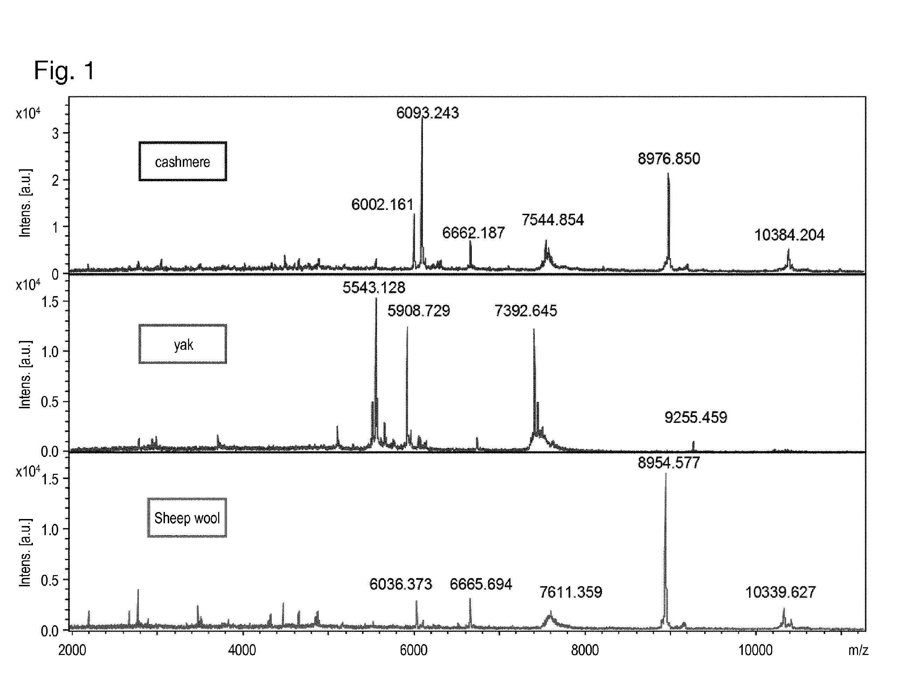

Analytical results are shown in Fig. 1. In addition, in Fig. 1, "cashmere" indicates the analytical results of the animal hair extract of the cashmere species of the genus *Capra* of the family Bovidae, "yak" indicates the analytical results of the animal hair extract of the yak species of the genus *Bos* of the family Bovida, and "sheep" indicates the analytical results of the animal hair extract of the sheep species of the genus *Ovis* of the family Bovidae.

[Example 2] Mass spectroscopy (UPLC-MS analyzer) of animal hair (also referred to as "raw wool" or "raw material") extracts

(1) Preparation of animal hair extracts

Similarly to Example 1, an extract was obtained from the animal hair of each of the cashmere species of the genus *Capra* of the family Bovidae, the Angora species of the genus *Capra* of the family Bovidae, the yak species of the genus *Bos* of the family Bovidae, the sheep species of the genus *Ovis* of the family Bovidae, the genus *Camelus* of the family Camelidae, the alpaca species of the genus *Lama* of the family Camelidae (or the alpaca species of the genus Vicugna), and the Angora rabbit species of the genus *Oryctolagus* of the family Leporidae.

(2) Mass spectroscopy

Each extract was centrifuged using a 0.22-$\mu$m filtration tube, and then transferred into a container for LC-MS analysis. As a UPLC-MS analyzer, Waters (R) Acquity UPLC system and Xevo™QT of MS were used. Analysis was conducted using Waters Acquity UPLC (R) BEH C4 (2.1mm $\times$ 50 mm, 1.7 $\mu$m) as a column, a column temperature of 60°C, an autosampler temperature of 5°C, and 5 $\mu$l of injection volume. Solvent A (0.1% formic acid) and solvent B (0.1% formic acid, acetonitrile) were used for mobile phase. Analysis was conducted at a flow rate of 0.2 ml/min. A separation program was comprised of 33 min. Linear gradients employed herein are as follows: 0 min (A : B = 90 : 10), 0-25 min (A : B = 40 : 60), 25-26 min (A : B = 5 : 95), 26-30 min (A : B = 5 : 95), 30-30.1 min (A : B = 90 : 10), and 30.1-33 min (A:B=90:10). Mass spectroscopy conditions were comprised of a capillary voltage of 3.0kV, a sample cone voltage of 30 kV, a desolvation temperature of 500°C, and a source temperature of 150°C.

(3) Analytical results

Based on the results of Example 1 and Example 2,

- molecular masses specific to the cashmere species of the genus *Capra* of the family Bovidae are as follows:

  8976.45 ± 0.02%, 7102.18 ± 0.02%, and 7074.18 ± 0.02%.

Also, the mass-to-charge ratios of molecular ions resulting from the above molecular masses or other m/z values specific to the cashmere species of the genus *Capra* of the family Bovidae are as follows.
10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, and 1415.83 ± 0.02%.

- Molecular masses specific to the yak species of the genus *Bos* of the family Bovidae are as follows.
  5948.90 ± 0.02%, and 2137.92 ± 0.02%.

Also, the mass-to-charge ratios of molecular ions resulting from the above molecular masses or mass-to-charge ratios (m/z values) specific to the yak species of the genus *Bos* of the family Bovidae are as follows.
9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, and 1069.98 ± 0.02%.

- Molecular masses specific to the sheep species of the genus *Ovis* of the family *Bovidae* are as follows.
  17538.88 ± 0.02%, and 17584.26 ± 0.02%.

Furthermore, mass-to-charge ratios of molecular ions resulting from the above molecular masses or mass-to-charge ratios (m/z values) specific to the sheep species of the genus *Ovis* of the family *Bovidae-specific* are as follows.
10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, and 1599.55 ± 0.02%.

- A molecular mass specific to the genus *Camelus* of the family Camelidae is as follows.
  2887.35 ± 0.02%

A mass-to-charge ratio specific to the genus *Camelus* of the family Camelidae is a mass-to-charge ratio of molecular ions resulting from the above molecular mass.

- A molecular mass specific to the Angora species of the genus *Capra* of the family Bovidae (also referred to as "mohair") is as follows.
  6329.52 ± 0.02%

A mass-to-charge ratio (m/z) specific to the Angora species of the genus *Capra* of the family Bovidae is a mass-to-charge ratio of molecular ions resulting from the above molecular mass.

- A molecular masses specific to the alpaca species of the genus *Lama* of the family Camelidae (or the alpaca species of the genus *Vicugna*) is as follows.
  6358.60 ± 0.02%, or 6342.70 ± 0.02%.

A mass-to-charge ratio specific to the alpaca species of the genus *Lama* of the family Camelidae (or the alpaca species of the genus *Vicugna*) is a mass-to-charge ratio of molecular ions resulting from the above molecular mass.

- A molecular mass specific to the Angora rabbit species of the genus *Oryctolagus* of the family Leporidae is as follows.
  1042.43 ± 0.02%

A mass-to-charge ratio specific to the Angora rabbit species of the genus *Oryctolagus* of the family Leporidae is a mass-to-charge ratio of molecular ions resulting from the above molecular mass.

[Example 3] Enzyme treatment and mass spectroscopy (MALDI-TOF mass spectroscope) of animal hair (also referred to as "raw wool" or "raw material") extracts

(1) Preparation of animal hair extracts

In a manner similar to Example 1, an extract was obtained from the animal hair of each of the cashmere species of the genus *Capra* of the family Bovidae, the Angora species of the genus *Capra* of the family Bovidae, the yak species of the genus *Bos* of the family Bovidae, the sheep species of the genus *Ovis* of the family Bovidae, the genus *Camelus* of the family Camelidae, the alpaca species of the genus *Lama* of the family Camelidae (or the alpaca species of the genus Vicugna), and the Angora rabbit species of the genus *Oryctolagus* of the family Leporidae.

(2) Enzyme treatment of extracts

1 $\mu$l of the thus obtained extract was mixed with 18 $\mu$l of a protease digestion buffer (50% acetonitrile, 0.0005% Trion X-45, 20 mM Tris-HCl (pH 8.8)), and then 1 $\mu$l of a protease solution (prepared by dissolving 20 $\mu$g of lysyl endopeptidase (Lys-C) in 400 $\mu$l of 2 mM Tris-HCl (pH 8.0)) was added, followed by 2.5 hours of reaction at 37°C.

(3) Mass spectroscopy

1 $\mu$l of an extract subjected to enzyme treatment was analyzed in a manner similar to Example 1 using CHCA ($\alpha$-cyano-4-hydroxycinnamic acid) as a matrix.

(4) Analysis results

Based on the analysis results of various samples, in the case of the yak species of the genus *Bos* of the family Bovidae, the mass-to-charge ratio of m/z = 1365.7 (the mass-to-charge ratio of molecular mass (1364.70)-derived molecular ions), and, the mass-to-charge ratio of m/z = 2287 (the mass-to-charge ratio of molecular mass (2286)-derived molecular ions) were found to be species-specific.

Based on the results of mass spectroscopy for samples obtained by treating the extract of yak (the animal hair of the yak species) with an enzyme, Fig. 3-1 shows an enlarged view around the mass-to-charge ratio of m/z = 1365.7, and, Fig. 3-2 shows an enlarged view around the mass-to-charge ratio of m/z = 2287. In addition, as a result of analyzing 50 cases (samples) of yak raw material, the mass-to-charge ratio (m/z = 1365.7, the mass-to-charge ratio of molecular mass (1364.70)-derived molecular ions) was detected for all 50 cases, and the mass-to-charge ratio of m/z = 2287 was detected for 40 cases.

[Example 4] Extraction from animal hair (also referred to as "raw wool" or "raw material") subjected to dyeing treatment etc., enzyme treatment and mass spectroscopy (MALDI-TOF mass spectroscope)

(1) Preparation of extracts

In a manner similar to Example 1, extracts were obtained from yak raw material (raw wool of the yak species of the genus *Bos* of the family Bovidae) subjected to dyeing treatment (metal complex dyeing, acid dyeing, reactive dyeing, and chrome dyeing), and yak raw material subjected to decoloring and then dyeing treatment (metal complex dyeing, acid dyeing, reactive dyeing, and chrome dyeing).

(2) Enzyme treatment of extracts

In a manner similar to Example 3, treatment with protease (lysyl endopeptidase (Lys-C)) was performed.

(3) Mass spectroscopy

1 $\mu$l of an extract subjected to enzyme treatment was analyzed in a manner similar to Example 3 using CHCA ($\alpha$-cyano-4-hydroxycinnamic acid) as a matrix.

(4) Analysis results

Analysis results are shown in Fig. 4a and Fig. 4b. The mass-to-charge ratio (m/z = 1365.7) was detected for all samples from yak raw material that had been subjected to decoloring and/or dyeing treatment. Determination of such yak or products can be difficult with the use of a conventional differentiation method using DNA. However, the method of the present invention enables the reliable identification of such yak and products as the animal hair of the yak species.

[Example 5-1] Enzyme treatment and mass spectroscopy (UPLC-MS analyzer) of animal hair (also referred to as "raw wool" or "raw material") extracts

(1) Preparation of animal hair extracts

In a manner similar to Example 1, an extract was obtained from animal hair of each of the cashmere species of the genus *Capra* of the family Bovidae, the Angora species of the genus *Capra* of the family Bovidae, the yak species of the genus *Bos* of the family Bovidae, the sheep species of the genus *Ovis* of the family Bovidae, the genus *Camelus* of the family Camelidae, the alpaca species of the genus *Lama* of the family Camelidae (or the alpaca species of the genus *Vicugna),* and the Angora rabbit species of the genus *Oryctolagus* of the family Leporidae.
In addition, an extraction buffer was added, the solution was kept at 37°C overnight, and then dialysis was performed against a 0.05M Tris-HCl-containing aqueous solution (pH9.5). The thus-obtained sample can also be used as an extract.

(2) Enzyme treatment of extracts

The thus-obtained extract and a protease digestion buffer were mixed in equal amounts. A protease solution (lysyl endopeptidase (Lys-C)) was added, followed by reaction at 37°C for a predetermined length of time. In addition, the protease digestion buffer and the protease solution (lysyl endopeptidase (Lys-C)) had the same compositions as described in Example 3.

(3) Mass spectroscopy

Each extract was centrifuged using a 0.22-$\mu$m filtration tube, and then transferred into a container for LC-MS analysis. As a UPLC-MS analyzer, Waters (R) Acquity UPLC system and Xevo™QT of MS were used. Analysis was conducted using Waters Acquity UPLC BEH C18 (2.1 mm × 100 mm, 1.7 $\mu$m) as a column, a column temperature of 40°C, an autosampler temperature of 5°C, and 5 $\mu$l of injection volume. Solvent A (0.1% formic acid) and solvent B (acetonitrile) were used for the mobile phase. Analysis was conducted at a flow rate of 0.4 ml/min. A separation program was comprised of 17 min. Linear gradients employed herein are as follows: : 0-1 min (A : B = 90 : 10), 1-11 min (A : B = 50 : 50), 11-11.5 min (A: B = 10 : 90), 11.5-14.5 min (A: B = 10 : 90), 14.5-14.6 min (A: B = 90 : 10), and 14.6-17 min (A : B = 10 : 90).
In addition, when Waters Acquity UPLC BEH C18 (2.1 mm×50 mm, 1.7$\mu$m) was used as a column, a separation program was comprised of 10 min. Linear gradients employed herein are as follows: 0-0.5 min (A: B = 90 : 10), 0.5-6.5 min (A : B = 50 : 50), 6.5-7 min (A : B = 10 : 90), 7-8.5 min (A : B = 10 : 90), 8.5-8.6 min (A : B = 90 : 10), and 8.6-10 min (A: B = 10 : 90).
Mass spectroscopy conditions were comprised of a capillary voltage of 3.0kV, a sample cone voltage of 30kV, a desolvation temperature of 500°C, and a source temperature of 150°C.

(4) Analysis results

To date, the cashmere species of the genus Capra of the family Bovidae (52 samples), the Angora species of the genus *Capra* of the family Bovidae (12 samples), the yak species of the genus *Bos* of the family Bovidae (70 samples), the sheep species of the genus *Ovis* of the family Bovidae (41 samples), the genus Camelus of the family Camelidae (16 samples), the alpaca species of the genus *Lama* of the family Camelidae (or the alpaca species of the genus *Vicugna*) (9 samples), and the Angora rabbit species of the genus *Oryctolagus* of the family Leporidae (9 samples) were analyzed.

(5) Discussion

Based on the results of Examples 3, 4 and 5,

• molecular masses specific to the cashmere species of the genus *Capra* of the family Bovidae are as follows.

2218. 19 ± 0. 02%, 3379. 48 ± 0.02%, and 2391. 99 ± 0. 02%.

Moreover, examples of mass-to-charge ratios (m/z values) specific to the cashmere species of the genus Capra of the family Bovidae include mass-to-charge ratios of molecular ions resulting from the above molecular masses (M). Examples thereof include 2219. 19 ± 0. 02%, 1110. 10 ± 0. 02%, 740. 40 ± 0. 02%, and 555. 55 ± 0. 02%.

- Molecular masses specific to the yak species of the genus *Bos* of the family Bovidae are as follows.
  1364. 70 ± 0. 02%, 1383. 72 ± 0. 02%, 1431. 67 ± 0. 02%, 2469. 27 ±. 02%, and 2495. 30 ± 0.02%.

Moreover, examples of mass-to-charge ratios (m/z values) specific to the yak species of the genus *Bos* of the family Bovidae-specific include mass-to-charge ratios of molecular ions resulting from the above molecular masses (M). Examples thereof include 1365. 7 ± 0. 02%, 683. 35 ± 0. 02%, and 455. 90 ± 0. 02%.

- Molecular masses specific to the sheep species of the genus *Ovis* of the family Bovidae are as follows.
  2479. 30 ± 0. 02%, 4977. 24 ± 0. 02%, 2340. 03 ±0. 02%, 2358. 02 ± 0. 02%, 1051. 41 ± 0. 02%, 1033. 39 ± 0. 02%, 2074. 12 ± 0. 02%, and 2174. 00 ± 0. 02%.

Moreover, examples of mass-to-charge ratios (m/z values) specific to the sheep species of the genus *Ovis* of the family Bovidae include mass-to-charge ratios of molecular ions resulting from the above molecular masses (M). Examples thereof include
2480. 30 ± 0. 02%, 1240. 65 ± 0. 02%, 827. 43 ± 0. 02%, 620. 82 ± 0. 02%, and 496. 86 ± 0. 02%, as well as
2075. 12 ± 0. 02%, 1038. 06 ± 0. 02%, 692. 37 ± 0. 02%, 519. 53 ± 0. 02%, and 415. 82 ± 0. 02%.

- Amass specific to the genus *Camelus* of the family Camelidae is as follows.
  2888. 35 ± 0.02%.

Moreover, examples of mass-to-charge ratios (m/z values) specific to the genus *Camelus* of the family Camelidae include mass-to-charge ratios of molecular ions resulting from the above molecular mass (M).

- Molecular masses specific to the alpaca species of the genus *Lama* of the family Camelidae (or alpaca species of the genus *Vicugna)* are as follows.
  4599.37 ± 0.02%, or 901.47 ± 0.02%, and 532.32 ± 0.02%.

Moreover, examples of mass-to-charge ratios (m/z values) specific to the alpaca species of the genus *Lama* of the family Camelidae (or the alpaca species of the genus *Vicugna*) include mass-to-charge ratios of molecular ions resulting from the above molecular masses (M).

- Molecular masses specific to the Angora rabbit species of the genus *Oryctolagus* of the family Leporidae are as follows.
  2303.07 ± 0.02%, 2502.15 ± 0.02%, 2058.02 ± 0.02%, and 1554.81 ± 0.02%.

Furthermore, as mass-to-charge ratios (m/z values) specific to the Angora rabbit species of the genus *Oryctolagus* of the family Leporidae, mass-to-charge ratios of molecular ions resulting from the above molecular masses (M) were detected, and were determined to be molecular masses and mass-to-charge ratios specific to various varieties, in addition to the elution time of each mass-to-charge ratio.
Among analysis results, the analysis results (total mass ion chromatogram) of cashmere (denoted as "cashmere" in Figs. 5-1 to 5-4) of the genus *Capra* of the family Bovidae, yak (denoted as "yak" in Figs. 5-1 to 5-4) of the yak species of the genus *Bos* of the family Bovidae, and sheep (denoted as "sheep" in Figs. 5-1 to 5-4) of the sheep species of the genus *Ovis* of the family Bovidae, and chromatograms of masses specific to various varieties are shown in Fig. 5-1 to Fig. 5-4.

[Example 5-2] Identification of unknown samples

(1) Preparation of extracts

Unknown samples (ranging from 15 mg to 25 mg) were prepared and the weights thereof were measured. In addition, theses samples can be conveniently prepared using a belt punch or the like.
The samples were crushed with a crusher, a protein extraction buffer was added to each sample, in a weight

30 times (μl) greater than that of the weight of the sample (mg), and then kept at a temperature. The protein extraction buffer had the same composition as that in Example 1.

(2) Enzyme treatment of extracts

35 μl of the thus obtained extract was mixed with 35 μl of a protease digestion buffer, and then a protease solution (lysyl endopeptidase (Lys-C)) was further added, followed by reaction at 37°C for a predetermined time length. In addition, the protease digestion buffer and the protease solution (lysyl endopeptidase (Lys-C)) had the same compositions as those in Example 3.

(3) Mass spectroscopy

Analysis was conducted by the method described in Example 5-1 (3) Mass spectroscopy.

(4)

Results were shown on a map (the horizontal axis: elution time, and the vertical axis: mass distribution).
Varieties can be identified by comparing with reference maps (maps prepared for various animal species of animal hair) using image analysis software and the like. MassLynx (Waters) was used as image analysis software, maps prepared for various animal species of animal hair are shown in Fig. 5-5 to Fig. 5-7.

[Example 6] Extraction from animal hair (also referred to as "raw wool" or "raw material") mixtures with varied mixture ratios, enzyme treatment, and mass spectroscopy (UPLC-MS analyzer)

(1) Preparation of extracts

Extracts were prepared in a manner similar to that in Example 5-1.
Each raw wool sample was prepared by mixing cashmere raw material (the raw wool of the cashmere species) with yak raw material (the raw wool of the yak species) at a mixture ratio = raw wool of the cashmere species : raw wool of the yak species = 100 : 0, 98 : 2, 95 : 5, 80 : 20, 65 : 35, 50 : 50, 35 : 65, 20 : 80, 5 : 95, or 0 : 100. An extract was obtained from each raw wool sample.

(2) Enzyme treatment of extracts

In a manner similar to Example 5-1, treatment with protease (lysyl endopeptidase (Lys-C)) was performed.

(3) Mass spectroscopy

In a manner similar to Example 5-1, Waters Acquity UPLC BEH C18 (2.1 mm × 100 mm, 1.7 μm) was used as a column, and analysis was conducted with a separation program (17 min).

(4) A method for calculating mixture ratio

An ion chromatogram of yak-species-specific mass-to-charge ratios is displayed, and the presence or the absence of ions is confirmed. When ions were detected, the peak area is calculated via analysis.
Moreover, the peak area of the mass-to-charge ratio of m/z = 778.90 detected commonly in cashmere and yak was also calculated via analysis.
Ratios of the peak area of the mass-to-charge ratios commonly detected in cashmere and yak to the peak area of yak-specific mass-to-charge ratios are plotted along the vertical axis, and the percentages of yak mixed in samples are plotted along the horizontal axis. In this graph, the above peak area ratios for the above-mixed raw wool samples were plotted to prepare a calibration curve.
The percentage of yak mixed in a sample was calculated from the above calibration curve and based on the peak area. Here, the percentage of cashmere species mixed in the same was found from a difference from 100% (specifically, by subtracting the percentage of yak from 100%).

(5) Analysis results

Fig. 6-1 shows the total mass ion chromatogram of each sample, and Fig. 6-2 shows an ion chromatogram (SIC) of the yak-species-specific mass-to-charge ratio of m/z = 683.35 (divalent molecular ions derived from the molecular mass (1364.70) corresponding to m/z = 1365.7). In the case of a mixture of cashmere raw material and yak raw material, the mass-to-charge ratios of m/z = 683.35 and 455.90, respectively, of multivalent molecular ions derived from the molecular mass (1364.70) corresponding to yak-species-specific mass-to-charge ratio of m/z = 1365.7 were confirmed successfully on the basis of the mixture ratio = cashmere raw material : yak raw material = 98 : 2. Mixture can be determined even if the percentage of yak mixed in a cashmere product is only 2%.

Moreover, a calibration curve prepared by plotting the peak areas and the percentages of cashmere or yak mixed in samples obtained from Figure 6-2 is shown in Fig. 6-3. In the case of mixture ratios = cashmere raw material : yak raw material = 98 : 2 to 0 : 100, a linear calibration curve can be prepared. According to the method of the present invention, when a cashmere product containing yak mixed therein is analyzed, the percentage of yak mixed therein, which is 2% or higher, can be specifically specified.

[Example 7] Extraction from animal hair (also referred to as "raw wool" or "raw material") subjected to dyeing treatment, or the like followed by enzyme treatment and mass spectroscopy

(UPLC-MS analyzer)

(1) Preparation of extracts

In a manner similar to Example 5-1, extracts were obtained from various types of raw wool subjected to dyeing treatment (metal complex dyeing, acid dyeing, reactive dyeing, and chrome dyeing), and various types of raw wool subjected to decoloring and then subjected to dyeing treatment (metal complex dyeing, acid dyeing, reactive dyeing, and chrome dyeing), and various types of raw wool subjected to water repellent finishing or softening.

In addition, the types of raw wool used herein were raw wool of the cashmere species of the genus *Capra* of the family Bovidae, that of the Angora species of the genus *Capra* of the family Bovidae, that of the yak species of the genus *Bos* of the family Bovidae, that of the sheep species of the genus *Ovis* of the family Bovidae, that of the genus *Camelus* of the family Camelidae, that of the alpaca species of the genus *Lama* of the family Camelidae (or the alpaca species of the genus *Vicugna),* and that of the Angora rabbit species of the genus *Oryctolagus* of the family Leporidae. Extracts were each obtained from these types of raw wool subjected to the above-mentioned treatments.

(2) Enzyme treatment of extracts

In a manner similar to Example 5-1, treatment with protease (lysyl endopeptidase (Lys-C) was performed.

(3) Mass spectroscopy

In a manner similar to Example 5-1, Waters Acquity UPLC BEH C18 (2.1 mm $\times$ 100 mm, 1.7 $\mu$m) was used as a column and analysis was conducted with a separation program (17 min).

(4) Analysis results

Analysis results are shown in Fig. 7-1 to Fig. 7-24.

[Example 8] Enzyme treatment and mass spectroscopy (MALDI-TOF mass spectroscope) of extracts from products

(1) Preparation of extracts

A sample (2 sheets in general. The number of such sheets to be used herein ranged from 2 to 5 depending on the thickness of the fabric) obtained by punching each product with a belt punch (5-mm diameter) was placed in a 1.5 ml tube. The weight was measured and the weight of an empty tube was subtracted from the total weight to find the weight of the sample. 500 $\mu$l of a protein extraction buffer (aqueous solution (pH9.5) containing 7 M urea, 0.05 M DTT (dithiothreitol) and 0.05 M Tris-HCl) was added to the sample and then the resultant was kept at 37°C overnight. After reaction, centrifugation was performed (5 minutes, 15000 rpm) at room temperature, and then the supernatant was collected, thereby preparing an extract.

Alternatively, the weight of each sample (4 sheets in general) obtained by punching each product with a belt punch (5-mm diameter) was measured in a manner similar to that described above. The resultant placed in a 2 ml tube was subjected to crushing using a bead crusher (Titec, $\mu$T-12) (specifically by running it for 30 seconds at 3000 rpm) and 5 zirconia beads with a diameter of 5 mm. This crushing was repeated 6 times. Subsequently, 500 $\mu$l of a protein

extraction buffer was added to the tube and then kept at 37°C overnight. After centrifugation, the supernatant was collected, thereby preparing an extract.

In addition, extracts were prepared from the animal hair product of the cashmere species (also referred to as "cashmere" or "cashmere product"), the animal hair product of the yak species (also referred to as "yak" or "yak product"), and the animal hair product of the sheep species (also referred to as "wool" or "wool product"), which had been identified according to the JIS (Japanese Industrial Standards) (identification by microscopy), and products containing the above animal species of animal hair, for which the percentages thereof mixed therein had been calculated according to the JIS (identification by microscopy).

(2) Enzyme treatment of extracts

In a manner similar to Example 3, treatment with protease (lysyl endopeptidase (Lys-C)) was performed.

(3) Mass spectroscopy

Analysis was conducted in a manner similar to Example 3.
To date,

- 24 samples of products with 100% cashmere label (identified) (also referred to as "100% cashmere" or 100% cashmere product"),
- 152 samples of products with 100% yak label (animal hair of the yak species) (identified) (also referred to as "100% yak" or "100% yak product"),
- 5 samples of products with 100% wool label (identified) (products with 100% sheep (animal hair of the sheep species) label (identified). Also referred to as "100% wool" or "100% wool product"), and
- 39 samples of products containing animal hair from various animal species mixed therein were analyzed.

Typical analysis results are shown in Fig. 8-1 to Fig. 8-3.

[Example 9] Enzyme treatment and mass spectroscopy (UPLC-MS analyzer) of extracts from products

(1) Preparation of extracts

In a manner similar to Example 8, extracts were prepared from products.

(2) Enzyme treatment of extracts

In a manner similar to Example 5-1, treatment with protease (lysyl endopeptidase (Lys-C) was performed.

(3) Mass spectroscopy

In a manner similar to Example 5-1, when Waters Acquity UPLC BEH C18 (2.1 mm $\times$ 100 mm, 1.7$\mu$m) was used as a column, analysis was conducted with a separation program (17 min). When Waters Acquity UPLC BEH C18 (2.1 mm $\times$ 50 mm, 1.7 $\mu$m) was used as a column, analysis was conducted with a separation program (10 min).

(4) Analysis results

To date,

- 16 samples of products with 100% cashmere label (identified) (also referred to as "100% cashmere" or 100% cashmere product"),
- 1 sample of products with 100% yak label (animal hair of the yak species) (identified) (also referred to as "100% yak" or "100% yak product"),
- 2 samples of products with 100% wool label (identified) (products with 100% sheep label (animal hair of the sheep species) (identified), also referred to as "100% wool" or "100% wool product"), and
- 36 samples of products containing hair form various animal species mixed therein were analyzed.

Among analysis results, the analysis results (total mass ion chromatogram) of the product with 100% cashmere label (identified) (denoted as "cashmere" in Figs. 9-1 to 9-4), the product with 100% yak label (identified) (denoted as "yak" in Figs. 9-1 to 9-4), and the product with 100% wool (identified) (denoted as "sheep" in Figs. 9-1 to 9-4), and the chromatograms of masses specific to various varieties are shown in Fig. 9-1 to Fig. 9-4.

[Example 10] Examination of amino acid sequences with the species-specific molecular masses (analysis of the relevant mass-to-charge ratios by MSMS)

(1) Preparation of extracts

In a manner similar to Example 5-1, extracts were each obtained from the animal hair of the cashmere species of the genus *Capra* of the family Bovidae, that of the yak species of the genus *Bos* of the family Bovidae, and that of the sheep species of the genus *Ovis* of the family Bovidae.

(2) Enzyme treatment of extracts

In a manner similar to Example 5-1, treatment with protease (lysyl endopeptidase (Lys-C) was performed.

(3) Mass spectroscopy

In a manner similar to Example 5-1, Waters Acquity UPLC BEH C18 (2.1 mm $\times$ 100 mm, 1.7$\mu$m) was used as a column and a separation program was performed (17 min). In addition, as mass (MSMS) analysis conditions, the mass ranging from 50 to 1500 Da and the collision voltage ranging from 25V to 35V were selected. As precursor ions,
**[0131]**    In the case of yak species-specific mass-to-charge ratios, the mass charge ratio of m/z = 683.35 of divalent molecular ions derived from the molecular mass (1364.70) corresponding to m/z = 1365.7 was selected.
In the case of cashmere-species-specific mass-to-charge ratios, the mass-to-charge ratio of m/z = 740.4 of trivalent molecular ions derived from the molecular mass (2218.19) corresponding to m/z = 2219.19 was selected.
In the case of sheep-species-specific mass-to-charge ratios, the mass-to-charge ratio of m/z = 620.8 of tetravalent molecular ions derived from the molecular mass (2479.3) corresponding to m/z = 2480.3, and, the mass-to-charge ratio of m/z = 692.4 of trivalent molecular ions derived from the molecular mass (2074.12) corresponding to m/z = 2075.12 were selected.

(4) Analysis results

Fig. 10-1 shows the analysis results of the yak species-specific mass-to-charge ratio of m/z = 683.35. Based on the detected masses, the yak species-specific mass-to-charge ratio of m/z = 683.35 was found to correspond to molecular ions resulting from a peptide and/or a protein comprising the amino acid sequence represented by F A A F (L or I) D K.
Fig. 10-2 shows the analysis results of the cashmere species-specific mass-to-charge ratio of m/z = 740.4. Based on the detected masses, the cashmere species-specific mass-to-charge ratio of m/z = 740.4 was found to correspond to molecular ions resulting from a peptide and/or a protein comprising the amino acid sequence represented by SD (L or I) EAVEQS (L or I) QMQFDVRPK.
Fig. 10-3 shows the analysis results of the sheep species-specific mass-to-charge ratio of m/z = 620.8. Based on the detected masses, the cashmere species-specific mass-to-charge ratio of m/z = 620.8 was found to correspond to molecular ions resulting from a peptide and/or a protein comprising at least one amino acid sequence represented by QF (L or I), FENEY, QNFVA (L or I) Q or an amino acid sequence comprising amino acid residues aligned in an order opposite thereto.
Fig. 10-4 shows the analysis results of the sheep species-specific mass-to-charge ratio of m/z = 692.4. Based on the detected masses, the cashmere species-specific mass-to-charge ratio of m/z = 692.4 was found to correspond to molecular ions resulting from a peptide and/or a protein comprising at least one amino acid sequence represented by SD (L or I) E, (Q or K) EE (L or I) (L or I) C (L or I) K or an amino acid sequence comprising amino acid residues aligned in an order opposite thereto.

[Example 11] Examination of the nucleotide sequence of yak species-specific molecular mass

(1) Extraction of DNA

25 mg of a sample was weighed out into a 1.5-ml tube, and then 25 $\mu$l of Proteinase K was added to 475 $\mu$l of a DNA extraction solution (50 mM Tris-HCl, 5 mM CaCl$_2$, 1 mM EDTA, 50 mM DTT, 2M GuHCl) to a volume of 500 $\mu$l, followed by 2 hours of reaction at 37°C. After the completion of the reaction, the resultant was subjected to centrifugation at 15,000 rpm for 5 minutes to collect the supernatant, thereby preparing a DNA solution. 15 µl of glass powder was added to the collected DNA solution. The solution was stirred well, and then left to stand at room temperature for 5 minutes. After 5 minutes, the solution was centrifuged at 10,000 rpm for 10 seconds. The supernatant was removed as far as possible, 750 µl of a washing buffer was added, pipetting was performed, and then centrifugation was performed at 10,000 rpm for 10 seconds. 750 µl of a washing buffer was added again, and then the same procedure was performed. Centrifugation was performed again to thoroughly remove a small amount of liquid adhered to the wall. 30 µl of TE buffer was added to the precipitate, suspension was performed well, and then the solution was kept at 55°C for 2 minutes. After 5 minutes, centrifugation was performed at 10,000 rpm for 10 seconds, and then 28 µl of the supernatant was collected. 30 µl of TE buffer was added again to the precipitate, and thus extraction was repeated. At the 2$^{nd}$ extraction, 30 µl of the supernatant was collected.

(2) PCR (1$^{st}$ ·outside)

After PCR was performed once with the extracted DNA, PCR was further performed using the product as a template. TaKaRa Taq™ (Takara Bio Inc.) was used for the 1$^{st}$ PCR for the outside. First, the 1$^{st}$ reaction composition is as follows: 5 µl of 10×PCR Buffer, 4 µl of dNTP Mixture, 1 µl of 100 pmol/µl primer_F out (5' CTC ACA CCC CTC AAC CTG GAG ATT GAC CCC AA 3'), 1 µl of 100 pmol/µl primer R out (5' GTT CTG ATA GAA CTG CAG CTT GGT CTC CAG 3'), 3 µl of the collected DNA solution, and 0.5 µl of Taq DNA Polymerase (5 U/µl). 35.5µl of dH$_2$O was added to a volume of 50 µl. The PCR program was comprised of a preliminary denaturation step (94°C for 2 minutes), a denaturation step (94°C for 0.5 minutes), an annealing step (55°C for 0.5 minutes), an extension step (72°C for 0.5 minutes), 4°C after reaction, and 35 cycles of the reaction.

After the completion of the reaction, 125 µl of ethanol was added to 50 µl of the PCR solution and then left to stand overnight at -80°C. On the next day, centrifugation was performed at 15,000 rpm for 30 minutes. The supernatant was discarded after centrifugation, 1 ml of 70% ethanol was added and then centrifugation was performed for 5 minutes, followed by 10 minutes of drying with a desiccator. After 10 minutes, the precipitate was dissolved in 15 µl of TE buffer.

(3) PCR (2nd·inside)

The portion further inside the DNA was amplified using the DNA amplified by the 1$^{st}$ PCR as a template. The 2$^{nd}$ PCR was performed using 2$^{nd}$ primers, primer_F in (5'GAG AAG GAG CAG ATC AAG 3') and primer_R in (5'TTC CTG GAG CAG CAG AAC3'). As an enzyme, TaKaRa Taq™ (Takara Bio Inc.) was used similar to that in the 1$^{st}$ PCR. The reaction composition is as follows: 0.5 µl of Taq DNA Polymerase (5 U/µl), 5 µl of 10 × PCR buffer, 4 µl of dNTP Mixture, 3 µl of the DNA solution (from among 15 µl), 1 µl of primer_F in, and 1 µl of primer_R in. 35.5 µl of dH$_2$O was added to a volume of 50 µl. The PCR program was comprised of a preliminary denaturation step (94°C for 2 minutes), a denaturation step (94°C for 0.5 minutes), an annealing step (40°C for 0.5 minutes), an extension step (72°C for 0.5 minutes), 4°C after the reaction, and 35 cycles of the reaction. After the completion of the reaction, 3% agarose electrophoresis was performed, thereby confirming the amplification of the DNA. Subsequently, a target band was excised and then added into an Eppendorf tube.

(4) Purification of DNA

The thus excised gel was frozen in a freezer at -80°C for about 10 minutes. The frozen gel was transferred into a cup within a filtration tube (Ultrafree-MC) and then centrifuged at 10,000 rpm and 4°C for 20 minutes. 3M NaCl in a volume one tenth the volume of a filtrate and 10 A$_{260}$ RNA$^{Mix}$ were added to the filtrate, ethanol in an amount 2.5 times greater than that of the solution was added to the solution and then the solution was left to stand at -80°C for at least 10 minutes. Subsequently, centrifugation was performed at 15,000 rpm and 4°C for 30 minutes. The supernatant was removed after centrifugation, 1 ml of 70% ethanol was added and then centrifugation was further performed at 15,000 rpm and 4°C for 5 minutes. The supernatant was removed after centrifugation, and then the precipitate was dried under reduced pressure using a desiccator.

(5) Ligation reaction and transformation

The precipitate was subjected to a blunt ending reaction using Klenow Fragment (Takara Bio Inc.). The reaction composition is as follows: DNA (precipitate) amplified by PCR, 2.5 µl of 1O×Klenow Fragment Buffer, 2 µl of 2.5 mM dNTP, and 0.1 U of Klenow Fragment. Sterile water was added to a volume of 25 µl. The resultant was subjected to 30 minutes of reaction at 37°C, and then the enzyme was inactivated at 70°C for 10 minutes. The blunt-ended PCR product and plasmid DNA, pUC19 (0.1 µg), digested with a restriction enzyme *Hinc* II were mixed. 3 M NaCl, in an amount one tenth the mixed solution, and 10 A$_{260}$ RNA$^{Mix}$ were added to the mixed solution. Ethanol was added in an amount 2.5 times greater than that of the mixed solution and then it was left to stand at -80°C for at least 10 minutes. Subsequently, centrifugation was performed at 15,000 rpm and 4°C for 30 minutes. The supernatant was removed after centrifugation, 1 ml of 70% ethanol was added, and then centrifugation was further performed at 15,000 rpm and 4°C for 5 minutes. The supernatant was removed after centrifugation and then the precipitate was dried under reduced pressure using a desiccator. After drying, a ligation reaction was performed for at least 30 minutes using Ligation Pack (Nippon Gene Co., Ltd.) at 16°C. The reaction composition is as follows: DNA (precipitate) amplified by PCR, pUC19 (precipitation), 2 $\mu$l of 10 $\times$ Ligation Buffer, 2.5 $\mu$l of 2 mg/ml BSA Solution, 1.5 $\mu$l of T4 DNA ligase, and 1 $\mu$l of 20 mmol/l Spermidine. Sterile water was added to an amount of 20 $\mu$l. After reaction, 10 $\mu$l of Ligation reaction solution was added to competent cells JM109. The resultant was left to stand on ice for 5 minutes and then spread over an LB-amp plate (containing X-Gal and IPTG), and then left to stand overnight at 37°C.

(6) Preparation of plasmid DNA

On the next day, the thus obtained white colonies were inoculated to 4 ml of an LB-amp medium, and then cultured overnight at 37°C. On the next day, 1.5 ml of the culture solution was dispensed into an Eppendorf tube, followed by 2 minutes of centrifugation at 15,000 rpm and room temperature. The medium was removed as far as possible, 1.5 ml of the culture solution was further added, and then centrifugation was performed at 15,000 rpm and room temperature for 2 minutes. After the medium was removed as far as possible, the cells were suspended well in 100 $\mu$l of Solution I (25 mM Tris-HCl (pH 8.0), 10 mM EDTA, 50 mM glucose). 200 $\mu$l of Solution II (2 M NaOH, 1% SDS) was added, and then the tube was shaken up and down for gentle stirring. 150 $\mu$l of Solution III (potassium acetate, glacial acetic acid) was further added and then the tube was immediately shaken up and down for stirring. 150 $\mu$l of phenol·chloroform was added, suspension was performed sufficiently, and then centrifugation was performed at 15,000 rpm and room temperature for 5 minutes. 400 $\mu$l of the top layer was absorbed and then transferred into a new tube. Ethanol was added to the resultant in an amount 2.5 times greater than that of the resultant and then left to stand at -80°C for at least 10 minutes. Subsequently, centrifugation was performed at 15,000 rpm and 4°C for 10 minutes. The supernatant was removed after centrifugation and then dissolved in 50 $\mu$l of TE buffer. 50 $\mu$l of 2 M NaCl and 0.2M MgCl$_2$ were added to and mixed well with the solution, and then the mixture was left to stand on ice for 10 minutes. After 5 minutes of centrifugation at 15,000 rpm and 4°C, the supernatant was transferred into another tube, 200 $\mu$l of ethanol was added, and then centrifugation was performed at 15,000 rpm and 4°C for 5 minutes. The supernatant was removed after centrifugation, 1 ml of 70% ethanol was added, and then centrifugation was performed at 15,000 rpm and 4°C for 3 minutes. The supernatant was removed after centrifugation, and then dried using a desiccator for 10 minutes. The precipitate was dissolved in 30 $\mu$l of sterile water. PCR was performed using 1 $\mu$l of the resultant as a template under the same conditions as those for the 2$^{nd}$ PCR. After PCR, 3% agarose electrophoresis was performed to confirm if cloning had been successfully carried out. Sequence confirmation was performed for those considered to have undergone successful cloning.
The DNA sequence was found to be GGT CTC AAC AAC AGG TTT GCT GCC TTC ATC GAC AAG GTG CGC.
When the DNA sequence was replaced by the amino acid sequence, the amino acid sequence was found to be G L N N R F A A F I D K. When the molecular mass of the sequence was calculated, it was found to be 1364.7 Da, indicating the mass-to-charge ratio (m/z = 1365.7) of molecular ions.

[Example 12] Examination of quantification of yak (animal hair of the yak species) using yak species-specific molecular masses

(1) Preparation of extracts

In a manner similar to Example 5-1, an extract was obtained from the animal hair of the yak species of the genus *Bos* of the family Bovidae.

(2) Enzyme treatment of extracts

A yak protein extract (protein concentration of 5.3 mg/ml) was demineralized by dialysis. The yak extract corresponding to the protein amount of 150 $\mu$g was treated with protease (lysyl endopeptidase (Lys-C)) in a manner similar to Example 5-1.

(3) Mass spectroscopy

In a manner similar to Example 5-1, analysis was conducted using Waters Acquity UPLC BEH C18 (2.1 mm $\times$ 100 mm, 1.7$\mu$m) as a column. In addition, an analytical sample was prepared by adding 1 $\mu$g of the synthetic peptide of G L N N R F A A F I D K (where "L" was composed of stable isotope 13C and 15N: molecular mass = 1371.66 Da) to a solution obtained by treating the yak extract with the enzyme, so that the total amount thereof was 100 µl. An injection volume was 4 µl. A separation program was comprised of 20 min. Linear gradients employed herein are as follows: 0 min (A : B = 90 : 10), 0-14 min (A: B = 50 : 50), 14-14.5 min (A: B = 10 : 90), 14.5-17.5 min (A: B = 10 : 90), 17.5-17.6 min (A : B = 90 : 10), and 17.6-20 min (A : B = 10 : 90). (4) A method for quantifying an unknown sample is as described below.

Extraction and mass spectroscopy can also be performed by a method similar to that in Example 13.

(i) An ion chromatogram of yak-species-specific mass-to-charge ratios is displayed and the presence or the absence of ions is confirmed. When ions are detected, the peak area is calculated via analysis. Next, molecular ions derived from a synthetic peptide (molecular mass=1371.66 Da) is also analyzed to calculate the peak area.

(ii) The synthetic peptide-derived peak area is converted into a concentration or an amount based on the concentration thereof, the resultant is compared with the peak area derived from an extracted fluid sample, and thus the peptide determined to be of the yak species, which is contained in the extracted fluid sample, is quantified.

(5) Analysis results

Analysis results are shown in Fig. 11.

The total mass ion chromatogram is on the top, the ion chromatogram (SIC) of the yak species-specific mass-to-charge ratio of m/z = 683.35 (divalent molecular ions derived from molecular mass (1364.70) corresponding to m/z = 1365.7) is on the middle, and the ion chromatogram (SIC) of the mass-to-charge ratio of m/z = 686.83 (divalent molecular ions derived from molecular mass (1371.66) corresponding to m/z = 1372.66) of the synthetic peptide of G L N N R F A A F I D K (where "L" is composed of stable isotopes 13C and 15N) is on the bottom.

Based on the ion chromatogram on the bottom, the peak area of the mass-to-charge ratio (m/z = 686.83) of the synthetic peptide is 248.13 per 0.04 µg of the amount of the synthetic peptide; that is, 0.000161 µg (0.161 ng) per peak area. Based on the ion chromatogram on the middle, the peak area of the yak species-specific mass-to-charge ratio of (m/z = 683.35) is 31.74; that is, 0.0051 µg (5.1 ng) in terms of the amount of the peptide.

Accordingly, if an extract corresponding to 150 µg of the protein was analyzed under the above conditions, and the yak species-specific peptide sequence of G L N N R F A A F I D K is revealed to be present in an amount of 0.0051 µg (5.1 ng), indicating 100% yak. Therefore, it was revealed that specification and/or quantification of the animal hair of the yak species can be performed based on the presence or the absence of the molecular ions resulting from a peptide and/or a protein comprising the sequence, and the ion chromatogram·peak area of specific masses.

[Example 13]

(1) Preparation of extracts

After crushing with a crusher in a manner similar to Example 8, a protein extraction buffer was added to a sample prepared by punching with a belt punch, in an amount (µl) 30 times greater than the weight (mg) of the sample, then extraction was performed.

In addition, identification was performed according to the JIS (Japanese Industrial Standards) (identified by microscopy).

Extracts were obtained from product samples prepared by mixing:

the animal hair product of the cashmere species (also referred to as "cashmere" or "cashmere product");
the animal hair product of the yak species (also referred to as "yak" or "yak product") ; and
the animal hair product of the sheep species (also referred to as "wool" or "wool product"), at the following mixture ratios (the number of samples obtained using a belt punch, weights, and the percentage of cashmere, yak, or wool mixed therein (wt%)).

- 4 cashmere samples (16.7 mg) : 0 wool sample
  _the percentage of wool mixed therein: 0%
- 3 cashmere samples (12.24 mg) : 1 wool sample (6.05 mg)
  _the percentage of wool mixed therein: 33.08%
- 2 cashmere samples (8.76 mg) : 2 wool samples (11.53 mg)
  _the percentage of wool mixed therein: 56.83%
- 1 cashmere sample (4.38 mg) : 3 wool samples (17.17 mg)
  _the percentage of wool mixed therein: 79.68%

- 0 cashmere sample : 4 wool samples (22.62 mg)
  _the percentage of wool mixed therein 100%
- 4 cashmere samples (17.92 mg) : 0 yak sample
  _the percentage of yak mixed therein: 0%
- 3 cashmere samples (13.74 mg) : 1 yak sample (5.30 mg)
  _the percentage of yak mixed therein: 27.84%
- 2 cashmere samples (8.25 mg) : 2 yak samples (10.01 mg)
  _the percentage of yak mixed therein: 54.82%
- 1 cashmere sample (3.97 mg) : 3 yak samples (15.64 mg)
  _the percentage of yak mixed therein: 79.76%
- 0 cashmere sample : 4 yak samples (19.95 mg)
  _the percentage of yak mixed therein: 100%

(2) Enzyme treatment of protein extracts

In a manner similar to Example 5-1, treatment with protease (lysyl endopeptidase (Lys-C) was performed. 35 $\mu$l of the thus-obtained extract was mixed with 35 $\mu$l of a protease digestion buffer. 2.5 $\mu$l of a protease solution (prepared by dissolving 20 $\mu$g of lysyl endopeptidase (Lys-C) in 400 $\mu$l of 2mM Tris-HCl (pH8.0)) was added, followed by 2.5 hours of reaction at 37°C.

(3) Mass spectroscopy

Analysis was conducted by the method described in Example 5-1 (3) Mass spectroscopy. Waters Acquity UPLC BEH C18 (2.1 mm $\times$ 100 mm, 1.7$\mu$m) was used as a column, and analysis was conducted with a separation program (17 min).

(4) A method for calculating the percentages of hair of animal species mixed in an unknown sample using the Examples is as follows.

An ion chromatogram of mass-to-charge ratios specific to each species (e.g., yak species and sheep species) is displayed, thereby confirming the presence or absence of ions. When ions are detected, the percentage of yak mixed therein is calculated from the calibration curve based on the peak area. The percentage of the animal hair of the cashmere species mixed therein is found a difference from 100% (specifically, by subtracting the percentage of the percentage of the yak species from 100%).

(5) Analysis results

Fig. 12-1 shows the total mass ion chromatogram of each sample (cashmere and yak). Fig. 12-2 shows the ion chromatogram (SIC) of the yak-species-specific mass-to-charge ratio of m/z = 683.35 (divalent molecular ions derived from molecular mass (1364.70) corresponding to m/z = 1365.7). Fig. 12-3 shows the total mass ion chromatogram of each sample (cashmere and sheep). Figure 12-4 shows the ion chromatogram (SIC) of the sheep-species-specific mass-to-charge ratio of m/z = 620.82 (tetravalent molecular ions derived from the molecular mass (2480.298) corresponding to m/z = 2480.298).

Moreover, a calibration curve prepared by plotting the peak areas and the percentages of cashmere/yak mixed therein (wt%) obtained from Fig. 12-2 is shown in Fig. 12-5. A calibration curve prepared by plotting the peak areas and the percentages of cashmere/sheep mixed therein (wt%) obtained from Fig. 12-4 is shown in Figure 12-6.

Industrial Applicability

Conventional visual differentiation is performed on the basis of the appearance of animal hair as a criteria, and thus is problematic in that identification results may differ depending on the skill of a differentiation expert and a method for treating animal hair or the product thereof, and the number of samples that can be identified per day is limited. In contrast, the present invention makes it possible to objectively identify a large number of samples within a short time with/using convenient procedures.

Moreover, unlike differentiation using DNA, constituent molecules of animal hair are directly analyzed according to the present invention, and thus the method of invention can exert an excellent effect such that a risk of being unable to analyze/differentiate depending on products is very low.

In particular, the present invention also exerts an excellent effect such that the presence or the absence of yak mixed in a cashmere product or a wool product and/or the percentage of yak mixed in such a product can be reliably identified within a short time.

All publications, patents, and patent applications cited herein are incorporated herein by reference in their entirety.

**Claims**

1. A method for specifying animal species of animal hair and/or quantifying the animal hair contained in a sample, comprising the steps of:

    (a) obtaining an analytical sample from the sample by pretreatment;
    (b) analyzing the analytical sample obtained in the step (a) using mass spectrometry and then obtaining a result; and
    (c) specifying animal species of the animal hair and/or quantifying the animal hair from the result obtain in the step (b).

2. The method according to claim 1, wherein, in addition to the mass spectrometry, liquid chromatography is used in combination with mass spectroscopy (also referred to as "LC-MS" or "UPLC-MS").

3. The method according to any one of claims 1 and 2, wherein the animal species is one or more species selected from the group consisting of a cashmere species of the genus *Capra* of the family Bovidae, an Angora species of the genus *Capra* of the family Bovidae, a yak species of the genus *Bos* of the family Bovidae, a sheep species of the genus *Ovis* of the family Bovidae, the genus *Camelus* of the family Camelidae, an alpaca species of the genus *Lama* of the family Camelidae (or an alpaca species of the genus *Vicugna*), and an Angora rabbit species of the genus *Oryctolagus* of the family Leporidae.

4. The method according to any one of claims 1 to 3, wherein the step (c) comprises comparing the result obtained in the step (b) with at least one of reference data obtained by performing the steps (a) and (b) under the same conditions for reference animal hair sample of which the animal species have been confirmed in advance, and then specifying animal species of the animal hair and/or quantifying the animal hair based on the masses, and/or the mass-to-charge ratios (also referred to as "m/z"), and/or the elution times of the relevant mass-to-charge ratios, detected specifically to the animal species of the animal hair.

5. The method according to claim 4, wherein in the step of specifying animal species of an animal hair and/or quantifying the animal hair, the reference data correspond to one or more types of mass and/or mass-to-charge ratio selected from the following masses and mass-to-charge ratios:

    (I) yak species

(1) the mass detected specifically to the yak species:

5948.90 ± 0.02%, or 2137.92 ± 0.02%

(2) the mass-to-charge ratio (m/z) of molecular ions resulting from the mass or another mass-to-charge ratio (m/z) detected specifically to the yak species:

9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, or 1069.98 ± 0.02%

(II) cashmere species

(1) the mass detected specifically to the cashmere species:

8976.45 ± 0.02%, 7102.18 ± 0.02%, or 7074.18 ± 0.02%,

(2) the mass-to-charge ratio (m/z) of molecular ions resulting from the mass or another mass-to-charge ratio (m/z) detected specifically to the cashmere species:

10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, or 1415.83 ± 0.02%

(III) sheep species

(1) the mass detected specifically to the sheep species: 17538.88 ± 0.02% or 17584.26 ± 0.02%
(2) the mass-to-charge ratio (m/z) of molecular ions resulting from the mass or another mass-to-charge ratio (m/z) detected specifically to the sheep species: 10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, or 1599.55 ± 0.02%

(IV) Angora species (also referred to as "mohair")

(1) the mass detected specifically to the Angora species: 6329.52 ± 0.02%
(2) the mass-to-charge ratio (m/z) of molecular ions calculated from the mass of (1)

(V) alpaca species of the genus *Lama* of the family Camelidae (or alpaca species of the genus *Vicugna*)

(1) the mass detected specifically to the alpaca species:

6358.60 ± 0.02%, or 6342, 70 ± 0.02%

(2) the mass-to-charge ratio (m/z) of molecular ions calculated from the mass of (1)

(VI) Angora rabbit species of the genus *Oryctolagus* of the family Leporidae (also referred to as "Angora")

(1) the mass detected specifically to the Angora rabbit species: 1042.43 ± 0.02%
(2) the mass-to-charge ratio (m/z) of molecular ions calculated from the mass of (1).

6. The method according to any one of claims 1 to 3, wherein the step (a) of obtaining an analytical sample from the sample by pretreatment includes a step of using lysyl endopeptidase (also referred to as "Lys-C").

7. The method according to claim 6, wherein the step (c) comprises comparing the result obtained in the step (b) with at least one of reference data obtained by performing the steps (a) and (b) under the same conditions for reference animal hair sample of which animal species have been confirmed in advance, and then specifying animal species of the animal haire and/or quantifying the animal hair based on the masses, and/or the mass-to-charge ratios (also referred to as "m/z"), and/or the elution times of the relevant mass-to-charge ratios, detected specifically to the animal hair of the animal species.

8. The method for specifying an animal species of animal hair according to claim 6, wherein the step (c) comprises displaying the result obtained in the step (b) by data processing on a map representing elution times, detected mass-to-charge ratios, and detected intensities, comparing the result with at least one of reference data obtained by performing the steps (a) and (b) under the same conditions for reference animal hair sample of which animal species have been confirmed in advance, and then specifying animal species of the animal hair based on the degree of correlation.

9. The method according to claim 7, wherein in the step of specifying an animal species of animal hair and/or quantifying the animal hair, the reference data correspond to one or more types of mass and/or mass-to-charge ratio selected from the following masses and mass-to-charge ratios,

(I) yak species of the genus *Bos* of the family Bovidae

(1) the mass detected specifically to the yak species:

1364.70 ± 0.02%, 1383.72 ± 0.02%, 1431.67 ± 0.02%, 2469.27 ± 0.02%, or 2495.30 ± 0.02%

(2) the mass-to-charge ratio (m/z) of molecular ions calculated from the mass of (1)

(II) cashmere species of the genus *Capra* of the family Bovidae

(1) the mass detected specifically to the cashmere species:

2218.19 ± 0.02%, 3379.48 ± 0.02%, or 2391.99 ± 0.02%,

(2) the mass-to-charge ratio (m/z) of molecular ions calculated from the mass of (1)

(III) sheep species of the genus *Ovis* of the family Bovidae

(1) the mass detected specifically to the sheep species,
2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, or 2174.00 ± 0.02%,
(2) the mass-to-charge ratio (m/z) of molecular ions calculated from the mass of (1)

(IV) the genus *Camelus* of the family Camelidae

(1) the mass detected specifically to the genus *Camelus*,
2888.35 ± 0.02%,
(2) the mass-to-charge ratio (m/z) of molecular ions calculated from the mass of (1)

(V) alpaca species of the genus *Lama* of the family Camelidae (or alpaca species of the genus *Vicugna*)

(1) the mass detected specifically to the alpaca species (or the alpaca species of the genus *Vicugna*),
4599.37 ± 0.02%, 901.47 ± 0.02%, or 532.32 ± 0.02%,
(2) the mass-to-charge ratio (m/z) of molecular ions calculated from the mass of (1)

(VI) Angora rabbit species of the genus *Oryctolagus* of the family Leporidae

(1) the mass detected specifically to the Angora rabbit species,
2303.07 ± 0.02%, 2502.15 ± 0.02%, 2058.02 ± 0.02%, or 1554.81 ± 0.02%,
(2) the mass-to-charge ratio (m/z) of molecular ions calculated from the mass of (1).

10. The method according to claim 7 or 9, wherein the above reference data are yak-species-specific mass-to-charge ratios and are the mass-to-charge ratios of molecular ions resulting from a peptide and/or a protein comprising the amino acid sequence represented by SEQ ID NO: 1.

11. An animal hair marker, which is any one of the following (1) to (5):

(1) a yak-specific peptide marker, which is shown in SEQ ID NO: 1;
(2) a cashmere-specific peptide marker, which is shown in SEQ ID NO: 3;
(3) a sheep-specific peptide marker, which contains a combination of 2 or more amino acid sequences shown in SEQ ID NOS: 4 to 9;
(4) a sheep-specific peptide marker, which contains a combination of 2 or more amino acid sequences shown in SEQ ID NOS: 10 to 13;
(5) a yak-specific nucleic acid marker, which is shown in SEQ ID NO: 2.

12. A method for calculating the percentages of 2 animal species of animal hair mixed in a sample, comprising the steps of

(a) obtaining an analytical sample from the sample by pretreatment;
(b) analyzing the analytical sample obtained in the step (a) using mass spectrometry and then obtaining a result;
(c) after the steps (a) and (b) are performed under the same conditions,
drawing an ion chromatogram displaying only the mass-to-charge ratios specific to one of the two animal species of animal hair, which are extracted from the total mass ion chromatogram of a sample containing the relevant animal hair of the animal species, performing data processing for the chromatogram, and then calculating the peak area of the animal-species-specific mass-to-charge ratios; and
(d) calculating the percentages of the animal hair of the animal species mixed in the sample from the peak area.

13. The method according to claim 12, wherein the following steps are added before the step (d):

(i) drawing an ion chromatogram of mass-to-charge ratios (m/z) derived from common molecular species shared by 2 animal species of animal hair, selecting the common mass-to-charge ratios of hair of each animal species, and then finding the peak area of the common mass-to-charge ratios;
(ii) calculating the peak area of animal-species-specific mass-to-charge ratios with respect to the peak area of the common mass-to-charge ratios.

14. The method according to 12 or 13, comprising plotting the peak areas and the percentages of animal species of animal hair mixed in a sample prepared by artificially mixing reference animal hair in the sample, the animal hair of which animal species have been confirmed in advance, on a graph, preparing a calibration curve, and then calculating the percentage of an animal species of animal hair mixed in an unknown sample from the peak area using the calibration curve.

15. A method for specifying 1 or more animal species of animal hair in a sample, and calculating the percentages of the animal hair of the animal species mixed in the sample when the sample contains animal hair of 2 or more animal species, comprising the steps of:

(a) obtaining an analytical sample from the sample by pretreatment;
(b) analyzing the analytical sample obtained in the step (a) using mass spectrometry and then obtaining a result;
(c) specifying the animal species of animal hair based on the result obtained in the step (b);
(d) when 2 or more animal species of animal hair are specified, calculating the peak area of the specified-animal-specific mass-to-charge ratios for each of the thus identified animal species; and
(e) calculating the percentage of animal hair of each animal species mixed in the sample through comparison with the peak area of a reference sample containing the 100% animal species.

16. The method according to claim 15, wherein the following steps are added before the step (e):

(i) drawring an ion chromatogram of mass-to-charge ratios (m/z) derived from the common molecular species shared by animal species of animal hair mixed in a sample, selecting the common mass-to-charge ratios of hair of each animal species, and then finding the peak area of the common mass-to-charge ratios;
(ii) calculating the peak area of animal-species-specific mass-to-charge ratios with respect to the peak area of the common mass-to-charge ratios.

17. The method according to any one of claims 12 to 16, comprising calculating the percentages of animal hair of 2 or more animal species (hereinafter, referred to as "N" species) mixed in a sample containing animal hair of "N-1" species, and then finding the percentages of N species of animal hair mixed therein based on a difference from 100%.

18. The method according to any one of 12 to 17 for calculating the percentage of one or more animal species of animal hair mixed in a sample containing animal hair of 1 or more animal species and non-animal-hair fibers, comprising a step of calculating the mixture ratio of non-animal-hair fibers to animal hair by the release method, the dissolution method, or the like specified in the JIS L1030, before the step (a) of obtaining an analytical sample from the sample by pretreatment, or, after a step of calculating the percentage of animal species of animal hair mixed in the sample.

19. The method according to any one of claims 12 to 18, wherein the mass-to-charge ratio specific to an animal species is one or more mass-to-charge ratios selected from the following mass-to-charge ratios,

(I) yak species

(1) the mass detected specifically to the yak species:

5948.90 ± 0.02%, or 2137.92 ± 0.02%

(2) the mass-to-charge ratio (m/z) of molecular ions calculated from the mass of (1) or another mass-to-charge ratio (m/z) detected specifically to the yak species: 9255.5 ± 0.02%, 7392.6 ± 0.02%, 5908.7 ± 0.02%, 5543.1 ± 0.02%, 5949.90 ± 0.02%, 1983.52 ± 0.02%, 1487.64 ± 0.02%, 1191.52 ± 0.02%, 2138.92 ± 0.02%, or 1069.98 ± 0.02%

(II) cashmere species

(1) the mass detected specifically to the cashmere species:

8976.45 ± 0.02%, 7102.18 ± 0.02%, or 7074.18 ± 0.02%,

(2) the mass-to-charge ratio (m/z) of molecular ions calculated from the mass of (1) or another mass-to-charge ratio (m/z) detected specifically to the cashmere species:

10384.2 ± 0.02%, 8976.9 ± 0.02%, 6662.2 ± 0.02%, 6093.2 ± 0.02%, 6002.2 ± 0.02%, 8977.45 ± 0.02%, 2244.50 ± 0.02%, 1795.78 ± 0.02%, 7103.18 ± 0.02%, 1776.54 ± 0.02%, 1421.44 ± 0.02%, 7075.18 ± 0.02%, 1769.55 ± 0.02%, or 1415.83 ± 0.02%

(III) sheep species

(1) the mass detected specifically to the sheep species:

17538.88 ± 0.02% or 17584.26 ± 0.02%

(2) the mass-to-charge ratio (m/z) of molecular ions calculated from the mass of (1) or another mass-to-charge ratio (m/z) detected specifically to the sheep species: 10339.6 ± 0.02%, 8954.6 ± 0.02%, 6665.7 ± 0.02%, 6036.4 ± 0.02%, 17539.88 ± 0.02%, 1949.84 ± 0.02%, 1754.82 ± 0.02%, 1595.47 ± 0.02%, 17585.26 ± 0.02%, 1955.10 ± 0.02%, 1759.47 ± 0.02%, or 1599.55 ± 0.02%

(IV) Angora species (also referred to as "mohair")

(1) the mass detected specifically to the Angora species: 6329.52 ± 0.02%
(2) the mass-to-charge ratio (m/z) of molecular ions calculated from the mass of (1)

(V) alpaca species of the genus *Lama* of the family Camelidae (or alpaca species of the genus *Vicugna*)

(1) the mass detected specifically to the alpaca species:

6358.60 ± 0.02% or 6342.70 ± 0.02%

(2) the mass-to-charge ratio (m/z) of molecular ions calculated from the mass of (1)

(VI) Angora rabbit species of the genus *Oryctolagus* of the family Leporidae (also referred to as "Angora")

(1) the mass detected specifically to the Angora rabbit species:

1042.43 ± 0.02%

(2) the mass-to-charge ratio (m/z) of molecular ions calculated from the mass of (1).

20. The method according to any one of claims 12 to 18, wherein the step (a) of obtaining an analytical sample from the sample by pretreatment includes a step of using lysyl endopeptidase (also referred to as "Lys-C"), and the animal-species-specific mass-to-charge ratio is 1 or more mass-to-charge ratios selected from the following mass-to-charge ratios.

(I) yak species of the genus *Bos* of the family Bovidae

(1) the mass detected specifically to the yak species
1364.70 ± 0.02%, 1383.72 ± 0.02%, 1431.67 ± 0.02%, 2469.27 ± 0.02%, or 2495.30 ± 0.02%
(2) the mass-to-charge ratio (m/z) of molecular ions calculated from the mass of (1)

(II) cashmere species of the genus *Capra* of the family Bovidae

(1) the mass detected specifically to the cashmere species,
2218.19 ± 0.02%, 3379.48 ± 0.02%, or 2391.99 ± 0.02%,
(2) the mass-to-charge ratio (m/z) of molecular ions calculated from the mass of (1)

(III) sheep species of the genus *Ovis* of the family Bovidae

(1) the mass detected specifically to the sheep species,
2479.30 ± 0.02%, 4977.24 ± 0.02%, 2340.03 ± 0.02%, 2358.02 ± 0.02%, 1051.41 ± 0.02%, 1033.39 ± 0.02%, 2074.12 ± 0.02%, or 2174.00 ± 0.02%,
(2) the mass-to-charge ratio (m/z) of molecular ions calculated from the mass of (1)

(IV) the genus *Camelus* of the family Camelidae

(1) the mass detected specifically to the genus *Camelus,* 2888.35 ± 0.02%,
(2) the mass-to-charge ratio (m/z) of molecular ions calculated from the mass of (1)

(V) alpaca species of the genus *Lama* of the family Camelidae (or alpaca species of the genus *Vicugna*)

(1) the mass detected specifically to the alpaca species (or, the alpaca species of the genus *Vicugna*),
4599.37 ± 0.02%, 901.47 ± 0.02%, or 532.32 ± 0.02%,
(2) the mass-to-charge ratio (m/z) of molecular ions calculated from the mass of (1)

(VI) Angora rabbit species of the genus *Oryctolagus* of the family Leporidae

(1) the mass detected specifically to the Angora rabbit species,
2303.07 ± 0.02%, 2502.15 ± 0.02%, 2058.02 ± 0.02%, or 1554.81 ± 0.02%,
(2) the mass-to-charge ratio (m/z) of molecular ions calculated from the mass of (1).

21. The method according to claim 13 or 16, wherein the step (a) of obtaining an analytical sample from the sample by pretreatment includes a step of using lysyl endopeptidase (also referred to as "Lys-C"), a common mass-to-charge ratio shared by the yak species, the cashmere species, and the sheep species is 1 or more mass-to-charge ratios selected from the following mass-to-charge ratios:

(1) the mass-to-charge ratio of molecular ions calculated from the mass of 1070.5 ± 0.02%;
(2) the mass-to-charge ratio of molecular ions calculated from the mass of 1555.76 ± 0.02%;
(3) the mass-to-charge ratio of molecular ions calculated from the mass of 1160.58 ± 0.02%; and
(4) the mass-to-charge ratio of molecular ions calculated from the mass of 602.30 ± 0.02%.

22. A method for quantifying animal hair in a sample, comprising the steps of:

(a) obtaining an analytical sample from the sample by pretreatment;

(b) adding an animal hair marker containing a stable isotope to the analytical sample obtained in the step (a), analyzing the analytical sample using mass spectrometry and then obtaining a result;

(c) calculating the peak area of animal-specific mass-to-charge ratios and the peak area of the mass-to-charge ratios of the animal hair marker containing the stable isotope, and then calculating the peak area of the mass-to-charge ratios of the animal hair marker in terms of concentration or amount based on the concentration of the animal hair marker added to the analytical sample; and

(d) quantifying animal hair contained in an extracted fluid sample through comparison with the peak area derived from the extracted fluid sample.

23. The method according to claim 22, wherein the step (a) of obtaining an analytical sample from the sample by pretreatment includes a step of using lysyl endopeptidase (also referred to as "Lys-C") and the animal hair marker is any one of the peptide markers of claim 11.

## Fig. 1

# Fig. 2-1

Raw material  Yak-specific mass charge ratio m/z=
1983.52 (z=3)
1487.64 (z=4)
5949.90 (z=1)

# Fig. 2-2

Raw material  Yak-specific mass charge ratio m/z=
1069.98 (z=2)
2138.92 (z=1)

EP 2 837 934 A1

## Fig. 2-3

Raw material  Cashmere-specific mass charge ratio m/z=
1795.78 (z=5)
8977.45 (z=1)

EP 2 837 934 A1

## Fig. 2-4

Raw material Cashmere-specific mass charge ratio m/z=
1421.44 (z=5)
7103.18 (z=1)

# Fig. 2-5

Raw material  Cashmere-specific mass charge ratio m/z=
1415.83 (z=5)
7075.18 (z=1)

EP 2 837 934 A1

# Fig. 2-6

Raw material  Sheep-specific mass charge ratio m/z=
1949.84 (z=9)
1754.82 (z=10)
17539.88 (z=1)

camel

angora

alpaka

moheya

▼ 10.03

sheep

yak

cashmere

0   2.0   4.0   6.0   8.0   10.0   12.0   14.0   16.0   18.0   20.0   22.0   24.0   26.0   Time(min)

## Fig. 2-7

Raw material  Sheep-specific mass charge ratio m/z=
1759.47 (z=10)
17585.26 (z=1)

EP 2 837 934 A1

# Fig. 2-8

Raw material  Angora (moheya)-specific mass charge ratio m/z=
1055.92 (z=6)
6330.52 (z=1)

EP 2 837 934 A1

Fig. 2-9

Raw material Alpaca-specific mass charge ratio m/z=
1272.72 (z=5)
6359.60 (z=1)

## Fig. 2-10

Raw material  Alpaca-specific mass charge ratio m/z=
1269.54 (z=5)
6343.70 (z=1)

EP 2 837 934 A1

## Fig. 2-11

Raw material  Angora rabbit-specific mass charge ratio m/z= 1043.43 (z=1)

# Fig. 3-1

Analytical example  Raw wool/Yak/Extraction/Lysyl endopeptidase treatment

Yak43
IKHTAMIR YAK
BLACK

Raw yak wool
Sample with the mass of 1365 detected    50/50

# Fig. 3-2

Analytical example  Raw wool/Yak/Extraction/Lysyl endopeptidase treatment

Yak43
IKHTAMIR YAK
BLACK

Raw yak wool
Sample with the mass of 2287 detected   40/50

EP 2 837 934 A1

# Fig. 4a

Analytical example  Raw wool/Yak/Extraction/Lysyl endopeptidase treatment
Confirmation 1 of the detection of masses when dyeing and/or decoloring treatments were varied.

# Fig. 4b

Analytical example  Raw wool/Yak/Extraction/Lysyl endopeptidase treatment
Confirmation 2 of the detection of masses when dyeing and/or decoloring treatments were varied.

EP 2 837 934 A1

## Fig. 5-1

Animal hair  Cashmere·yak·sheep (total mass ion chromatogram)

## Fig. 5-2

Raw material Cashmere-specific mass (2218.19)-derived molecular ion chromatogram

Cashmere

▼ 6.64

Yak

Sheep

0 5.0 10.0 Time(min)

# Fig. 5-3

Raw material Yak-specific mass (1364.70)-derived molecular ion chromatogram

EP 2 837 934 A1

# Fig. 5-4

Raw material  Sheep-specific mass (2479.30)-derived molecular ion chromatogram

EP 2 837 934 A1

Fig. 5-5

## Fig. 5-6

## Fig. 5-7

Sheep

EP 2 837 934 A1

# Fig. 6-1

Extraction/Enzyme: Lys-C/Raw wool samples with varied mixture ratios of cashmere to yak/TIC

Cashmere : Yak

Cashmere 100

Cashmere 98
Yak 2

Cashmere 95
Yak 5

Cashmere 80
Yak 20

Cashmere 65
Yak 35

Cashmere 50
Yak 50

Cashmere 35
Yak 65

Cashmere 20
Yak 80

Cashmere 5
Yak 95

Yak100

0   2.0   4.0   6.0   8.0   10.0   12.0   14.0   16.0   18.0   Time(min)

EP 2 837 934 A1

Fig. 6-2

Extraction/Enzyme: Lys-C/Raw wool samples with varied mixture ratios of cashmere to yak/Confirmation of the detection of the mass of yak in each sample

# Fig. 6-3

(Horizontal axis: yak peak area/peak areas of mass charge ratios commonly detected in both cashmere and yak)
(Vertical axis: Percentage of yak mixed in)
Mass charge ratio commonly detected in both cashmere and yak is m/z=778.90±1%

calibration curve of the percentage of yak mixed in

Yak (%)

$y = 105.75x - 1.2135$

Linear
(calibration curve
of the percentage
of yak mixed in)

## Fig. 7-1

Confirmation of the detection of mass after dyeing
Cashmere-specific mass (2218.19)-derived molecular ion chromatogram

6.64

Raw material
(unprocessed)

Acid dye

Metal
complex dye

Reactive dye

Chrome dye

4.0    5.0    6.0    7.0    8.0   Time(min)

EP 2 837 934 A1

# Fig. 7-2

Confirmation of the detection of mass after dyeing
Yak-specific mass (1364.7)-derived molecular ion chromatogram

▼ 4.70

Raw material
(unprocessed)

Acid dye

Metal
complex dye

Reactive dye

Chrome dye

4.2    5.0    6.0    7.0    8.0    Time(min)

EP 2 837 934 A1

## Fig. 7-3

Confirmation of the detection of mass after dyeing
Sheep-specific mass (2479.30)-derived molecular ion chromatogram

▼ 4.65

Raw material
(unprocessed)

Acid dye

Metal
complex dye

Reactive dye

Chrome dye

3.4     4.0     5.0     6.0     7.0     Time(min)

# Fig. 7-4

Confirmation of the detection of mass after dyeing
Camel-specific mass (2888.35)-derived molecular ion chromatogram

EP 2 837 934 A1

## Fig. 7-5

Confirmation of the detection of mass after dyeing
Alpaca-specific mass (4599.37)-derived molecular ion chromatogram

▼ 9.45

Raw material
(unprocessed)

Acid dye

Metal
complex dye

Reactive dye

Chrome dye

9.0                    10.0                    Time(min)

EP 2 837 934 A1

# Fig. 7-6

Confirmation of the detection of mass after dyeing
Angora rabbit-specific mass (2303.07)-derived molecular ion chromatogram

▼ 3.93

Raw material
(unprocessed)

Acid dye

Metal
complex dye

Reactive dye

Chrome dye

3.0                    4.0                    5.0Time(min)

EP 2 837 934 A1

# Fig. 7-7

Confirmation of the detection of mass after dyeing
Cashmere-specific mass (2218.19)-derived molecular ion chromatogram

Raw material (unprocessed)

Bleaching→ Acid dye

Bleaching→ Metal complex dye

Bleaching→ Reactive dye

Bleaching→ Chrome dye

6.64

4.0        5.0        6.0        7.0        8.0        Time(min)

EP 2 837 934 A1

Fig. 7-8

Confirmation of the detection of mass after dyeing
Yak-specific mass (1364.7)-derived molecular ion chromatogram

4.70

Raw material
(unprocessed)

Bleaching→
Acid dye

Bleaching→
Metal complex dye

Bleaching→
Reactive dye

Bleaching→
Chrome dye

5.0　6.0　7.0　8.0　Time(min)

# Fig. 7-9

Confirmation of the detection of mass after dyeing
Sheep-specific mass (2479.30)-derived molecular ion chromatogram

▼ 4.65

Raw material
(unprocessed)

Bleaching→
Acid dye

Bleaching→
Metal complex dye

Bleaching→
Reactive dye

Bleaching→
Chrome dye

4.0          5.0          6.0          7.0    Time(min)

# Fig. 7-10

Confirmation of the detection of mass after dyeing
Camel-specific mass (2888.35)-derived molecular ion chromatogram

▼ 3.99

Raw material
(unprocessed)

Bleaching→
Acid dye

Bleaching→
Metal complex dye

Bleaching→
Reactive dye

Bleaching→
Chrome dye

3.0          4.0          5.0          6.0          Time(min)

# Fig. 7-11

Confirmation of the detection of mass after dyeing
Alpaca-specific mass (4599.37)-derived molecular ion chromatogram

▼ 9.45

Raw material
(unprocessed)

Bleaching→
Acid dye

Bleaching→
Metal complex dye

Bleaching→
Reactive dye

Bleaching→
Chrome dye

9.0          10.0          Time(min)

## Fig. 7-12

Confirmation of the detection of mass after dyeing
Angora rabbit-specific mass (2303.07)-derived molecular ion chromatogram

▼ 3.93

Raw material
(unprocessed)

Bleaching→
Acid dye

Bleaching→
Metal complex dye

Bleaching→
Reactive dye

Bleaching→
Chrome dye

3.0                    4.0                    5.0 Time(min)

EP 2 837 934 A1

## Fig. 7-13

Confirmation of the detection of mass after water repellent finishing
Cashmere-specific mass (2218.19)-derived molecular ion chromatogram

EP 2 837 934 A1

Fig. 7-14

Confirmation of the detection of mass after water repellent finishing
Yak-specific mass (1364.7)-derived molecular ion chromatogram

4.70

Raw material (unprocessed)

Fluororesin processing

Silicone resin

Wax-based

Octaethylene urea-based

Time(min)

5.0    6.0    7.0    8.0

EP 2 837 934 A1

## Fig. 7-15

Confirmation of the detection of mass after water repellent finishing
Sheep-specific mass (2479.30)-derived molecular ion chromatogram

▼ 4.65

Raw material
(unprocessed)

Fluororesin processing

Silicone resin

Wax-based

Octaethylene
urea-based

4.0          5.0          6.0          7.0        Time(min)

## Fig. 7-16

Confirmation of the detection of mass after water repellent finishing
Camel-specific mass (2888.35)-derived molecular ion chromatogram

EP 2 837 934 A1

# Fig. 7-17

Confirmation of the detection of mass after water repellent finishing
Alpaca-specific mass (4599.37)-derived molecular ion chromatogram

▼ 9.45

Raw material
(unprocessed)

Fluororesin processing

Silicone resin

Wax-based

Octaethylene
urea-based

9.0        10.0        Time(min)

# Fig. 7-18

Confirmation of the detection of mass after water repellent finishing
Angora rabbit-specific mass (2303.07)-derived molecular ion chromatogram

EP 2 837 934 A1

## Fig. 7-19

Confirmation of the detection of mass after softening
Cashmere-specific mass (2218.19)-derived molecular ion chromatogram

Raw material
(unprocessed)

Amino-modified
silicone-based

Polymer wax-based

6.64

4.0    5.0    6.0    7.0    8.0 Time(min)

## Fig. 7-20

Confirmation of the detection of mass after softening
Yak-specific mass (1364.7)-derived molecular ion chromatogram

4.70

Raw material
(unprocessed)

Amino-modified
silicone-based

Polymer wax-based

5.0    6.0    7.0    8.0    Time(min)

EP 2 837 934 A1

## Fig. 7-21

Confirmation of the detection of mass after softening
Sheep-specific mass (2479.30)-derived molecular ion chromatogram

Raw material
(unprocessed)

Amino-modified
silicone-based

Polymer wax-based

EP 2 837 934 A1

# Fig. 7-22

Confirmation of the detection of mass after softening
Camel-specific mass (2888.35)-derived molecular ion chromatogram

Raw material
(unprocessed)

Amino-modified
silicone-based

Polymer wax-based

2.0    3.0    4.0    5.0    6.0    Time(min)

## Fig. 7-23

Confirmation of the detection of mass after softening
Alpaca-specific mass (4599.37)-derived molecular ion chromatogram

Raw material
(unprocessed)

Amino-modified
silicone-based

Polymer wax-based

9.0      10.0      Time(min)

9.45

## Fig. 7-24

Confirmation of the detection of mass after softening
Angora rabbit-specific mass (2303.07)-derived molecular ion chromatogram

Raw material
(unprocessed)

Amino-modified
silicone-based

Polymer wax-based

3.93

3.0    4.0    5.0 Time(min)

Fig. 8-1

Product labeled as 100% cashmere (identified)

Fig. 8-2

Product labeled as 100% wool (identified)

Fig. 8-3

Product labeled as 100% yak (identified)

## Fig. 9-1

Product Cashmere·yak·sheep (total mass ion chromatogram)

Fig. 9-2

Product  Cashmere-specific mass (2218.19)-derived molecular ion chromatogram

Cashmere

▼ 6.9

Yak

Sheep

2.0    4.0    6.0    8.0    10.0    12.0    Time(min)

# Fig. 9-3

Product  Yak-specific mass (1364.70)-derived molecular ion chromatogram

# Fig. 9-4

Product Sheep-specific mass (2479.30)-derived molecular ion chromatogram

EP 2 837 934 A1

Fig. 10-1

????? F A A F I D K

EP 2 837 934 A1

## Fig. 10-2

Fig. 10-3

Fig. 10-4

## Fig. 11

Examination of the quantification of yak wool (animal hair of the yak variety) using yak variety-specific molecular mass

# Fig. 12-1

Extraction/Enzyme: Lys-C/Products with varied mixture ratios/TIC

Cashmere: Yak

Cashmere 4

Cashmere 3
Yak 1

Cashmere 2
Yak 2

Cashmere 1
Yak 3

Yak 4

2.0    4.0    6.0    8.0    10.0    12.0    Time(min)

# Fig. 12-2

Extraction/Enzyme: Lys-C/Products with varied mixture ratios/Confirmation of the detection of the mass of yak in each sample

Cashmere: Yak

## Fig. 12-3

Extraction/Enzyme: Lys-C/Products with varied mixture ratios/TIC

Cashmere: Sheep

Cashmere 4

Cashmere 3
Sheep 1

Cashmere 2
Sheep 2

Cashmere 1
Sheep 3

Sheep 4

Time(min)

2.0   4.0   6.0   8.0   10.0   12.0

## Fig. 12-4

Extraction/Enzyme: Lys-C/Products with varied mixture ratios/Confirmation of the detection of the mass of sheep in each sample

Cashmere: Sheep

EP 2 837 934 A1

# Fig. 12-5

(Horizontal axis: yak peak area)
(Vertical axis: Yak weight/total weight x 100)

Yak $\quad y = 0.5124x$
$R^2 = 0.99432$

wt%

Peak area

# Fig. 12-6

(Horizontal axis: sheep peak area)
(Vertical axis: Sheep weight/total weight x 100)

## INTERNATIONAL SEARCH REPORT

| International application No. |
| --- |
| PCT/JP2013/061345 |

A. CLASSIFICATION OF SUBJECT MATTER
*G01N27/62*(2006.01)i, *C07K5/00*(2006.01)i, *C07K7/00*(2006.01)i, *G01N33/483*
(2006.01)i

According to International Patent Classification (IPC) or to both national classification and IPC

B. FIELDS SEARCHED

Minimum documentation searched (classification system followed by classification symbols)
G01N27/62-27/70, C07K5/00, C07K7/00, G01N33/483

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamIII), UniProt/GeneSeq

C. DOCUMENTS CONSIDERED TO BE RELEVANT

| Category* | Citation of document, with indication, where appropriate, of the relevant passages | Relevant to claim No. |
| --- | --- | --- |
| X Y  A | Hollemeyer, K. et al., Identification and Quantification of Feathers, Down, and Hair of Avian and Mammalian Origin Using Matrix-Assisted Laser Desorption/Ionization Time-of-Flight Mass Spectrometry, Analytical Chemistry, 2002.11.01, vol.74, no.23, pp.5960-5968 | 1-5,15 6-8,12-14, 16-19,22,23 9,10,20,21 |
| X Y A | Sen'i Sangyo ni Kakaru Heisei 21 Nendo 'Joho Gyomu' ni Okeru 'Cashmere Hyoji no Tekiseika ni Kakaru Chosa Jigyo' Itaku Seika Hokokusho, Organization for Small & Medium Enterprises and Regional Innovation, Japan, 2010.02 | 1-4,15 6-8 9,10,20,21 |

| ☒ Further documents are listed in the continuation of Box C. | ☐ See patent family annex. |
| --- | --- |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 19 July, 2013 (19.07.13) | 06 August, 2013 (06.08.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2013/061345

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-241721 A (Medical Proteoscope Co., Ltd.), 09 October 2008 (09.10.2008), & US 2006/0194329 A1 & EP 1626274 A1 & WO 2004/090526 A1 & CA 2521108 A | 8 |
| Y | JP 2010-503852 A (Koninklijke Philips Electronics N.V.), 04 February 2010 (04.02.2010), paragraph [0065] & US 2010/0298153 A1 & EP 2069797 A & WO 2008/032235 A2 & CN 101517416 A & RU 2009113801 A | 6,7 |
| Y | JP 2005-522713 A (Thermo Finnigan, L.L.C.), 28 July 2005 (28.07.2005), paragraphs [0009] to [0010], [0015] & US 2006/0141631 A1 & EP 1495332 A & WO 2003/089937 A2 & CA 2484078 A & CN 1692282 A & AU 2003230957 A | 12-14,16-19 |
| Y | JP 2011-522210 A (Inserm (Institut National de la Sante et de la Recherche Medicale)), 28 July 2011 (28.07.2011), claims 1, 4 & US 2010/0173786 A1 & EP 2167974 A & WO 2008/145763 A1 & CA 2689304 A | 22,23 |
| X Y | Yu, Z et al., Annotation of sheep keratin intermediate filament genes and their patterns of expression, Experimental Dermatology, 2011.03.10, Vol.20, No.7, pp.582-588 | 11 23 |

Form PCT/ISA/210 (continuation of second sheet) (July 2009)

EP 2 837 934 A1

INTERNATIONAL SEARCH REPORT

International application No.

PCT/JP2013/061345

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
       See extra sheet.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (July 2009)

**INTERNATIONAL SEARCH REPORT**

International application No.

PCT/JP2013/061345

Continuation of Box No.III of continuation of first sheet(2)

Document 1: Hollemeyer, K. et al., Identification and Quantification of Feathers, Down, and Hair of Avian and Mammalian Origin Using Matrix-Assisted Laser Desorption/Ionization Time-of-Flight Mass Spectrometry, Analytical Chemistry, 2002.11.01, vol.74, no.23, pp.5960-5968

Document 2: Sen'i Sangyo ni Kakaru Heisei 21 Nendo 'Joho Gyomu' ni Okeru 'Cashmere Hyoji no Tekiseika ni Kakaru Chosa Jigyo' Itaku Seika Hokokusho, Organization for Small & Medium Enterprises and Regional Innovation, Japan, 2010.02

Both document 1 and document 2 (pp. 18-20) set forth identification of animal hair species such as cashmere by obtaining an analysis sample from a sample through a pretreatment, analyzing the analysis sample with use of a mass spectrometry method, and comparing the result with the mass charge ratios specific to animal hair species obtained by known samples.

Document 1 also sets forth 1769 as the mass charge ratio specific to cashmere (Table 2). Consequently, the invention of claims 1, 3, 4 and 15 is not novel over the inventions set forth in documents 1 and 2, and the invention of claim 5 is not novel over the invention set forth in document 1. The invention of claims 1, 3, 4 and 15 and the invention of claim 5 therefore do not have a special technical feature.

With respect to claim 2, LC-MS is a well-known art so that it is needless to mention a document, and does not have a new effect. Consequently, claim 2 does not have a special technical feature.

Therefore, eight invention groups each having a special technical feature indicated below are involved in claims.

Meanwhile, the inventions of claims 1-5 having no special technical feature are classified into invention group 1.

(Invention group 1) the inventions of claims 1-5 and 15

The invention having no special technical feature.

(Invention group 2) the inventions of claims 6-10

Inventions, the special technical feature of which is the use of lysylendopeptidase in the pretreatment

(Invention group 3) A part of the invention of claim 11, which is related to animal hair markers that are defined by (1) and (5)

Inventions, the special technical feature of which is a peptide marker specific to yak hair or a nucleic acid marker encoding same

(Invention group 4) A part of the invention of claim 11, which is related to animal hair markers that are defined by (2)

Inventions, the special technical feature of which is a peptide marker specific to cashmere hair

(Invention group 5) A part of the invention of claim 11, which is related to animal hair markers that are defined by (3)

Inventions, the special technical feature of which is a peptide marker specific to sheep hair and comprising a combination of two or more amino acid sequences represented by SEQ ID NOS: 4-9

(Invention group 6) A part of the invention of claim 11, which is related to animal hair markers that are defined by (4)

(Continued to next extra sheet)

Form PCT/ISA/210 (extra sheet) (July 2009)

**INTERNATIONAL SEARCH REPORT**

International application No.

PCT/JP2013/061345

Inventions, the special technical feature of which is a peptide marker specific to sheep hair and comprising a combination of two or more amino acid sequences represented by SEQ ID NOS: 10-13
(Invention group 7) claims 12-14 and 16-21
Inventions, the special technical feature of which is to calculate the mixing ratio of animal hair samples from a peak area by forming an ion chromatogram
(Invention group 8) claims 22-23
Inventions, the special technical feature of which is addition of an animal hair marker comprising a stable isotope

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003297332 A
- JP 2004121229 A
- JP H1115616 B
- JP 2000210084 A
- JP 11135993 A
- JP 2000325098 A
- JP 3566570 B
- JP 2012089662 A